# EUROPÄISCHE PATENTSCHRIFT

(11) **EP 0 350 733 B1**
(45) Veröffentlichungstag und Bekanntmachung des Hinweises auf die Patenterteilung: **20.08.1997**
(21) Anmeldenummer: 89111950.5
(22) Anmeldetag: 30.06.1989
(51) Int. Cl.: C07D 401/04, A61K 31/47, C07D 471/04, C07D 498/04, C07D 487/04, C07D 519/00, C07D 513/04, A61K 31/535, A61K 31/54, C07D 407/14, C07D 215/56

(54) **7-(1-Pyrrolidinyl)-3-chinolon- und -naphthyridoncarbon-säure-Derivate, Verfahren zu ihrer Herstellung sowie substituierte mono-und bicyclische Pyrrolidinderivate als Zwischenprodukte zu ihrer Herstellung, und sie enthaltende antibakterielle Mittel und Futterzusatzstoffe**
7-(1-Pyrrolidinyl)-3-quinolone- and -naphthyridone-carboxylic-acid derivatives, method for their preparation and for substituted mono- and bi-cyclic pyrrolidine intermediates, and their antibacterial and feed additive compositions
Dérivés d'acides 7-(1-pyrrolidinyl)-3-quinolone et -naphtyridone carboxyliques, procédé pour leur préparation

(30) Priorität: 15.07.1988 DE 3824072; 01.03.1989 DE 3906365
(43) Veröffentlichungstag der Anmeldung: 17.01.1990
(60) Teilanmeldung: 96113744.5
(73) Patentinhaber: BAYER AG, 51368 Leverkusen (DE)
(72) Erfinder: Petersen, Uwe, Dr., D-5090 Leverkusen (DE); Schenke, Thomas, Dr., D-5060 Bergisch-Gladbach 2 (DE); Krebs, Andreas, Dr., D-5068 Odenthal (DE); Grohe, Klaus, Dr., D-5068 Odenthal (DE); Schriewer, Michael, Dr, D-5068 Odenthal (DE); Haller, Ingo, Dr., D-5600 Wuppertal 1 (DE); Metzger, Karl Georg, Dr., D-5600 Wuppertal 1 (DE); Endermann, Rainer, Dr., D-5600 Wuppertal 1 (DE); Zeiler, Hans-Joachim, Dr., D-5600 Wuppertal 1 (DE)

(56) Entgegenhaltungen:
- EP-A- 347 851
- EP-A- 0 106 489
- EP-A- 0 241 206
- JP-A- 259 388
- JP-A- 5 880 989
- JOURNAL OF MEDICINAL CHEMISTRY, Band 27, Nr. 12, Dezember 1984, Seiten 1543-1549, American Chemical Society; H. Egawa et al.: "Pyridonecarboxylic acids as antibacterial agents. 4. Synthesis and antibacterial activity of 7-(3-amino-1- pyrrolidinyl)-1-ethyl-6-fluoro-1,4-dihydro-4-oxo-1,8-naphthyridine-3-carboxylic acid and its analogues"
- CHEMICAL ABSTRACTS, Band 104, 1986, Seite 521, Zusammenfassung Nr.50861t, Columbus, Ohio, US; & JP-A-60 126 284
- CHEMICAL ABSTRACTS, Band 111, 1989, Seite 742, Zusammenfassung Nr.97210q, Columbus, Ohio, US; & JP-A-64 03 181
- CHEMICAL ABSTRACTS, Band 111, 1989, Seite 721, Zusammenfassung Nr. 153779w, Columbus, Ohio, US; & JP-A-01 56 673

## Beschreibung

Die Erfindung betrifft neue in 7-Position mit bicyclischen Pyrrolidinderivaten substituierte 3-Chinolon- und -Naphthyridoncarbonsäure-Derivate, Verfahren zu ihrer Herstellung sowie diese enthaltende antibakterielle Mittel und Futterzusatzstoffe.

Es ist bereits eine Reihe von 3-Chinolon- und Naphthyridoncarbonsäuren bekanntgeworden, die in 7-Position durch einen Pyrrolidinylring substituiert sind. Deutsche Patentanmeldung 3 318 145, Europäische Patentanmeldungen 106 489, 153 826 und 241 206.

In der europäischen Patentanmeldung EP-A-0 347 851 (Prioritäten vom 21. Juni 1988 und 10. März 1989, Offenlegungstag: 27. Dezember 1989) werden Chinoloncarbonsäurederivate mit der allgemeinen Formel 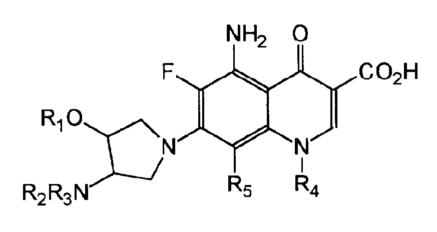 sowie deren Verwendung als antibakterielle Mittel offenbart.

Es wurde gefunden, daß die neuen 3-Chinolon- und -Naphthyridoncarbonsäure-Derivate der Formel (I) 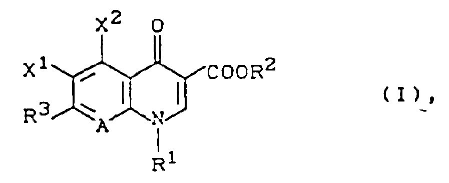 in welcher
X¹ für Halogen,
X² für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen,
R¹ für Alkyl mit 1 bis 4 Kohlenstoffatomen, Alkenyl mit 2 bis 4 Kohlenstoffatomen, Cycloalkyl mit 3 bis 6 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,
R² für Wasserstoff, Alkyl mit 1 bis 4 Kohlenstoffatomen oder (5-Methyl-2-oxo-1,3-dioxol-4-yl)-methyl,
R³ für einen Rest der Struktur 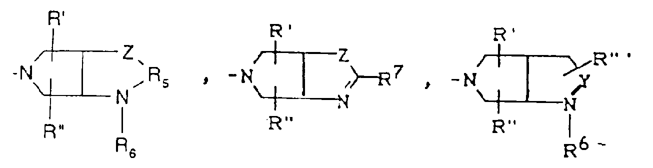 steht, worin
   R⁵ eine gegebenenfalls durch Methyl ein- oder zweifach substituierte C₁-C₃-Alkylenbrücke bedeutet,
   R⁶ für H, gegebenenfalls durch Hydroxy substituiertes C₁-C₄-Alkyl, sowie Aryl, Heteroaryl, Benzyl C₁-C₄-Alkoxycarbonyl, C₁-C₄-Acyl, (5-Methyl-2-oxo-1,3-dioxol-4-yl)-methyl oder C₃-C₆-Cycloalkyl,
   R⁷ für H oder C₁-C₄-Alkyl,
   R' für H, CH₃ oder Phenyl,
   R" für H, CH₃ oder Phenyl,
   R"' für H oder CH₃,
   Y für O, CH₂, CH₂CH₂ oder CH₂-0 stehen kann, wobei die Verknüpfung der CH₂-0-Gruppe zum Stickstoff sowohl über O als auch über CH₂ erfolgen kann,
   Z für O oder S stehen kann,
A für N oder C-R⁸ steht, worin
   R⁸ für H, Halogen, Methyl, Cyano, Nitro, Hydroxy oder Methoxy steht oder auch gemeinsam mit R¹ eine Brücke der Struktur 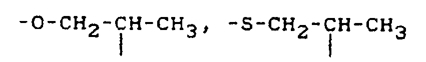 oder 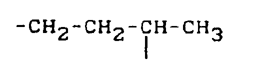 bilden kann,
      und deren pharmazeutisch verwendbaren Hydrate und Säureadditionssalze sowie die Alkali-, Erdalkali-, Silber-und Guanidiniumsalze der zugrundeliegenden Carbonsäuren eine hohe antibakterielle Wirkung insbesondere im grampositiven Bereich aufweisen.

Bevorzugt sind die Verbindungen der Formel (I) 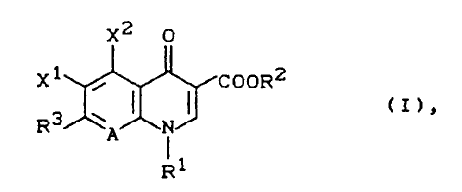 in welcher
X¹ für Fluor oder Chlor,
X² für Wasserstoff, Amino, Alkylamino mit 1 bis 2 Kohlenstoffatomen, Dimethylamino, Hydroxy, Methoxy, Mercapto, Methylthio, Phenylthio, Fluor, Chlor,
R¹ für Alkyl mit 1 bis 3 Kohlenstoffatomen, Alkenyl mit 2 bis 3 Kohlenstoffatomen, Cycloalkyl mit 3 bis 5 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,
R² für Wasserstoff, Alkyl mit 1 bis 3 Kohlenstoffatomen oder (5-Methyl-2-oxo-1,3-dioxol-4-yl)-methyl,
R³ für einen Rest der Struktur 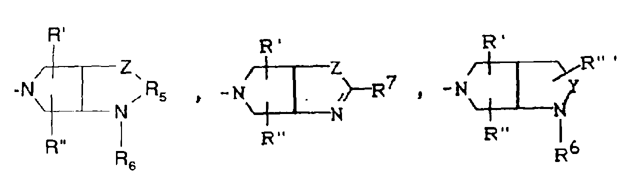 steht, worin
   R⁵ eine gegebenenfalls durch Methyl ein- oder zweifach substituierte C₁-C₂-Alkylenbrücke bedeutet,
   R⁶ für H, gegebenenfalls durch Hydroxy substituiertes C₁-C₃-Alkyl, sowie Phenyl Benzyl, C₁-C₄-Alkoxycarbonyl, C₁-C₂-Acyl, (5-Methyl-2-oxo-1,3-dioxol-4-yl)-methyl oder C₃-C₅ Cycloalkyl,
   R⁷ für H oder C₁-C₂-Alkyl,
   R' für H oder CH₃,
   R" für H oder CH₃,
   R"' für H oder CH₃,
   Y für O, CH₂, CH₂CH₂ oder CH₂-0 stehen kann, wobei die Verknüpfung der CH₂-0-Gruppe zum Stickstoff sowohl über O als auch über CH₂ erfolgen kann,
   Z für O oder S stehen kann,
A für N oder C-R⁸ steht, worin
   R⁸ für H, Fluor, Chlor, Brom, Hydroxy oder Methoxy steht oder auch gemeinsam mit R¹ eine Brücke der Struktur 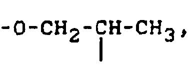 bilden kann.

Besonders bevorzugt sind die Verbindungen der Formel (I) 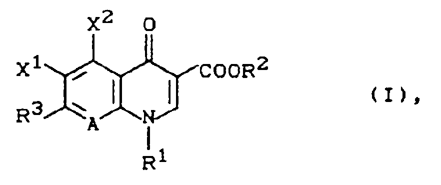 in welcher
X¹ für Fluor,
X² für Wasserstoff, Amino, Methylamino, Fluor,
R¹ für Alkyl mit 1 bis 2 Kohlenstoffatomen, Vinyl, Cyclopropyl, 2-Hydroxyethyl, 2-Fluorethyl, Methoxy Methylamino, 4-Fluorphenyl, 2,4-Difluorphenyl,
R² für Wasserstoff, Alkyl mit 1 bis 2 Kohlenstoffatomen,
R³ für einen Rest der Struktur 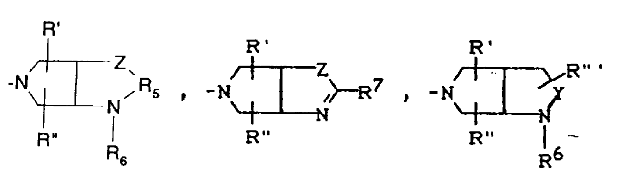 steht, worin
   R⁵ eine gegebenenfalls durch Methyl ein- oder zweifach substituierte C₁-C₂-Alkylenbrücke bedeutet,
   R⁶ für H, CH₃, C₂H₅, HOCH₂CH_{2;} Benzyl, C₁-C₄-Alkoxycarbonyl, C₁-C₂-Acyl,
   _{R}7 für H oder CH₃,
   R' für H oder CH₃,
   R" für H oder CH₃,
   R"' für H oder CH₃,
   Y für O, CH₂, CH₂CH₂ oder CH₂-0 stehen kann, wobei die Verknüpfung der CH₂-0-Gruppe zum Stickstoff sowohl über O als auch über CH₂ erfolgen kann,
   Z für O oder S stehen kann,
A für N oder C-R⁸ steht, worin
   R⁸ für H, Fluor, Chlor oder Methoxy steht, oder auch gemeinsam mit R¹ eine Brücke der Struktur 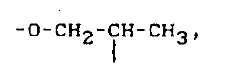 bilden kann.

Weiterhin wurde gefunden, daß man die Verbindungen der Formel (I) erhalt, wenn man Verbindungen der Formel (II) 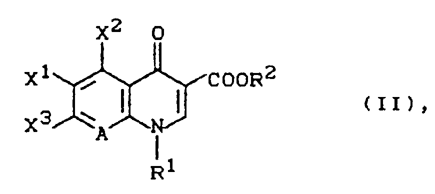 in welcher
A, R¹, R², X¹ und X² die oben angegebene Bedeutung haben und
X³ für Halogen, insbesondere Fluor oder Chlor steht, mit Verbindungen der Formel (III) 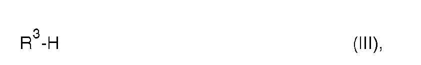 in welcher
R³ die oben angegebene Bedeutung hat,

gegebenenfalls in Gegenwart von Säurefängern umsetzt, und gegebenenfalls in R³ enthaltene Schutzgruppen abspaltet (Methode A).

Erfindungsgemäße Verbindungen der Formel (I) 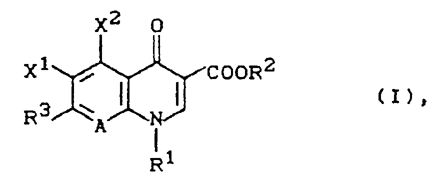 in welcher
X¹, R¹, R², R³ und A die oben angegebene Bedeutung haben und
X² für Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen oder Arylthio steht,

können auch erhalten werden, indem man eine Verbindung der Formel (IV) 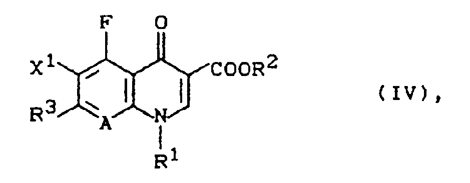 in welcher
X¹; R¹, R², R³ und A die oben angegebene Bedeutung haben,
   mit Verbindungen der Formel (V) 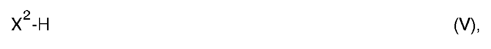 in welcher
X² die oben angegebene Bedeutung hat, gegebenenfalls in Gegenwart von Säurefängern umsetzt (Methode B). Erfindungsgemäße Verbindungen der Formel (la) 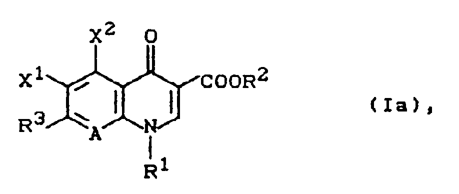 in welcher
X¹; X², R¹, R² und A die oben angegebene Bedeutung haben und R³ für einen Rest der Struktur 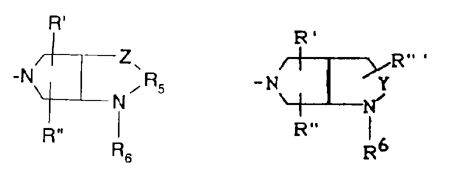 steht, worin
   R⁵, R⁶, R', R", R"', Y und Z die oben angegebene Bedeutung haben, können auch erhalten werden, indem man eine Verbindung der Formel (VI) 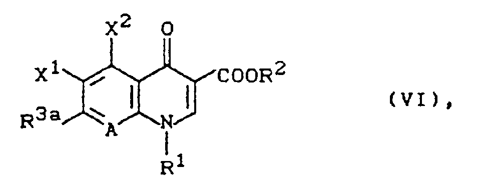 in welcher
X¹, X², R¹, R² und A die oben angegebene Bedeutung haben und
_{R}3a für einen Rest der Struktur 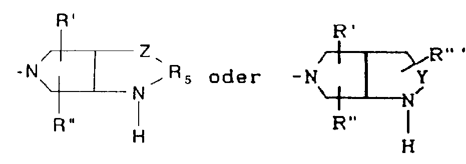 steht,
   worin R⁵, R', R", R"', Y und Z die oben angegebene Bedeutung haben,
   mit Verbindungen der Formel (VII) 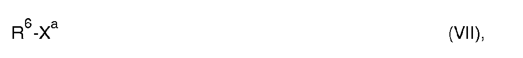 in welcher
R⁶ die oben angegebene Bedeutung hat und
X^{a} für Chlor, Brom, lod, Hydroxy oder Acyloxy steht,

gegebenenfalls in Gegenwart von Säurefängern umsetzt (Methode C).

Verwendet man beispielsweise 1-Cyclopropyl-6,7,8-trifluor-1,4-dihydro-4-oxo-3-chinolincarbonsäure und 1-Methyl-octahydropyrrolo[3,4-b]pyridin als Ausgangsstoffe, so kann der Reaktionsverlauf durch folgendes Formelschema wiedergegeben werden:

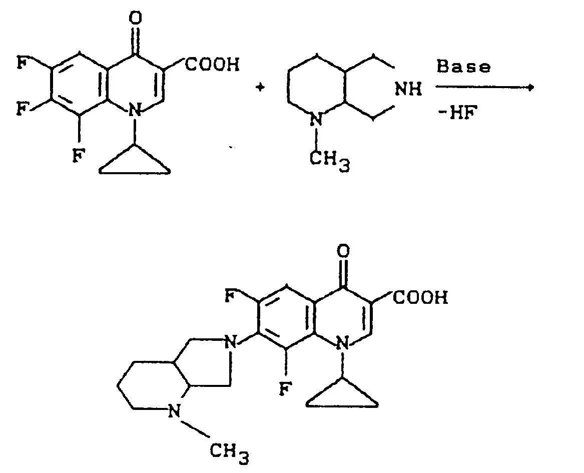

Verwendet man beispielsweise 1-Cyclopropyl-5,6,8-trifluor-1,4-dihydro-7-(2-methyl-2,7-diazabicyclo[3.3.0] oct-3-yl)-4-oxo-3-chinolincarbonsäure und Ammoniak als Ausgangsstoffe, so kann der Reaktionsablauf durch folgendes Formelschema wiedergegeben werden:

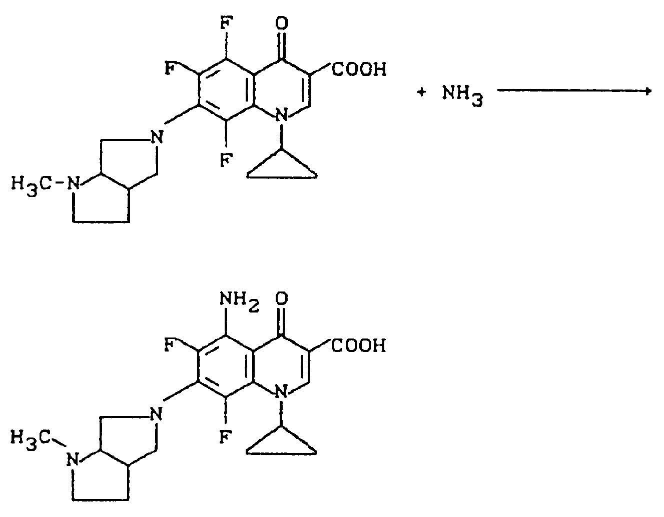

Verwendet man beispielsweise 1-Cyclopropyl-7-(2,7-diazabicyclo[3.3.0]oct-7-yl)-6-fluor-1,4-dihydro-4-oxo-3-chi- nolincarbonsäure und Ethanol/Chlorwasserstoff als Ausgangsstoffe, so kann der Reaktionsverlauf durch folgendes Formelschema wiedergegeben werden:

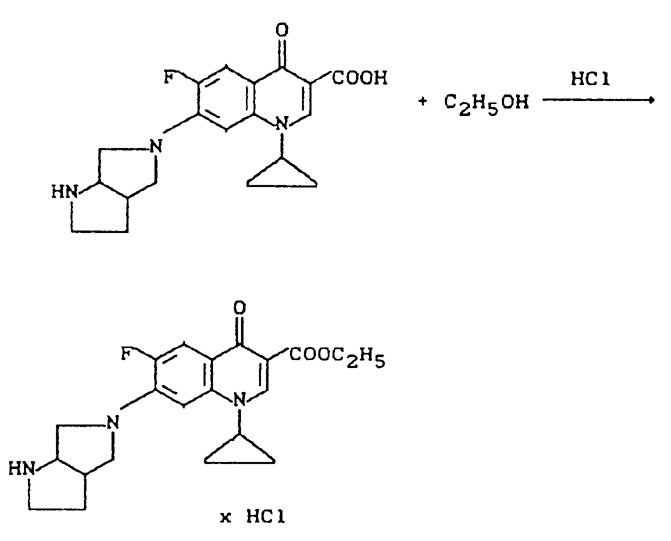

Die als Ausgangsstoffe verwendeten Verbindungen der Formel (II) sind bekannt oder können nach bekannten Methoden hergestellt werden. Als Beispiele seien genannt:
7-Chlor-1-cyclopropyl-6-fluor-1,4-dihydro-4-oxo-3-chinolincarbonsäure (Deutsche Patentanmeldung 3 142 854),
1-Cyclopropyl-6,7-difluor-1,4-dihydro-4-oxo-3-chinolincarbonsäure (Europäische Patentanmeldung 113091),
6-Chlor-1-cyclopropyl-7,8-difluor-1,4-dihydro-4-oxo-3-chinolincarbonsäure (Deutsche Patentanmeldung 3 420 743),
8-Chlor-1-cyclopropyl-6,7-difluor-1,4-dihydro-4-oxo-3-chinolincarbonsäure (Deutsche Patentanmeldung 3 420 743),
1-Cyclopropyl-6,7,8-trifluor-1,4-dihydro-4-oxo-3-chinolincarbonsäure (Deutsche Patentanmeldung 3 318 145),
6,8-Dichlor-1-cyclopropyl-7-fluor-1,4-dihydro-4-oxo-3-chinolincarbonsäure (Deutsche Patentanmeldung 3 420 743),
1-Cyclopropyl-6,7-difluor-1,4-dihydro-8-methyl-4-oxo-3-chinolincarbonsäure,
1-Cyclopropyl-7-chlor-6-fluor-1,4-dihydro-8-nitro-4-oxo-3-chinolincarbonsäure,
6,7-Difluor-1 -ethyl-1,4-dihydro-4-oxo-3-chinolincarbonsäure,
7-Chlor-6-fluor-1-ethyl-1,4-dihydro-4-oxo-3-chinolincarbonsäure,
7-Chlor-6-fluor-1,4-dihydro-1-(2-hydroxyethyl)-4-oxo-3-chinolincarbonsäure,
6,7-Difluor-1-(2-fluorethyl)-1,4-dihydro-4-oxo-3-chinolincarbonsäure,
8-Chlor-1-(2,4-difluorphenyl)-6,7-difluor-1,4-dihydro-4-oxo-3-chinolincarbonsäure (Europäische Patentanmeldung 235 762),
7-Chlor-6-fluor-1,4-dihydro-1-methoxy-4-oxo-3-chinolincarbonsäure,
7-Chlor-6-fluor-1,4-dihydro-1-methylamino-4-oxo-3-chinolincarbonsäure,
6,7-Difluor-1,4-dihydro-4-oxo-1 -phenyl-3-chinolincarbonsäure,
7-Chlor-1-cyclopropyl-6-fluor-1,4-dihydro-4-oxo-1,8-naphthyridin-3-carbonsäure,
6,7-Dichlor-1 -cyclopropyl-1,4-dihydro-4-oxo-1,8-naphthyridin-3-carbonsäure,
1-Cyclopropyl-6,7,8-trifluor-1,4-dihydro-4-oxo-3-chinolincarbonsäureethylester (Deutsche Patentanmeldung 3 318 145),
9,10-Difluor-2,3-dihydro-3-methyl-7-oxo-7H-pyrido-[1,2,3-de][1,4]benzoxacin-6-carbonsäure (Europäische Patentanmeldung 47 005),
8,9-Difluor-6,7-dihydro-5-methyl-1-oxo-1H,5H-benzoli[i;j]-chinolicin-2-carbonsäure,
7-Chlor-6-fluor-1-phenyl-1,4-dihydro-4-oxo-1,8-naphthyridin-3-carbonsäure (Europäische Patentanmeldung 153 580),
7-Chlor-6-fluor-1-(4-fluorphenyl)-1,4-dihydro-4-oxo-1,8-naphthydrin-3-carbonsäure (Europäische Patentanmeldung 153 580),
6,7,8-Trifluor-1,4-dihydro-1-methylamino-4-oxo-3-chinolincarbonsäure (Deutsche Patentanmeldung 3 409 922),
1-Amino-6,7,8-trifluor-1,4-dihydro-4-oxo-3-chinolincarbonsäure (Deutsche Patentanmeldung 3 409 922),
6,7,8-Trifluor-1,4-dihydro-1 -dimethylamino-4-oxo-3-chinolincarbonsäure (Deutsche Patentanmeldung 3 409 922),
7-Chlor-6-fluor-1,4-dihydro-8-nitro-4-oxo-1-phenyl-3-chinolincarbonsäure,
7-Chlor-6-fluor-1-(4-fluorphenyl)-1,4-dihydro-8-nitro-4-oxo-3-chinolincarbonsäure,
6,7-Difluor-1-(4-fluorphenyl)-1,4-dihydro-8-methyl-4-oxo-3-chinolincarbonsäure,
6-Chlor-7-fluor-1-(4-fluorphenyl)-1,4-dihydro-4-oxo-3-chinolincarbonsäure (Europäische Patentanmeldung 131 839),
5,6,7,8-Tetrafluor-1-(2,4-difluorphenyl)-1,4-dihydro-4-oxo-3-chinolincarbonsäure,
5,7-Dichlor-6-fluor-1-(2,4-difluorphenyl)-1,4-dihydro-4-oxo-3-chinolincarbonsäure,
5,7-Dichlor-1-cyclopropyl-6-fluor-1,4-dihydro-4-oxo-3-chinolincarbonsäure,
6-Chlor-7-fluor-1-(2,4-difluorphenyl)-1,4-dihydro-4-oxo-3-chinolincarbonsäure (Europäische Patentanmeldung 131 839),
6,7,8-Trifluor-1-(4-fluorphenyl)-1,4-dihydro-4-oxo-3-chinolincarbonsäure (Europäische Patentanmeldung 154 780),
6,7,8-Trifluor-1-(2,4-difluorphenyl)-1,4-dihydro-4-oxo-3-chinolincarbonsäure (Europäische Patentanmeldung 154 780),
6,7,8-Trifluor-1,4-dihydro-4-oxo-1-phenyl-3-chinolincarbonsäure (Europäische Patentanmeldung 154780),
7-Chlor-1-ethyl-6-fluor-1,4-dihydro-4-oxo-1,8-naphthyridin-3-carbonsäure,
6,7-Difluor-1,4-dihydro-4-oxo-1 -vinyl -3-chinolincarbonsäure,
1-Cyclopropyl-5,6,7,8-tetrafluor-1,4-dihydro-4-oxo-3-chinolincarbonsaure,
5-Amino-1-cyclopropyl-6,7,8-trifluor-1,4-dihydro-4-oxo-chinolincarbonsäure,
1-Cyclopropyl-6,7,8-trifluor-1,4-dihydro-5-hydroxy-4-oxo-3-chinolincarbonsäure,
1-Cyclopropyl-6,7-difluor-1,4-dihydro-8-methoxy-4-oxo-3-chinolincarbonsäure.

Die als Ausgangsbverbindungen verwendeten Verbindungen der Formel (III) sind zum Teil neu. Sie können nach folgenden Verfahren hergestellt werden.
1. Ausgehend von dem N-geschützten 3,4-Epoxypyrrolidin (1) (Deutsche Offenlegungsschrift 1 929 237, US-Patent 4 254 135), das gegebenenfalls noch eine oder zwei Methyl- oder Phenylreste tragen kann, werden die Ausgangsverbindungen der Formel (III) hergestellt.
   R9 = Benzyl, Acyl, Alkoxycarbonyl, Benzyloxycarbonyl, Trialkylsilyl, Sulfonyl (Beispiele für Schutzgruppen),
   X3 = Abgangsgruppe wie Halogen, Alkyl- oder Arylsulfonyloxy

   Aus den 3,4-Epoxypyrrolidinen (1) erhält man über eine Cyclisierung mit Thionylchlorid die Ausgangsverbindungen der Formel (III h): 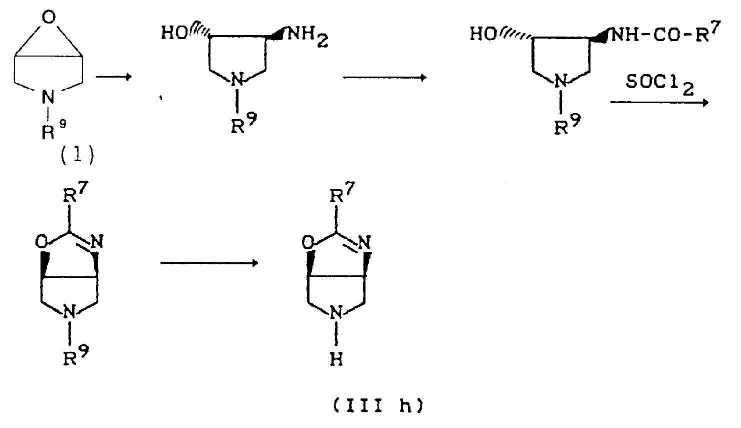
2. Durch Umsetzen der 3,4-Epoxypyrrolidine (1) mit Ethanolaminen erhält man durch intramolekulare Veretherung die Ausgangsverbindungen der Formel (III i): 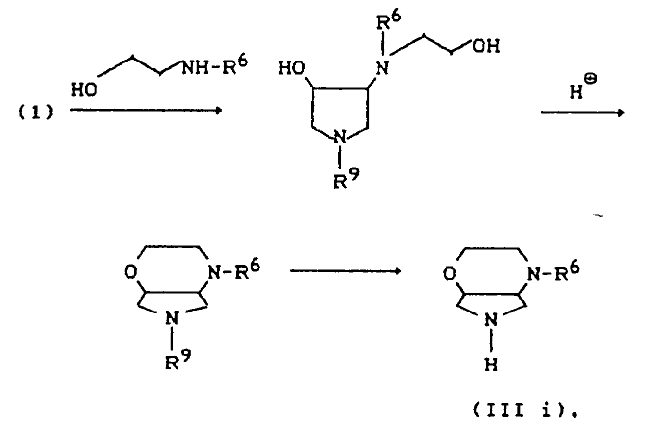
3. Die Ausgangsverbindungen der Formel (III j) erhält man aus Aminoacetaldehyddimethylacetal über eine intramolekulare 1,3-dipolare Cycloaddition. 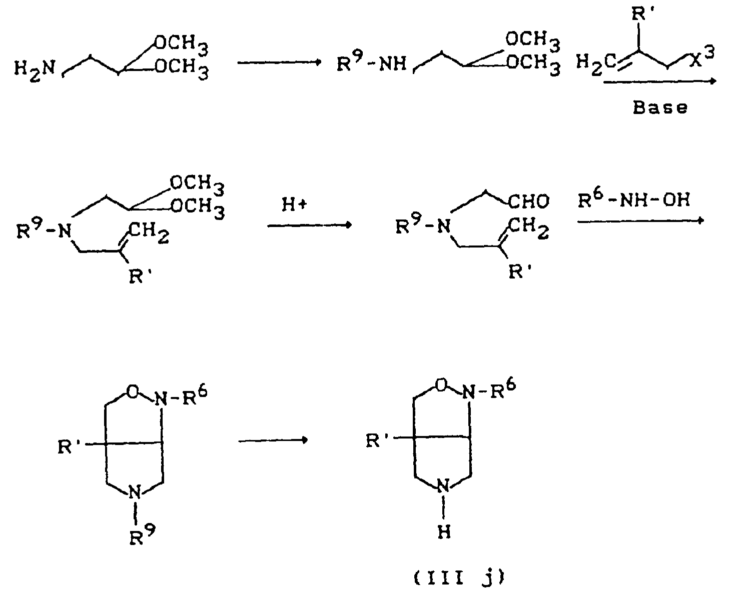
4. Ausgehend von Pyridin-2,3-dicarbonsäure-N-benzylimid werden Ausgangsverbindungen (III k) beziehungsweise (III 1) über die angegebenen Reaktionsschritte hergestellt. 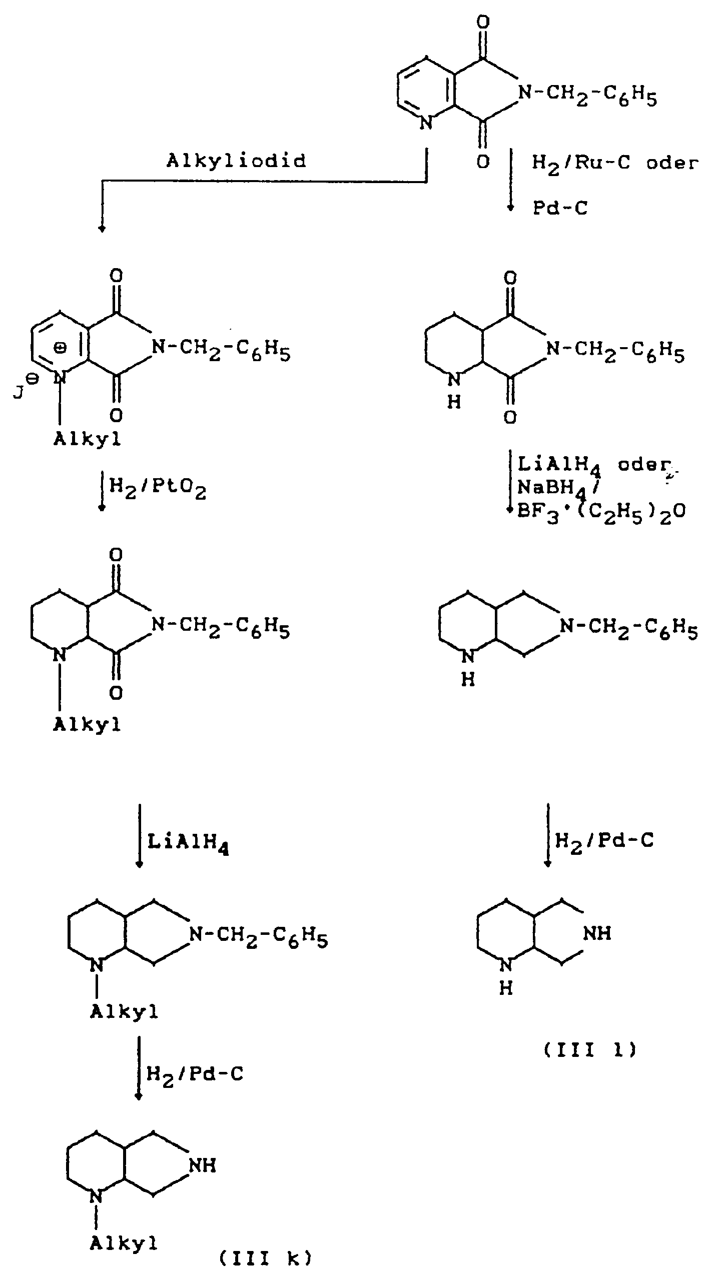
5. N-Benzyl-maleinsäureimid addiert 2-Chlorethylamine zu den 3-(2-chlorethylamino)-succinimiden, die zu den Ausgangsverbindungen der Formel (III m) umgesetzt werden: 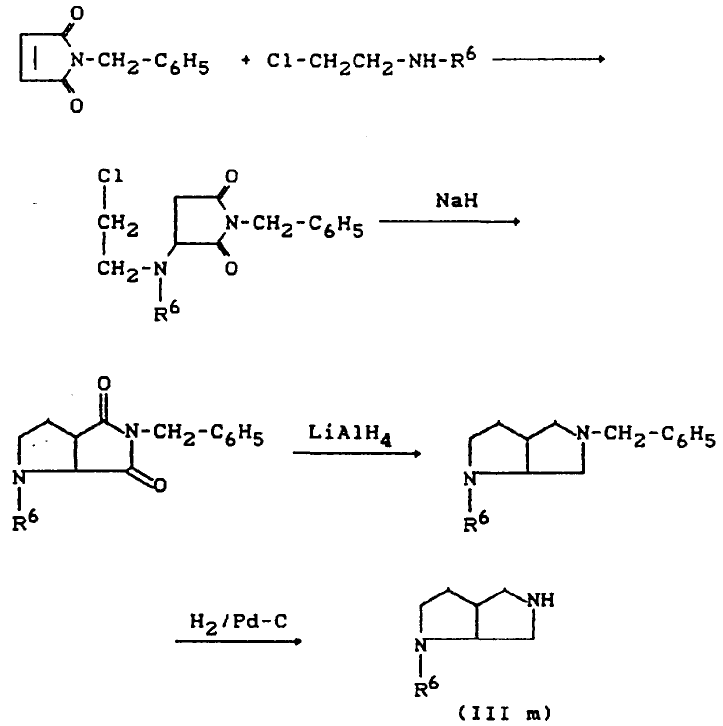
6. 2-Methyl-2-propenal-dimethylhydrazon reagiert mit N-Benzylmaleinsäureimid zu einem Cycloaddukt, welches nach der angegebenen Reaktionsfolge in die Ausgangsverbindung (III n) überführt werden kann.

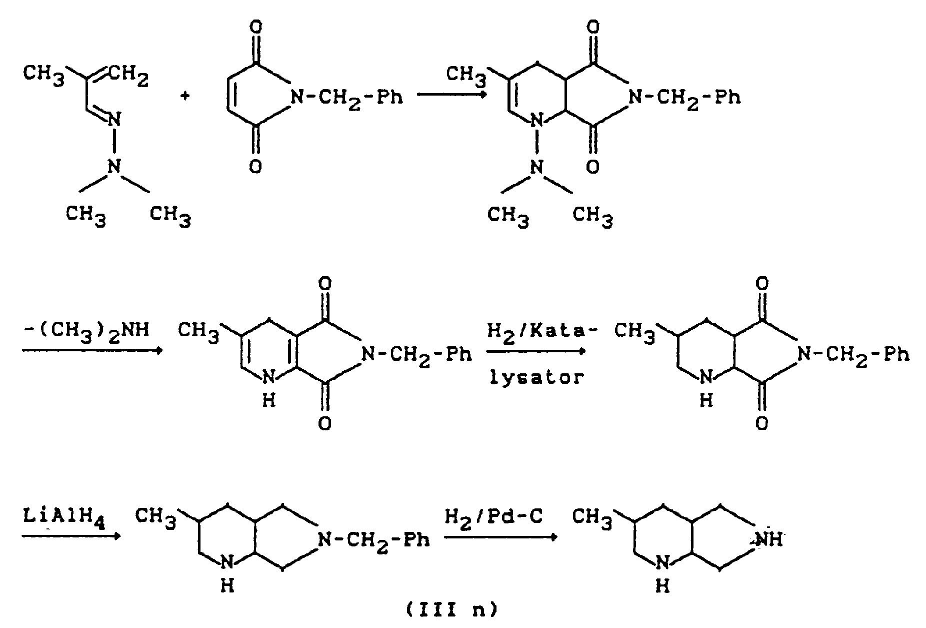

Nach diesem allgemeinen Formelschema lassen sich zum Beispiel die folgenden Ausgangsverbindungen herstellen. Sie können als Diastereomerengemische, in diastereomerenreiner und auch enantionmerenreiner Form hergestellt und eingesetzt werden.
3-Amino-4-methoxy-2-phenylpyrrolidin,
4-Methoxy-3-methylamino-5-phenylpyrrolidin,
3-Methyl-2,7-diazabicyclo[3.3.0]octan,
4-Methyl-2,7-diazabicyclo[3.3.0]octan,
5-Methyl-2,7-diazabicyclo[3.3.0]octan,
3,5-Dimethyl-2,7-diazabicyclo[3.3.0]octan,
1,5-Dimethyl-2,7-diazabicyclo[3.3.0]octan,
2-Oxa-4,7-diazabicyclo[3.3.0]octan,
3,3-Dimethyl-2-oxa-4,7-diazabicyclo[3.3.0]octan,
3-Oxa-2,7-diazabicyclo[3.3.0]octan,
1,2-Dimethyl-3-oxa-2,7-diazabicyclo[3.3.0]octan,
2,5-Dimethyl-3-oxa-2,7-diazabicyclo[3.3.0]octan,
2,8-Dimethyl-3-oxa-2,7-diazabicyclo[3.3.0]octan,
5-Methyl-3-oxa-2,7-diazabicyclo[3.3.0]octan,
2-Oxa-4,7-diazabicyclo[3.3.0]oct-3-en,
3-Methyl-2-oxa-4,7-diazabicyclo[3.3.0]oct-3-en,
3-Phenyl-2-oxa-4,7-diazabicyclo[3.3.0]oct-3-en,
6-Methyl-2-oxa-4,7-diazabicyclo[3.3.0]oct-3-en,
8-Methyl-2-oxa-4,7-diazabicyclo[3.3.0]oct-3-en,
3-Methyl-2,8-diazabicyclo[4.3.0]nonan,
4-Methyl-2,8-diazabicyclo[4.3.0]nonan,
5-Methyl-2,8-diazabicyclo[4.3.0]nonan,
6-Methyl-2,8-diazabicyclo[4.3.0]nonan,
3-Methyl-2-oxa-5,8-diazabicyclo[4.3.0]nonan,
4-Methyl-2-oxa-5,8-diazabicyclo[4.3.0]nonan,
1-Methyl-2-oxa-5,8-diazabicyclo[4.3.0]nonan,
3,5-Dimethyl-2-oxa-5,8-diazabicyclo[4.3.0]nonan,
2-Thia-5,8-diazabicyclo[4.3.0]nonan,
5-Methyl-2-thia-5,8-diazabicyclo[4.3.0]nonan,
3,5-Dimethyl-2-thia-5,8-diazabicyclo[4.3.0]nonan,
3-Oxa-2,8-diazabicyclo[4.3.0]nonan,
2-Methyl-9-oxa-2,8-diazabicyclo[4.3.0]nonan,
4-Methyl-3-oxa-2,8-diazabicyclo[4.3.0]nonan,
2,5-Dimethyl-3-oxa-2,8-diazabicyclo[4.3.0]nonan,
3-Oxa-5,8-diazabicyclo[4.3.0]nonan,
5-Methyl-3-oxa-5,8-diazabicyclo[4.3.0]nonan,
1,5-Dimethyl-3-oxa-5,8-diazabicyclo[4.3.0]nonan,
4,4-Dimethyl-3-oxa-5,8-diazabicyclo[4.3.0]nonan,

Die Umsetzung von (11) mit (III) gemäß Methode A, bei der die Verbindungen (III) auch in Form ihrer Hydrochloride eingesetzt werden können, wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden,

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5,4,0] undec-7-en (DBU) oder überschüssiges Amin (III).

Die Reaktionstemperaturen können in einem größeren Bereich variiert werden Im allgemeinen arbeitet man zwischen etwa 20 und 200°C, vorzugsweise zwischen 80 und 180°C.

Die Umsetzung kann bei Normaldruck, aber auch bei erhöhtem Druck durchgeführt werden. Im allgemeinen arbeitet man bei Drucken zwischen etwa 1 und 100 bar, vorzugsweise zwischen 1 und 10 bar.

Bei der Durchführung des erfindungsgemäßen Verfahrens setzt man auf 1 Mol der Carbonsäure (11) 1 bis 15 Mol, vorzugsweise 1 bis 6 Mol der Verbindung (111) ein.

Freie Hydroxygruppen können während der Umsetzung durch eine geeignete Hydroxyschutzgruppe, zum Beispiel durch den Tetrahydropyranylrest, geschützt und nach Beendigung der Reaktion wieder freigesetzt werden (siehe J.F. W. McOmie, Protective Groups in Organic Chemistry (1973), Seite 104).

Freie Aminofunktionen können während der Umsetzung durch eine geeignete Aminoschutzgruppe, zum Beispiel durch den Ethoxycarbonyl- oder den ter.-Butoxycarbonylrest, geschützt und nach Beendigung der Reaktion durch Behandlung mit einer geeigneten Säure wie Chlorwasserstoffsäure oder Trifluoresigsäure wieder freigesetzt werden (siehe Houben-Weyl, Methoden der oganischen Chemie, Band E4, Seite 144 (1983); J.F.W. McOmie, Protective Groups in Organic Chemistry (1973), Seite 43).

Die Umsetzung von (IV) mit (V) gemäß Methode B wird vorzugsweise in einem Verdünnungsmittel wie Dimethylsulfoxid, Dioxan, N,N-Dimethylformamid, N-Methylpyrrolidon, Hexamethyl-phosphorsäure-trisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden

Als Säurebinder können alle üblichen anorganischen und organischen Säurebindemittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo-[2.2.2]octan (DABCO) oder 1,8-Diazabicyclo[5.4.0)undec-7-en (DBU).

Die Reaktionstemperaturen können in einem größeren Bereich variiert werden. Im allgemeinen arbeitet man zwischen etwa 70 und etwa 200°C, vorzugsweise zwischen 100 und 180°C.

Die Umsetzung kann bei Normaldruck, aber auch bei erhöhtem Druck durchgeführt werden. Im allgemeinen arbeitet man bei Drucken zwischen etwa 1 bar und etwa 100 bar, vorzugsweise zwischen 1 und 10 bar.

Bei der Durchführung des erfindungsgemäßen Verfahrens gemäß Methode B setzt man auf 1 Mol der Verbindung (IV) 1 bis 50 Mol, vorzugsweise 1 bis 30 Mol der Verbindung (V) ein.

Zur Herstellung der erfindungsgemäßen Ester wird die zugrundeliegende Carbonsäure vorzugsweise in überschüssigem Alkohol in Gegenwart von starken Säuren, wie Schwefelsäure, wasserfreiem Chlorwasserstoff, Methansulfonsäure, p-Toluolsulfonsäure oder sauren Ionenaustauschern, bei Temperaturen von etwa 20° bis 200°C, vorzugsweise etwa 60° bis 120°C umgesetzt. Das entstehende Reaktionswasser kann auch durch azeotrope Destillation mit Chloroform, Tetrachlormethan, Benzol oder Toluol entfernt werden.

Die Herstellung von Estern gelingt auch vorteilhaft durch Erhitzen der zugrundeliegenden Säure mit Dimethylformamiddialkylacetal in einem Lösungsmittel wie Dimethylformamid.

Die als Prodrug verwendeten (5-Methyl-2-oxo-1,3-dioxol-4 yl-methyl)-ester werden durch Umsetzung eines Alkalisalzes der zugrundeliegenden Carbonsäure mit 4-Brommethyl- oder4-Chlormethyl-5-methyl-1 ,3-dioxol-2-on in einem Lösungsmittel wie Dimethylformamid, Dimethylacetamid, N-Methylpyrrolidon, Dimethylsulfoxid oder Tetramethylharnstoff bei Temperaturen von etwa 0° bis 100°C, vorzugsweise 0° bis 50°C, erhalten.

Die Herstellung der Säureadditionssalze der erfindungsgemäßen Verbindungen erfolgt in üblicher Weise zum Beispiel durch Lösen des Betains in überschüssiger wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser oder einem Alkohol wie Glykolmonomethylether erhitzen und anschließend bis zur Trockne eindampfen oder das ausgefallene Salz absaugen. Als pharmazeutisch verwendbare Salze sind beispielsweise die Salze von Salzsäure, Schwefelsäure, Essigsäure, Glykolsäure, Milchsaure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure zu verstehen.

Die Alkali- oder Erdalkalisalze der erfindungsgemäßen Carbonsäuren werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium-oder Calciumsalze. Durch Umsetzung eines Alkali- oder Erdalkalisalzes mit einem geeigneten Silbersalz wie Silbernitrat werden die entsprechenden Silbersalze erhalten.

Außer den in den Beispielen genannten Wirkstoffen können ebenfalls die in Tabelle 1 beispielhaft aufgeführten Verbindungen hergestellt werden, wobei diese Verbindungen sowohl als Diastereomerengemische als auch als diastereomerenreine oder enantiomerenreine Verbindungen vorliegen können.

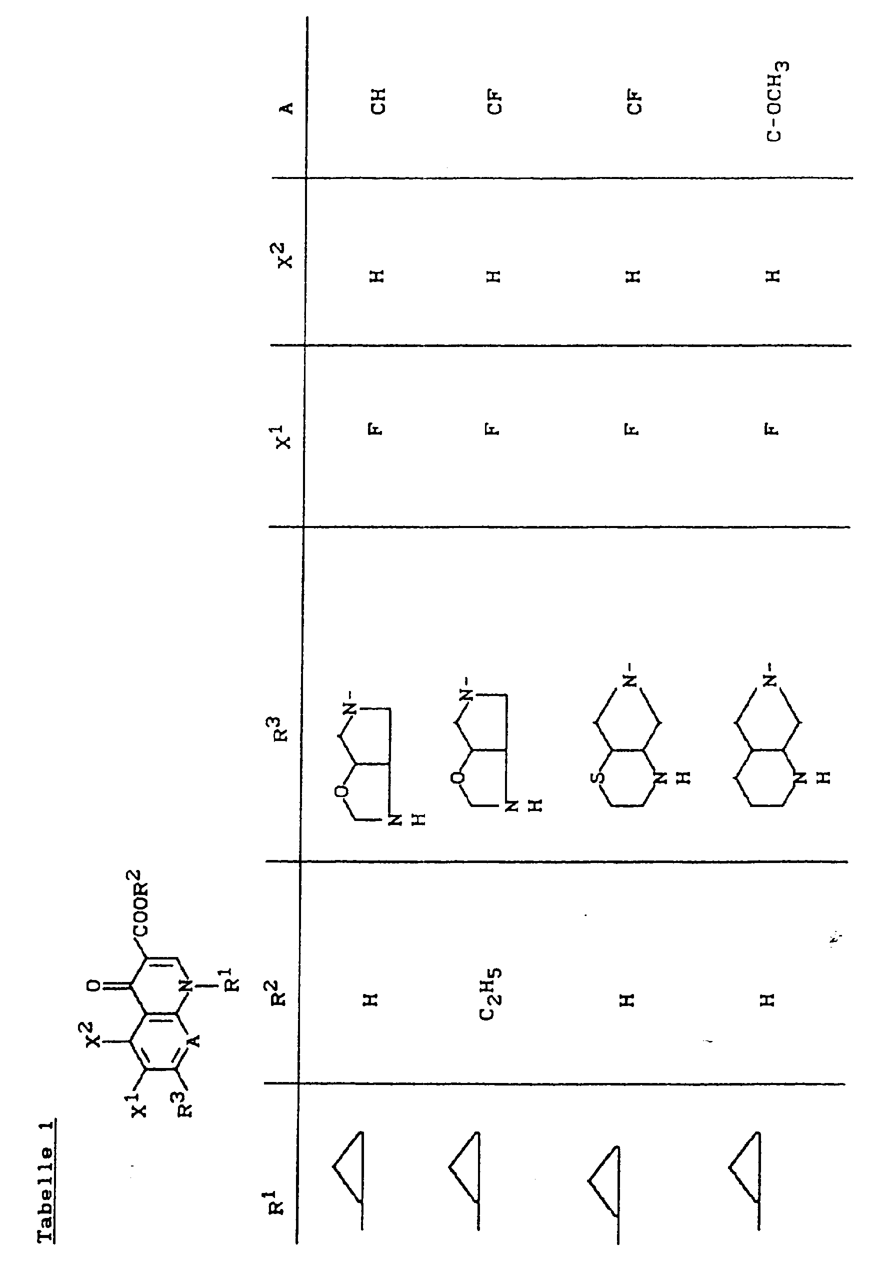

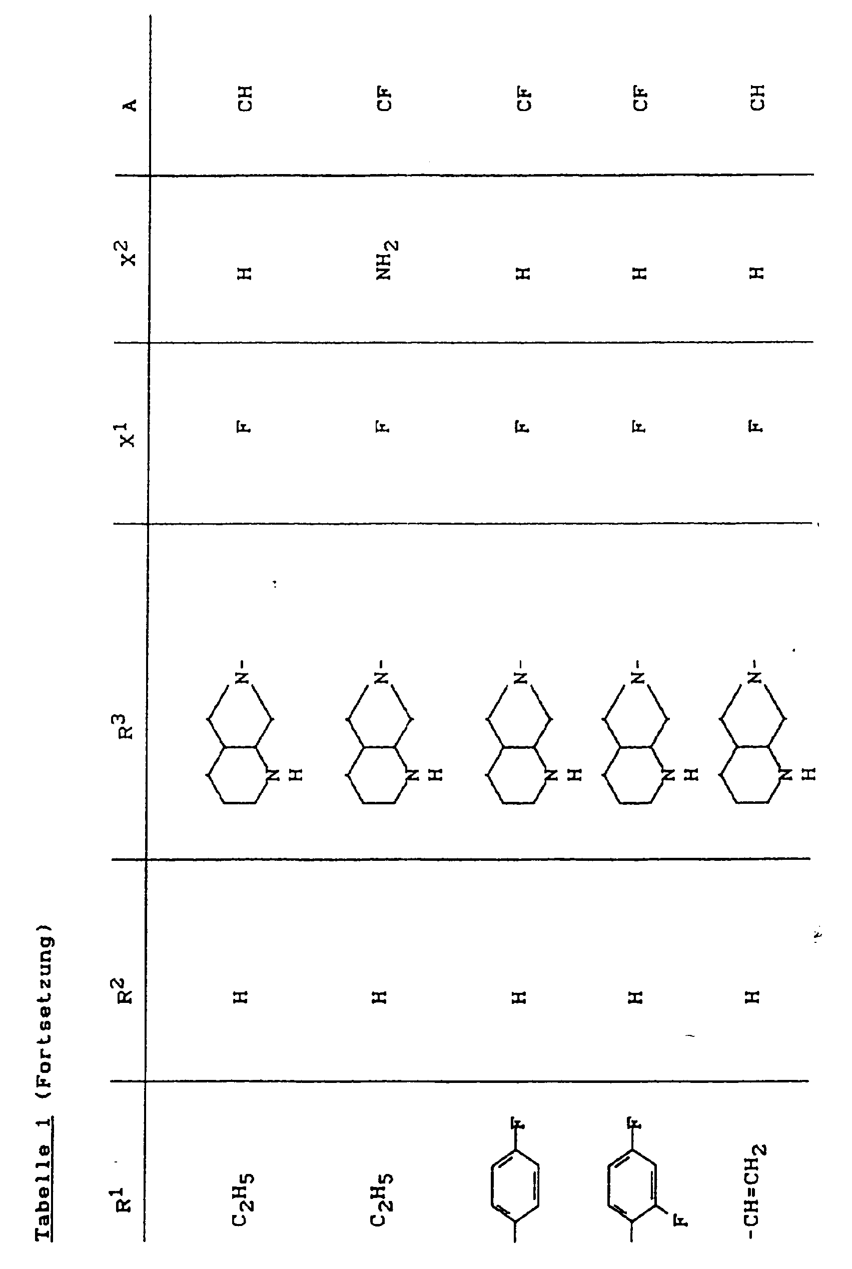

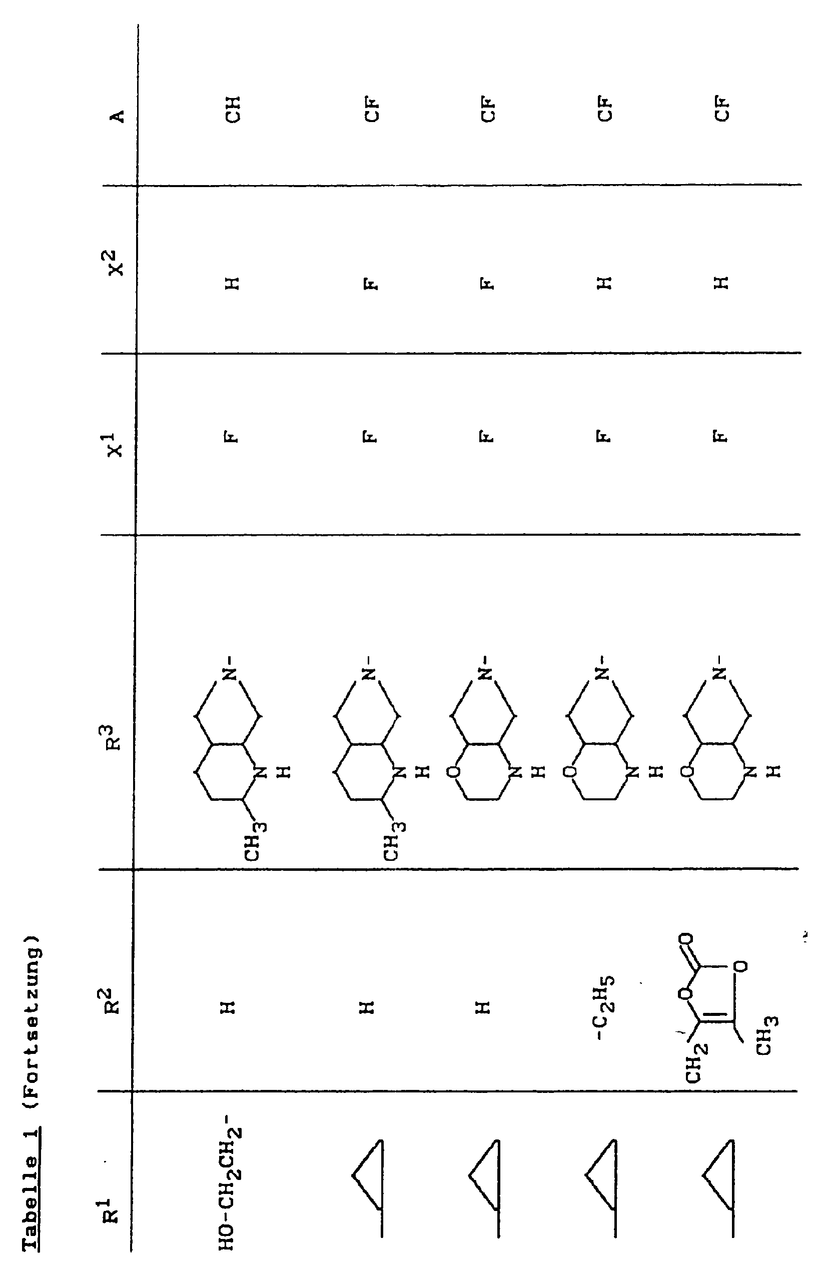

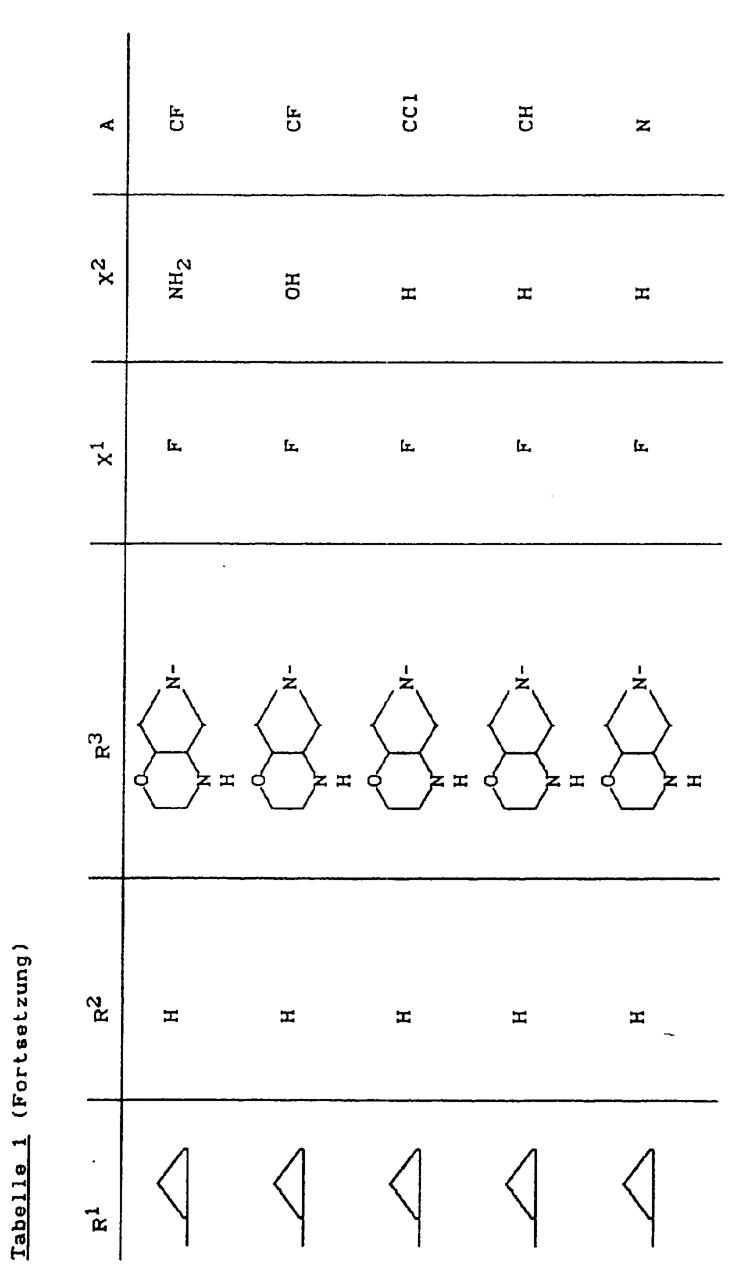

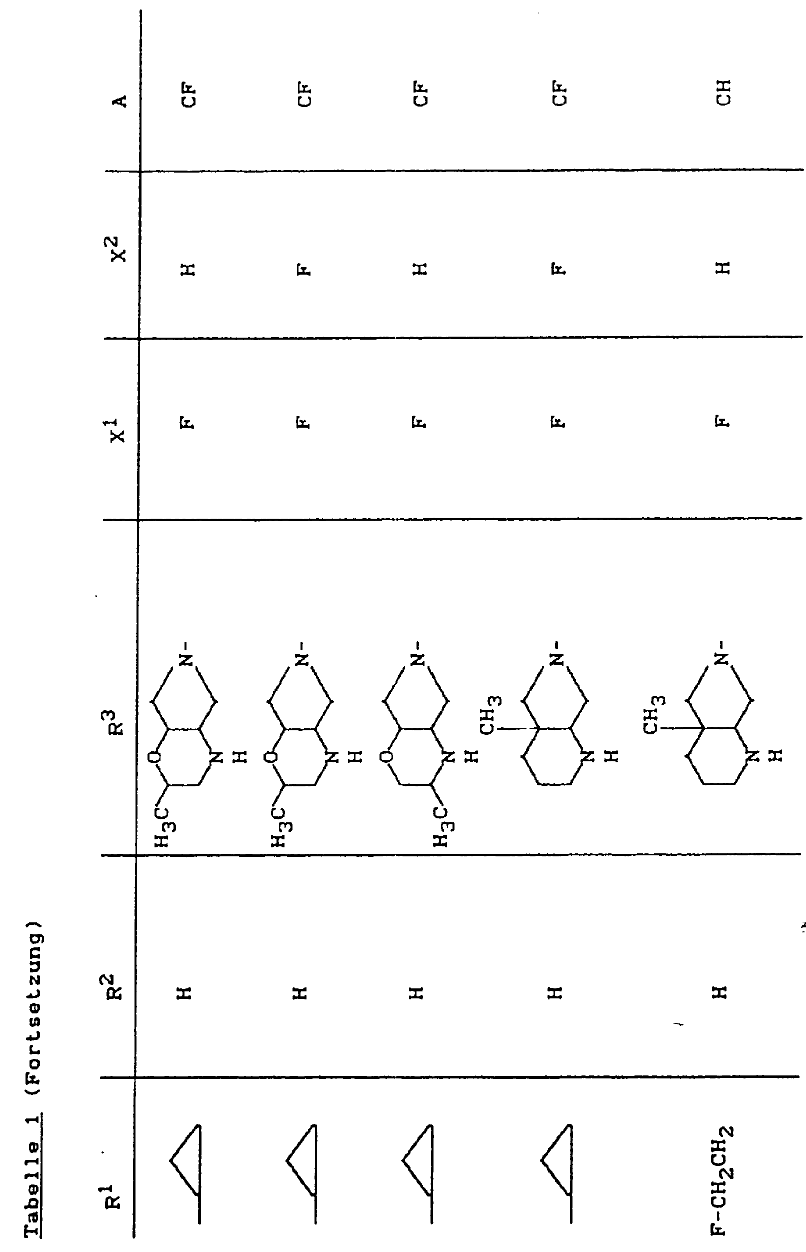

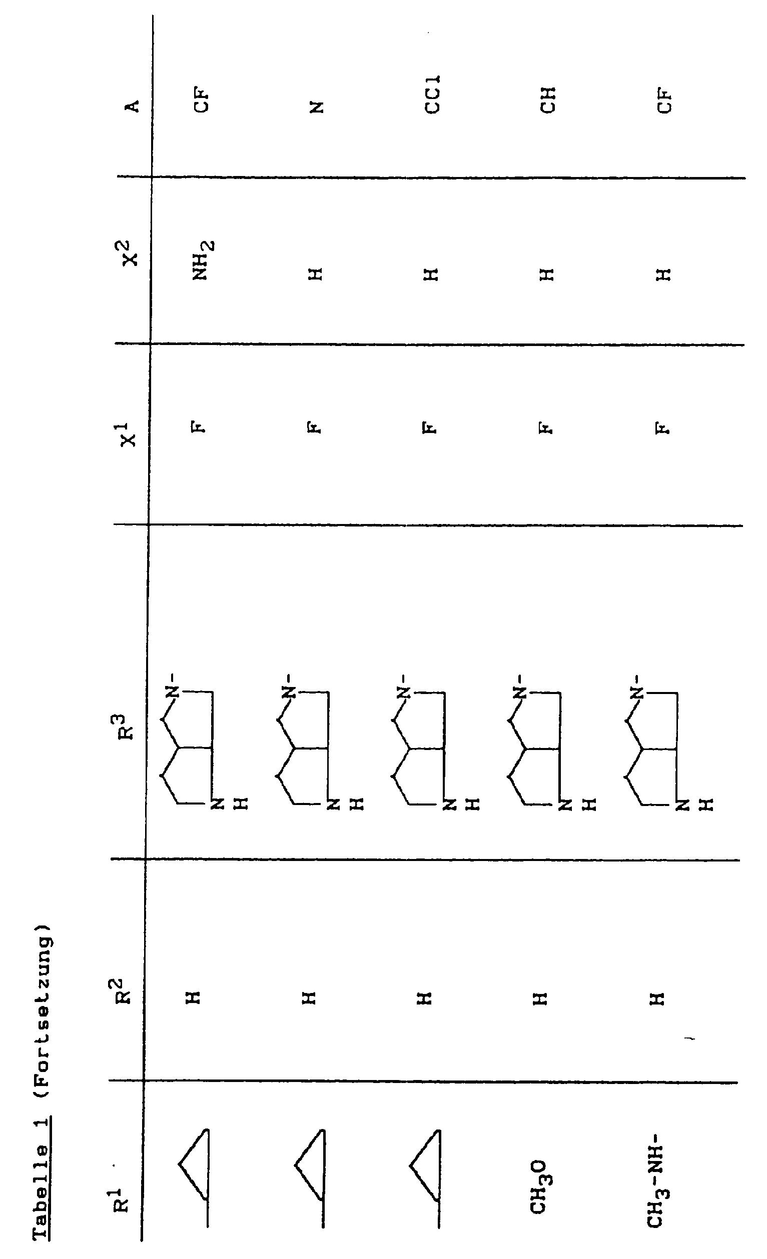

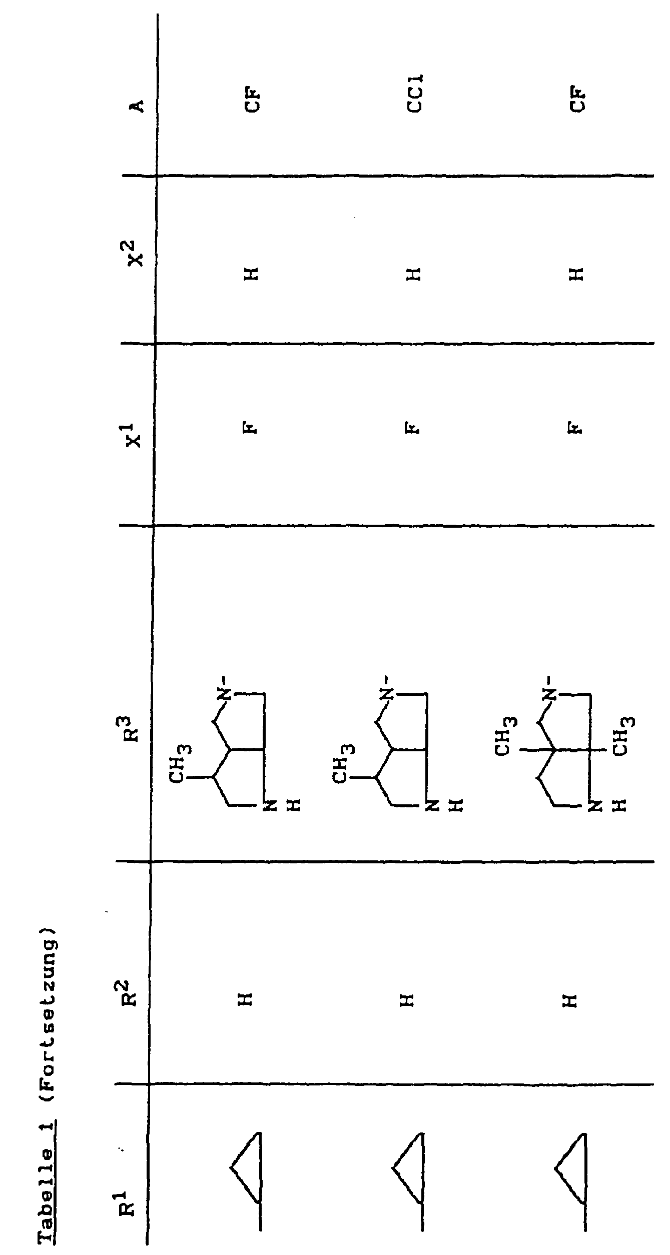

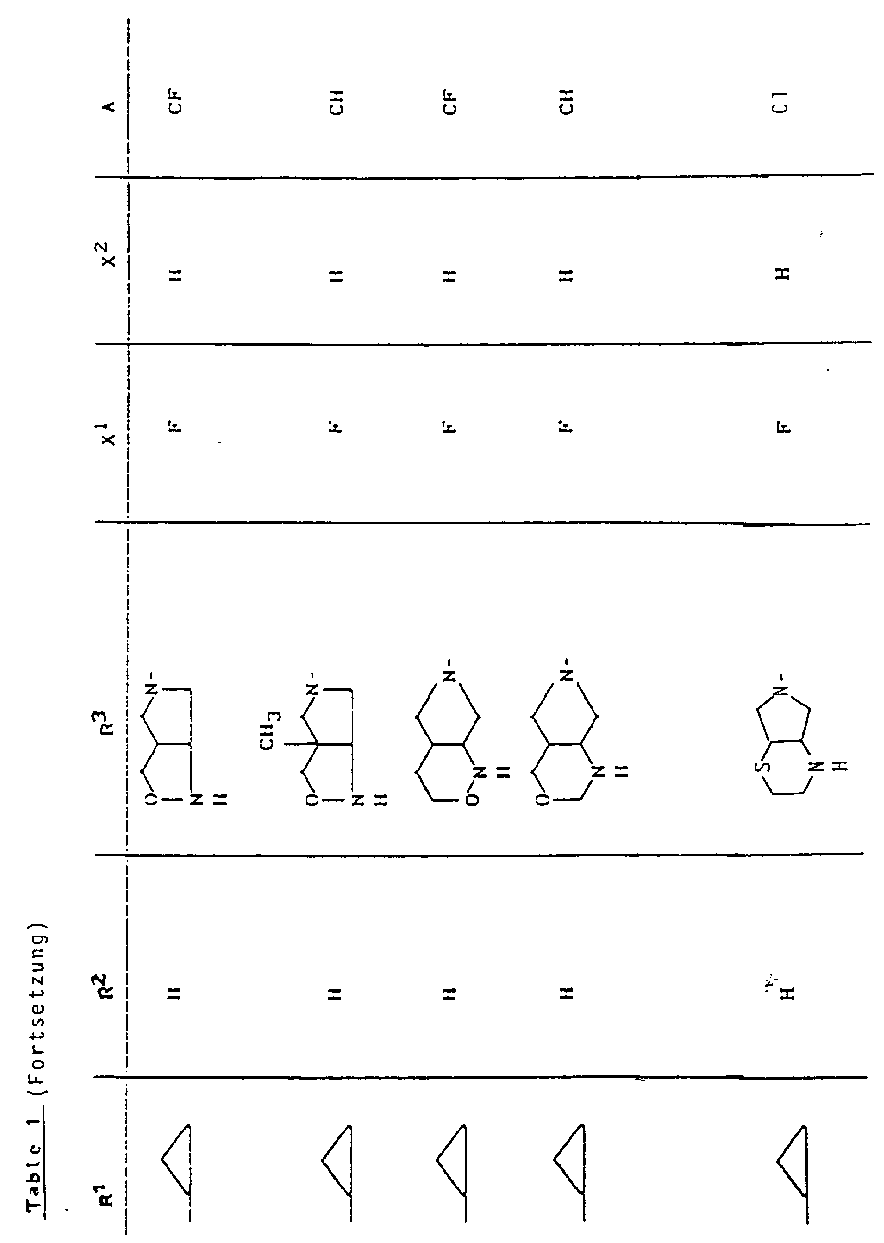

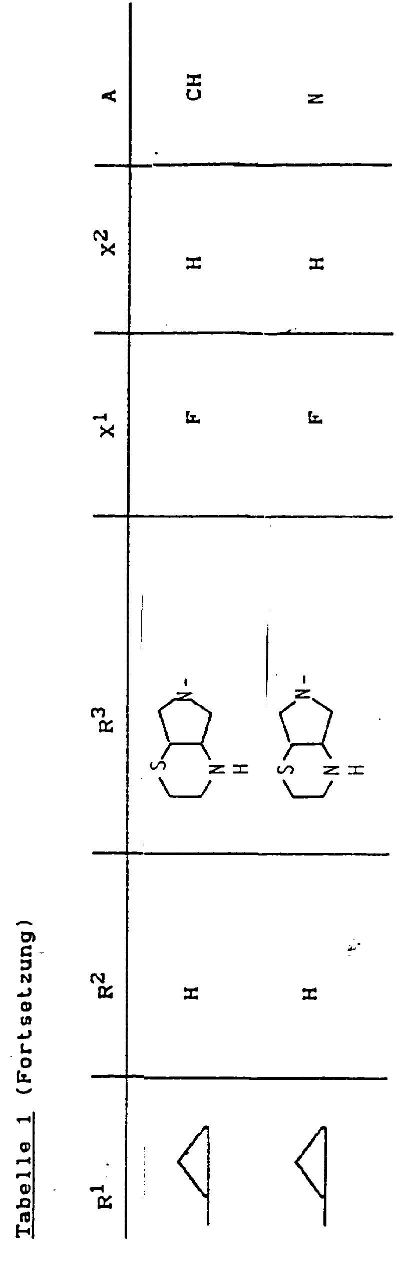

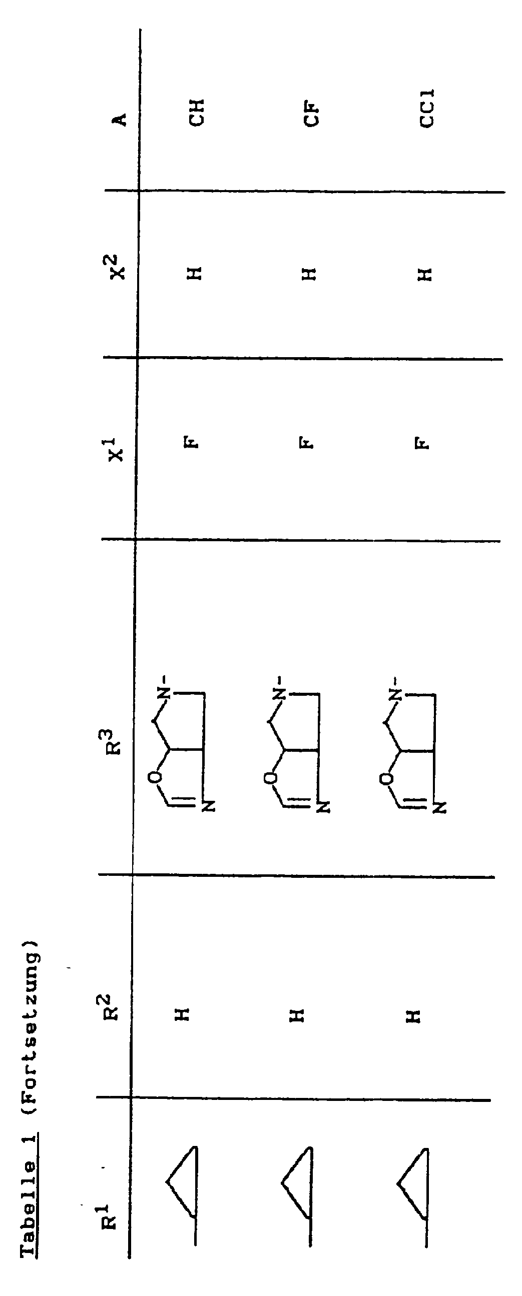

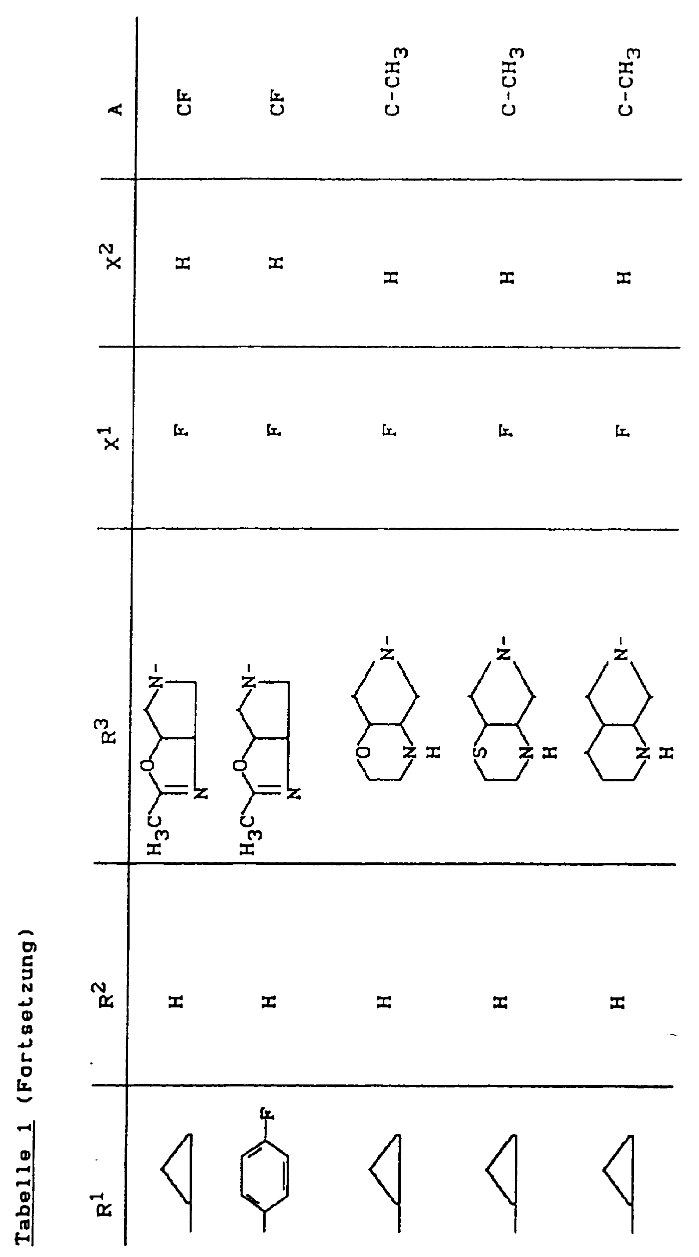

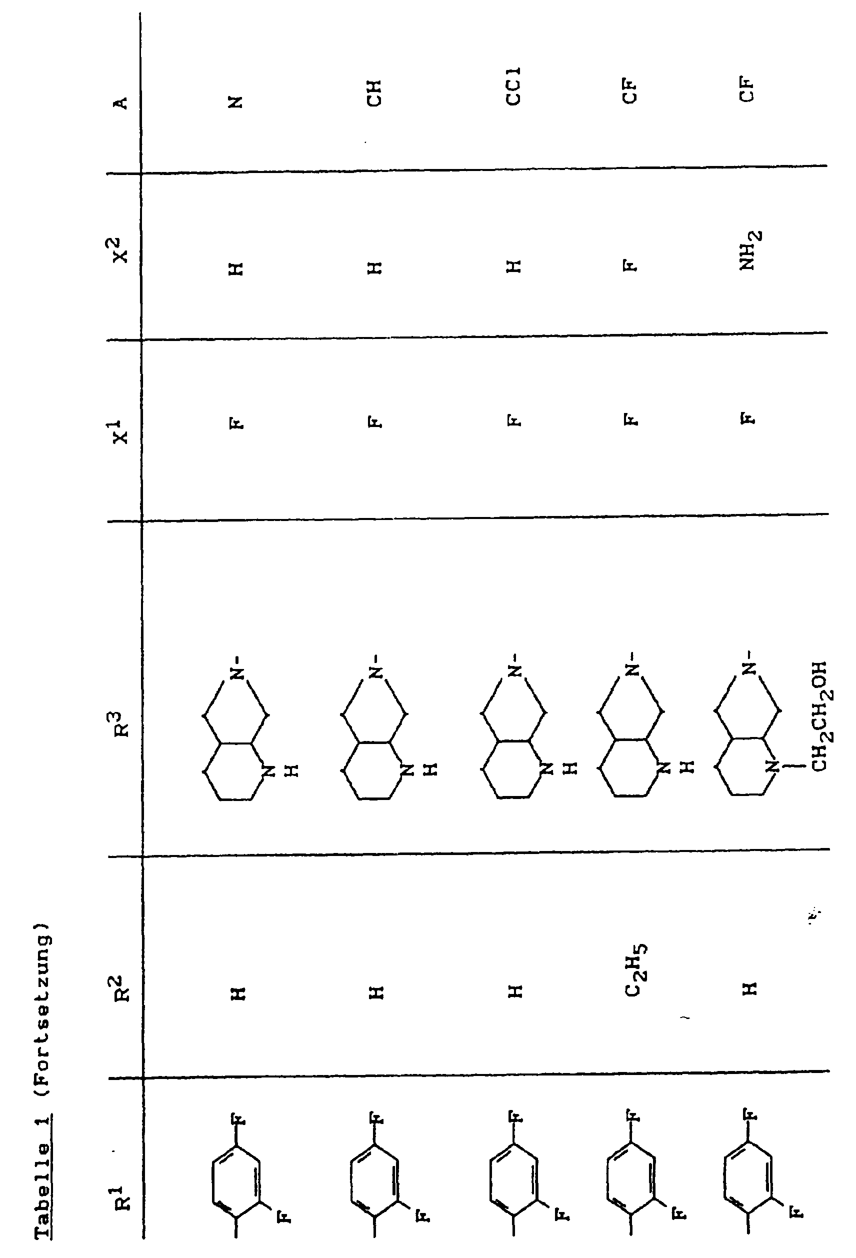

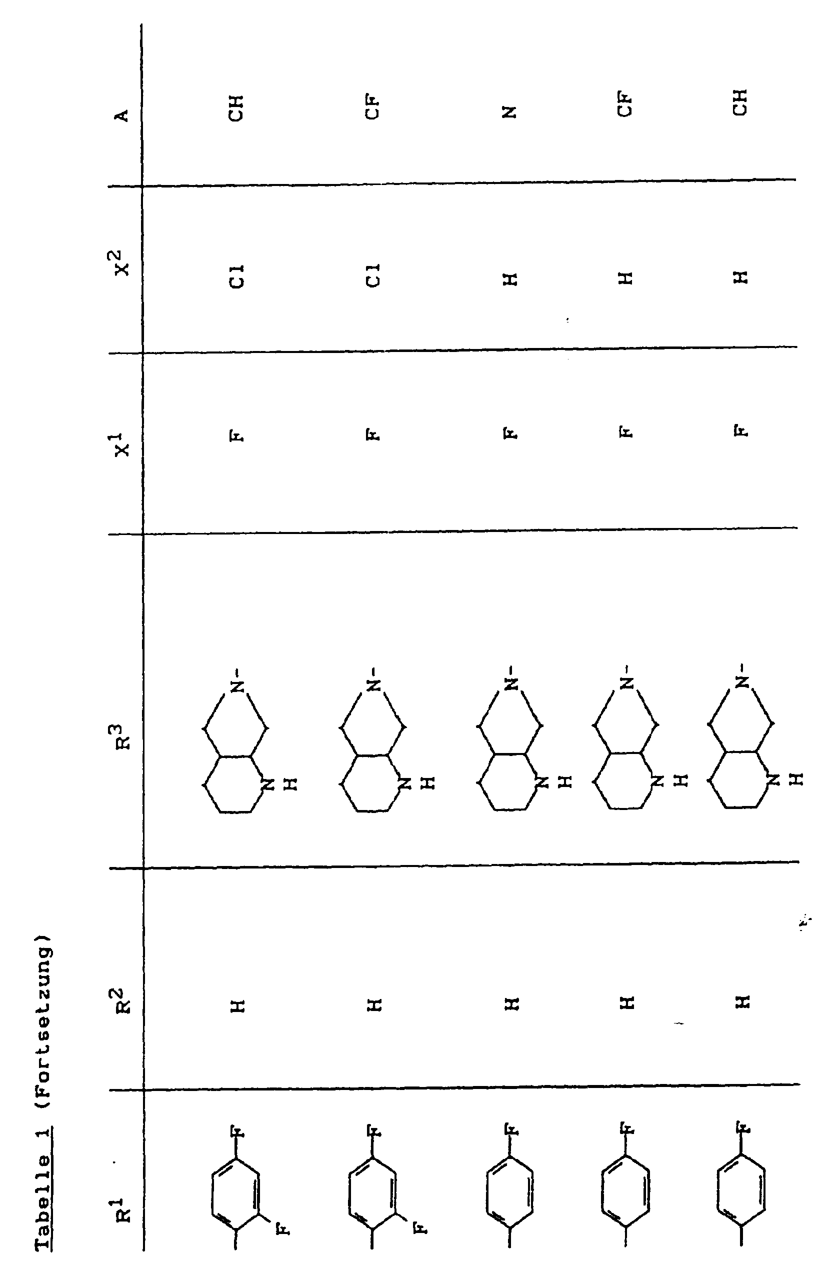

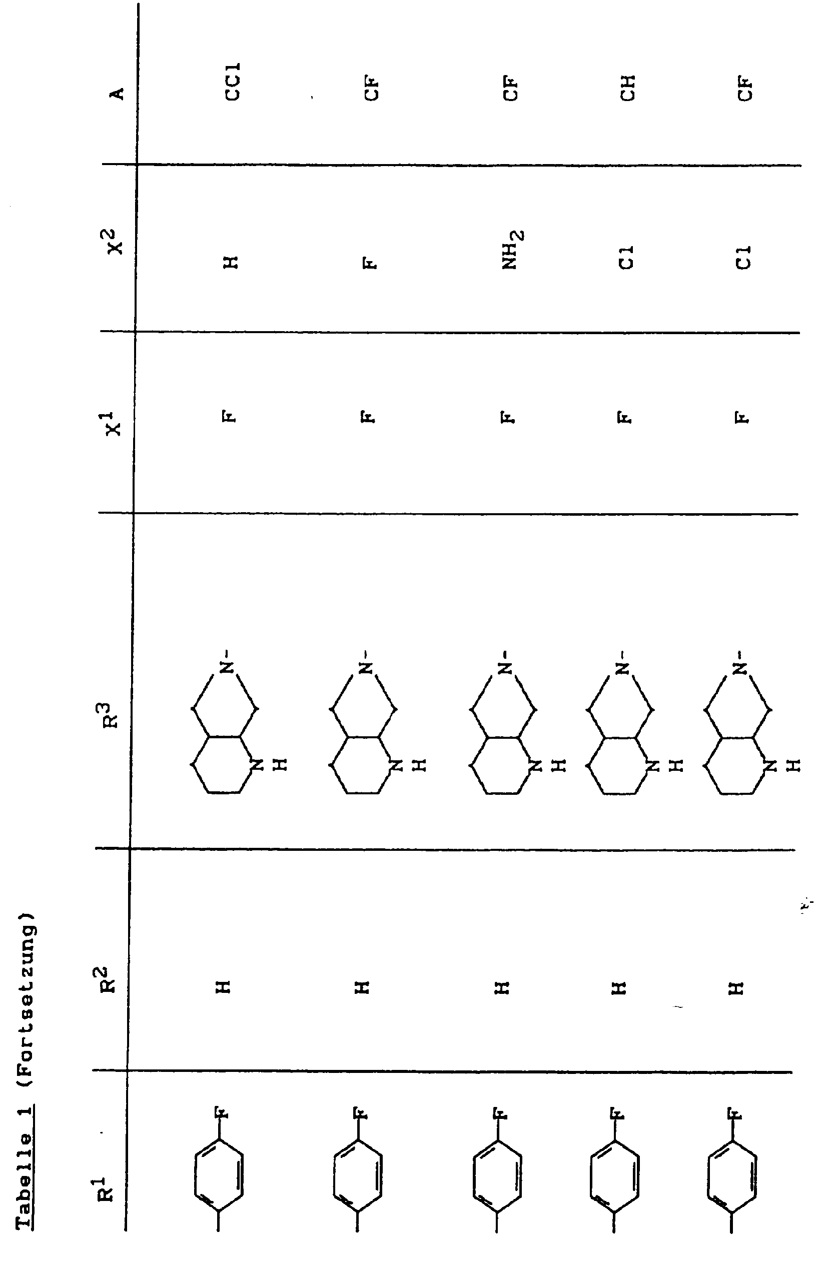

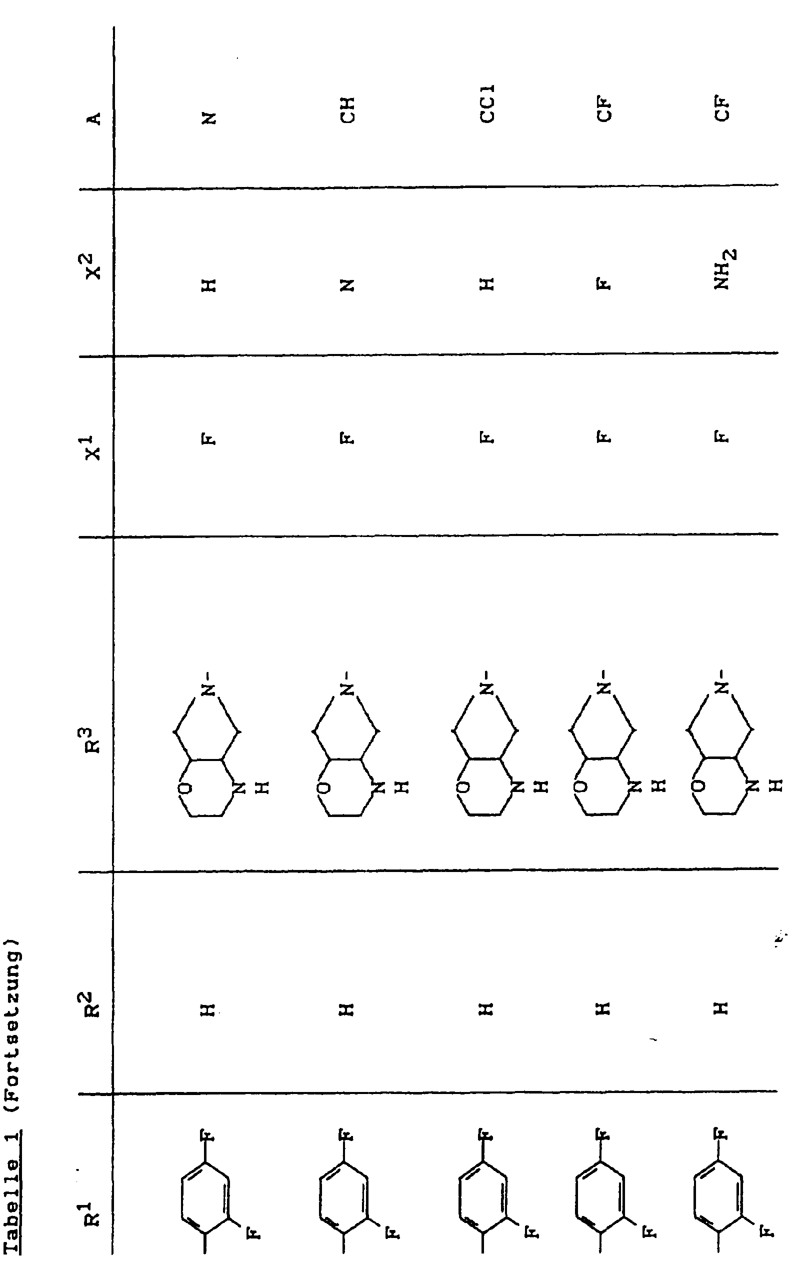

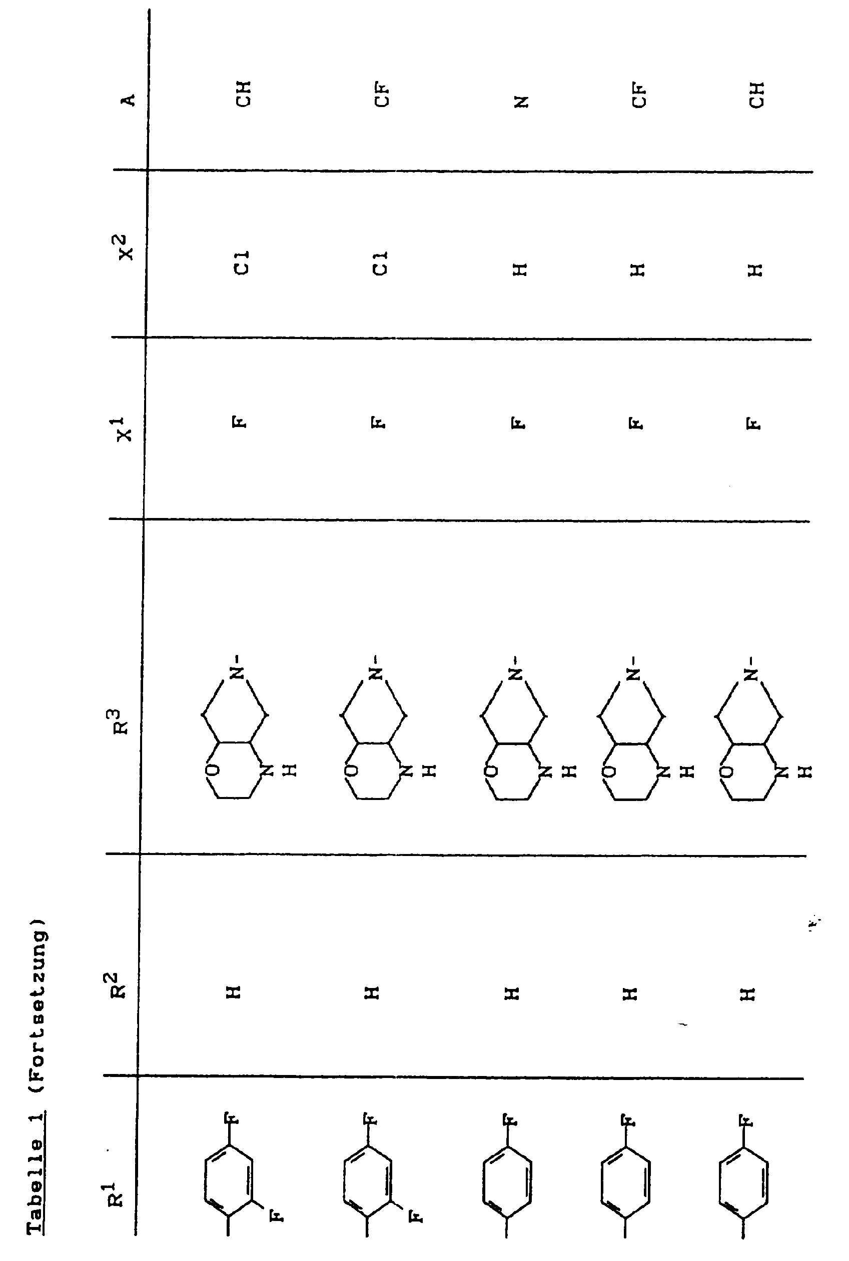

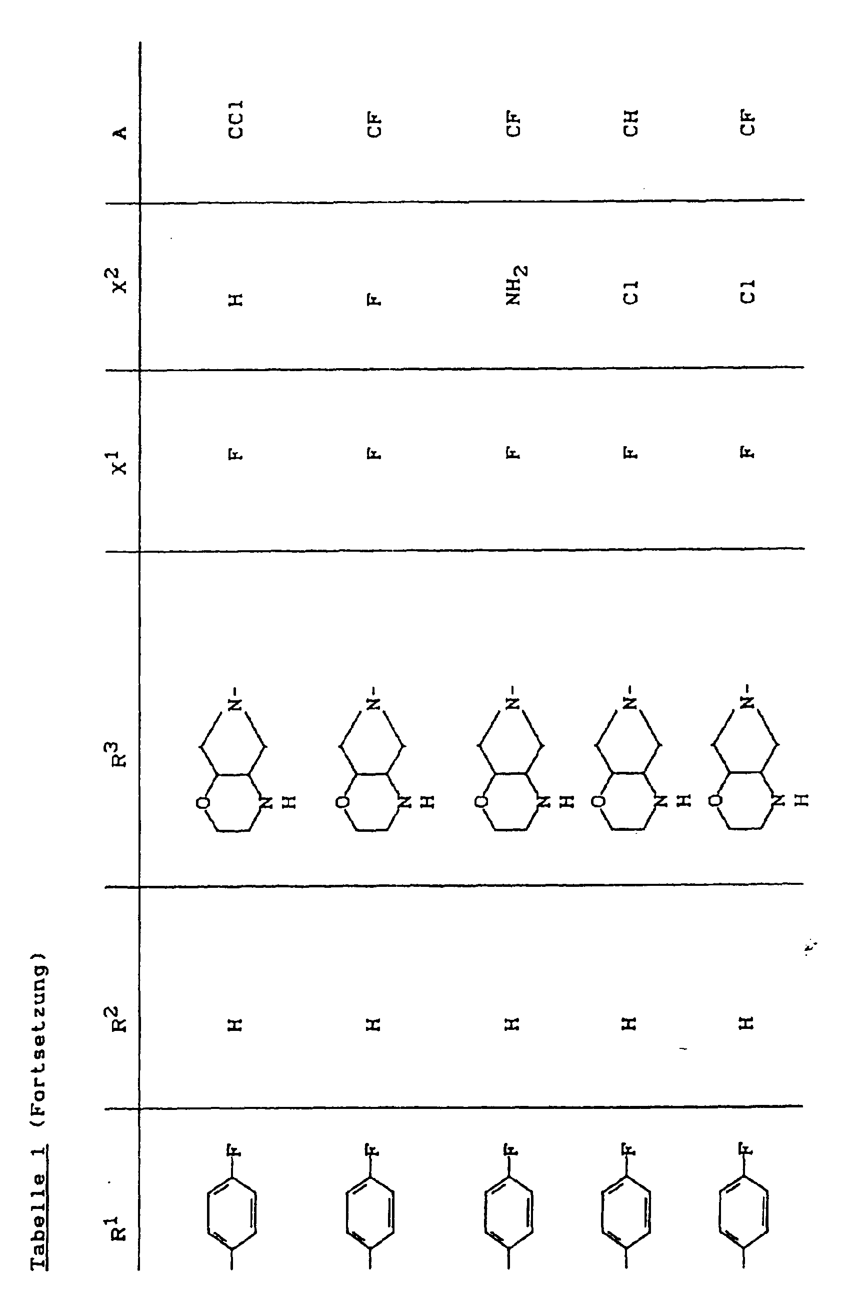

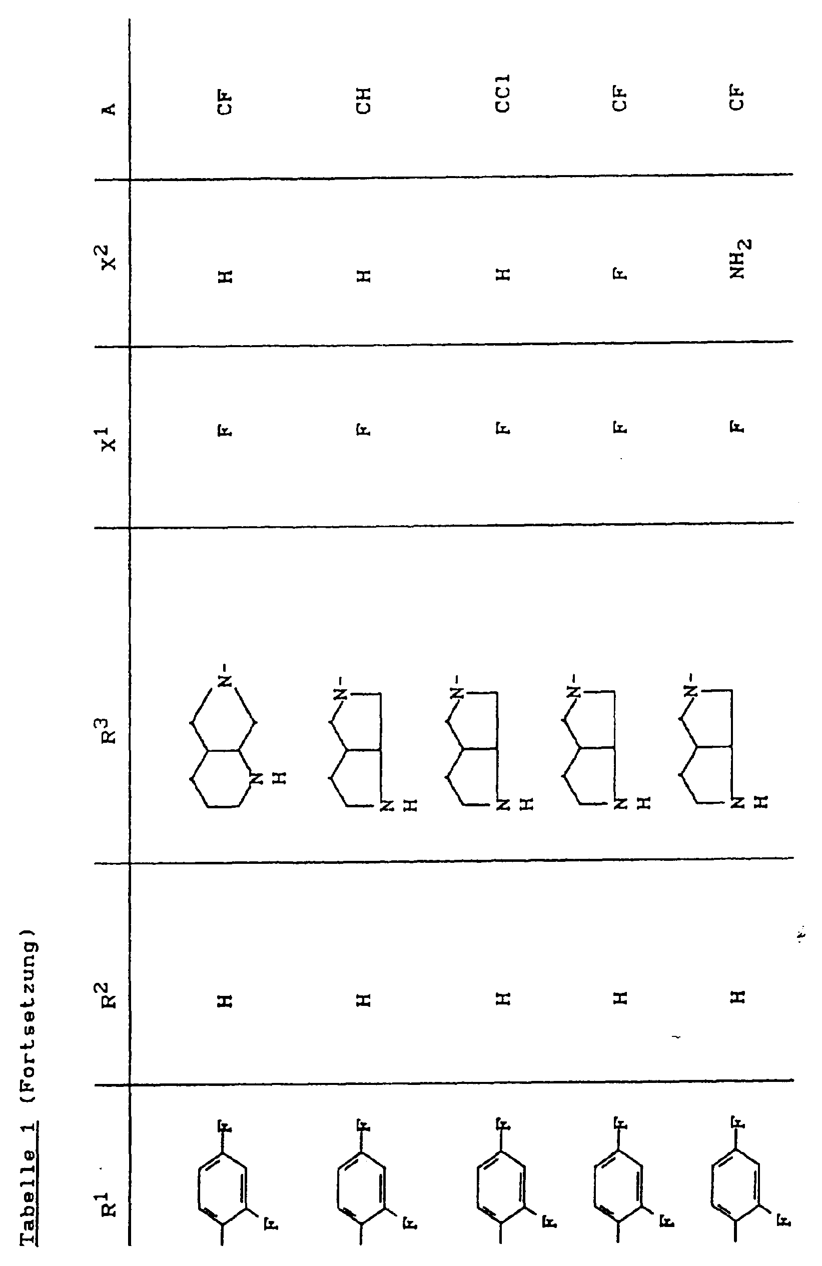

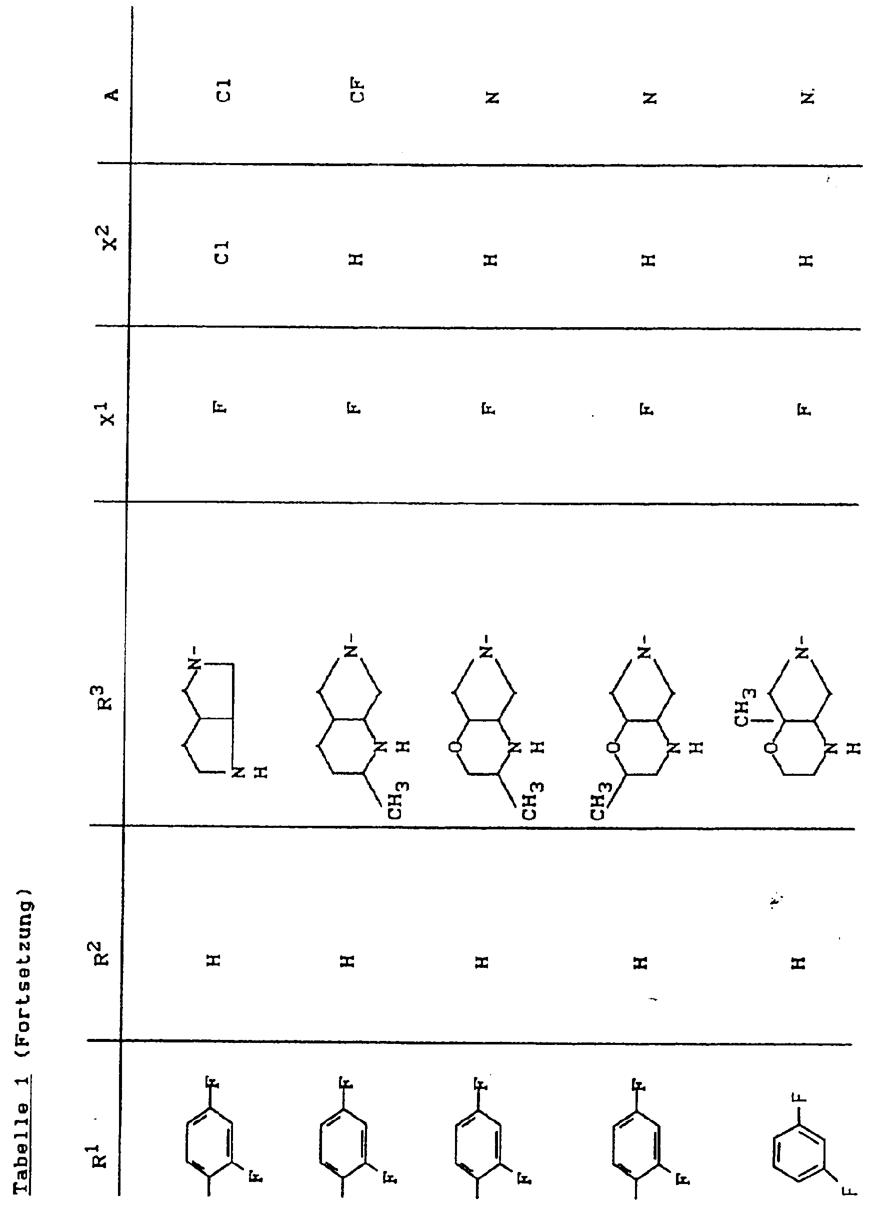

### Beispiel für eine erfindungsgermäße Tablette

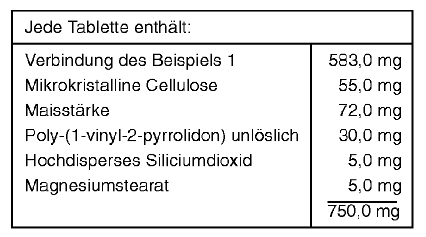

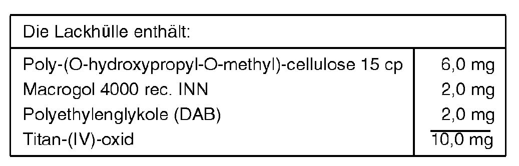

Die erfindungsgemäßen Verbindungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:
Gram-positive Kokken, z.B. Staphylokokken (Staph. aureus, Staph. epidermidis) und Streptokokken (Strept, agalactiae, Strept. faecalis, Strept pneumoniae, Strept. pyogenes); gram-negative Kokken (Neisseria gonorrhoeae) sowie gram-negative Stäbchen wie Enterobakteriaceen, z.B. Escherichia coli, Haemophilus infiuenzae, Citrobacter (Citrob. freundii, Citrob. divernis), Salmonella und Shigella; ferner Klebsiellen (Klebs. pneumoniae, Klebs, oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealylicum) sowie Mykobakterien, z.B. Mycobacterium tuberculosis.

Die obige Aufzählungvon Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seien beispielsweise genannt:
Infektionskrankheiten beim Menschen wie zum Beispiel Otitis, Pharyngitis, Pneumonie, Peritonitis, Pyelonephritis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen-und Gelenkinfektionen, zystische Fibrose, Hautinfektionen, postoperative Wundinfektionen, Abszesse, Phlegmone, Wundinfektionen, infizierte Verbrennungen; Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt:
Schwein: Coli-diarrhoe, Enterotoxämie, Sepsis, Dysenterie, Salmonellose, Mastitis-Metritis-Agalaktie-Syndrom, Mastitis;
Wiederkäuer (Rind, Schaf, Ziege): Diarrhoe, Sepsis, Bronchopneumonie, Salmonellose, Pasteurellose, Mykoplasmose, Genitalinfektionen;
Pferd: Bronchopneumonien, Fohlenlähme, puerperale und postpuerperale Infektionen, Salmonellose;
Hund und Katze: Bronchopneumonie, Diarrhoe, Dermatitis, Otitis, Harnwegsinfekte, Prostatitis;
Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borrelia, Treponema, Nocardia, Rikettsien, Yersinia, erweitert.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z. B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B quarternäre Ammoniumverbindungen, (g) Netzmittel, z B. Cetyl-alkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. C₁₄-Alkohol mit C₁₆-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulveroder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z,B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z B. Pfefferminzöl und Eukalyptusöl und Süßmittel, z B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophthalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

Die minimalen Hemmkonzentrationen (MHK) wurden per Reihenverdünnungsverfahren auf Iso-Sensitest Agar (Oxoid) bestimmt. Für jede Prüfsubstanz wurde eine Reihe von Agarplatten hergestellt, die bei jeweils doppelter Verdünnung abfallende Konzentrationen des Wirkstoffes enthielten. Die Agarplatten wurden mit einem Multipoint-Inokulator (Denley) beimpft. Zum Beimpfen wurden Ubernachtkulturen der Erreger verwandt, die zuvor so verdünnt wurden, daß jeder Impfpunkt ca. 10⁴ koloniebildende Partikel enthielt. Die beimpften Agarplatten wurden bei 37°C bebrütet, und das Keimwachstum wurde nach ca. 20 Stunden abgelesen. Der MHK-Wert (pg/ml) gibt die niedrigste Wirkstoffkonzentration an, bei der mit bloßem Auge kein Keimwachstum zu erkennen war.

In der nachstehenden Tabelle sind die MHK-Werte einiger der erfindungsgemäßen Verbindungen im Vergleich zu Ciprofloxacin angegeben.

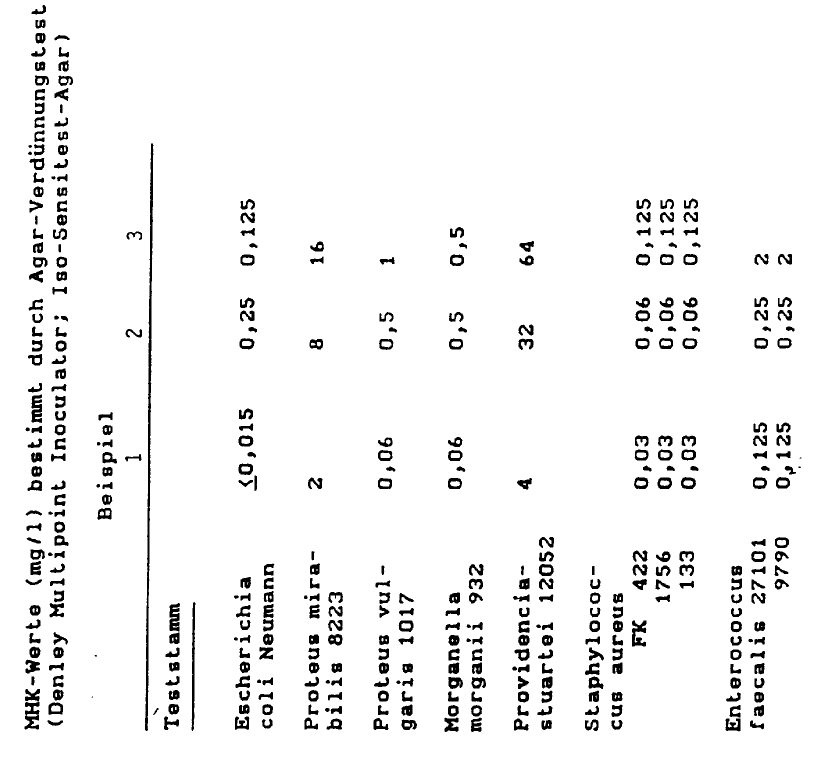

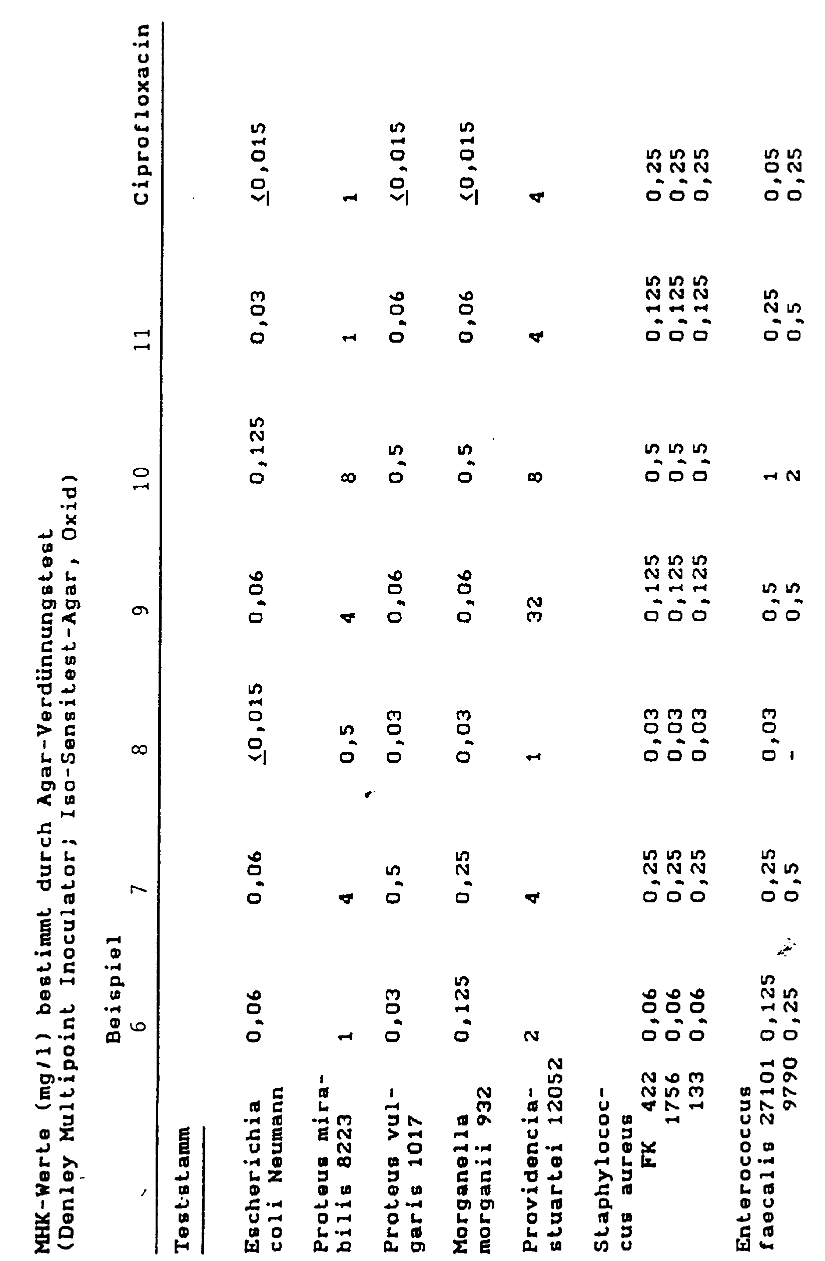

Die folgenden Beispiele erläutern die Erfindung:
Herstellung der Zwischenprodukte:

### Beispiel A

### trans-3-Amino-1-benzyl-4-hydroxy-pyrrolidin

Man erhitzt 8,9 g (50 mmol) 3-Benzyl-6-oxa-3-azabicyclo-[3.1.0]hexan in 75 ml Ammoniaklösung (25%ig) 8 Stunden im Autoklaven auf 120°C. Die Lösung wird eingeengt und der Rückstand destilliert.
Ausbeute: 6 g (62,4% der Theorie)
Siedepunkt: 130-140°C/0,1 mbar
Schmelzpunkt: 82-84°C

### Beispiel B

### trans-1-Benzyl-4-hydroxy-3-(2-hydroxyethylamino)-pyrrolidin

Man erhitzt 40 g (0,22 mol) 3-Benzyl-6-oxa-3-azabicyclo-[3.1.0]hexan mit 42 g (0,68 mol) 2-Aminoethanol in 450 ml Wasser über Nacht unter Rückfluß. Man extrahiert die Lösung einmal mittert.-Butylmethylether und engt die wäßrige Phase ein. Der Rückstand wird destilliert.
Ausbeute: 34,1 g (65,6% der Theorie)
Siedepunkt: 190°C/0,1 mbar

### Beispiel C

### trans-1-Benzyl-4-hydroxy-3-(2-hydroxyethyl-methjylamino)-pyrrolidin

Man setzt 17,5 g (0,1 mol) 3-Benzyl-6-oxa-3-azabicyclo-[3.1.0]hexan mit 17 g (0,1 mol) Methylaminoethanol analog Beispiel D a) in 200 ml Wasser um.
Ausbeute: 18,2 g (73% der Theorie)
Siedepunkt: 180-190°C/0,1 mbar

### Beispiel D

### 2-Oxa-5,8-diazabicyclo[4.3.0]nonan-Dihydrochlorid

### a) 8-Benzyl-2-oxa-5,8-diazabicyclo[4.3.0]nonan

Man erhitzt 15,6 g (66 mmol) 1-Benzyl-4-hydroxy-3-(2-hydroxyethylamino)-pyrrolidin in einem Gemisch aus 60 ml konzentrierter Schwefelsäure und 20 ml Wasser 6 Stunden unter Rückfluß. Man stellt mit konzentrierter Natronlauge alkalisch, saugt ausgeschiedenes Natriumsulfat ab und extrahiert das Filtrat mit Chloroform. Man trocknet über Kaliumcarbonat, engt ein und destilliert den Rückstand.
Ausbeute: 4,1 g (28,5% der Theorie)
Siedepunkt: 122-128°C (0,08 mbar)

### b) 2-Oxa-5,8-diazabicyclo[4.3.0]nonan-Dihydrochlorid

Man hydriert eine Lösung von 4 g (18,2 mmol) 8-Benzyl-2-oxa-5,8-diazabicyclo[4.3.0]nonan in 100 ml Methanol und 3,5 ml konzentrierter Salzsäure an 2 g Palladium-Aktivkohle (10% Pd) bei 80°C und 100 bar. Der Katalysator wird abfiltriert und mit Wasser gewaschen. Die Filtrate werden eingeengt und durch Verreiben mit wenig Methanol kristallisiert. Man saugt ab, wäscht die Kristalle mit Aceton und trocknet an der Luft.
Ausbeute: 1,85 g (51 % der Theorie)
Schmelzpunkt: 280°C unter Zersetzung

### c) 2-Oxa-5,8-diazabicyclo[4.3.0]nonan

Man hydriert 7,2 g (33 mmol) 8-Benzyl-2-oxa-5,8-diazabicyclo[4.3.0]nonan in 400 ml Methanol mit 2,5 g Palladium- Aktivkohle (10 % Pd) bei 50 bar und 100°C. Man saugt den Katalysator ab, engt das Filtrat ein und destilliert den Rückstand
Ausbeute: 3,1 g (73,4 % der Theorie); cis-trans Isomerengemisch 1:7
Siedepunkt: 58°C/0,1 mbar.

### d) trans-2-Oxa-5,8-diazabicyclo[4.3.0]nonan

Analog Beispiel B wird 3-Benzyl-6-oxa-3-azabicyclo-[3.1.0]hexan mit 2-(Benzylamino)-ethanol zu trans-1-Benzyl-3-[N-benzyl-N-(2-hydroxyethyl)-amino]-4-hydroxypyrrolidin umgesetzt und anschließend analog Beispiel D a) zu 5,8-Dibenzyl-2-oxa-5,8-diazabicyclo[4.3.0]nonan umgesetzt und chromatographisch gereinigt (Kieselgel, Cyclohexan/ tert.-Butylmethylether/Essigsäureethylester 1:1:1).

Die hydrogenolytische Debenzylierung erfolgt analog Beispiel D c) zu trans-2-Oxa-5,8-diazabicyclo[4.3.0]nonan, Siedepunkt: 60°C/0,1 mbar.

### Beispiel E

### 5-Methyl-2-oxa-5,8-diazabicyclo[4.3.0]nonan-Dihydrochlorid

### a) 8-Benzyl-5-methyl-2-oxa-5,8-diazabicyclo[4.3.0] nonan

Wie in Beispiel D a) setzt man 18 g (71,9 mmol) 1-Benzyl-4-hydroxy-3-(2-hydroxyethyl-methy-amino)-pyrrolidin in 60 ml konzentrierter Schwefelsäure und 30 ml Wasser um.
Ausbeute: 10 g (60% der Theorie)
Siedepunkt: 122°C/0,08 mbar

### b) 5-Methyl-2-oxa-5,8-diazabicyclo[4.3.0]nonan-Dihydrochlorid

Man hydriert eine Lösung von 9,4 g (40 mmol) 8-Benzyl-5-methyl-2-oxa-5,8-diazabicyclo[4.3.0]nonan in 150 ml Methanol und 7,4 ml konzentrierter Salzsäure an 3 g Palladium-Aktivkohle (10% Pd) bei 80°C und 100 bar. Der Katalysator wird abgesaugt und das Filtrat eingeengt. Der Rückstand wird mit Butanol/Aceton 1:1 verrieben, die Kristalle abgesaugt und im Exsikkator über P₄O₁₀ getrocknet. Das Produkt ist sehr hygroskopisch.
Ausbeute: 8,2 g (95% der Theorie)
Massenspektrum: m/ₑ 142 (M⁺), 112 (M⁺⁻CH₂0), 100 (M⁺⁻CH₂-N=CH₂), 82 (C₄H₄N0⁺), 68 (C₄H₆N⁺)

### Beispiel

### 2-Methyl-3-oxa-2,7-diazabicyclo[3.3.0]octan

### a) N-(2,2-Dimethoxyethyl)-carbamidsäureethylester

Zu 214 g (2 mol) Aminoacetaldehyddimethylacetal in 1 1 Toluol und 90 g NaOH in 500 ml Wasser tropft man 214 g (2 mol) Chlorameisensäureethylester bei 10°C. Man rührt noch zwei Stunden bei Raumtemperatur, trennt die wäßrige Phase ab, sättigt sie mit Kochsalz und extrahiert mit Toluol. Die Toluollösungen werden über Magnesiumsulfat getrocknet, eingeengt und destilliert.
Ausbeute 338 g (95,4% der Theorie)
Siedepunkt: 60°C/0,03 mbar

### b) N-Allyl-N-(2,2-dimethoxyethyl)-carbamidsäureethylester

Man legt 20 g Natriumhydrid (80% im Paraffinöl) in 500 ml Toluol vor und tropft bei 80°C 89 g (0,5 mol) N-(2,2-Di- methoxyethyl)-carbamidsäureethylester hinzu. Man rührt eine Stunde bei 80°C und tropft dann 73 g (0,6 mol) Allylbromid innerhalb von drei Stunden hinzu. Man rührt über Nacht bei 80°C, bringt die Salze mit Wasser in Lösung und trennt die organische Phase ab Die wäßrige Phase wird mit Toluol extrahiert, die organischen Phasen über Kaliumcarbonat getrocknet, eingeengt und der Rückstand destilliert.
Ausbeute 68 g 162,6X der Theorie)
Siedepunkt: 65°C/0,09 mbar

### c) N-Allyl-N (2-oxoethyl)-carbamidsäureethylester

Man erhitzt 68 g (0,313 mol) N-Allyl-N-(2,2-dimethoxyethyl)-carbamidsäureethylester mit 150 ml Ameisensäure eine Stunde auf 100°C, Man gießt auf Eis, extrahiert mehrfach mit Methylenchlorid, wäscht die organischen Phasen mit Natriumhydrogencarbonatlösung, trocknet über Magnesiumsulfat, engt ein und destilliert.
Ausbeute: 46,7 g (87,2% der Theorie)
Siedepunkt: 58° C/0,09 mbar

### d) 2-Methyl-3-oxa-2,7-diazabicyclo[3.3.0)octan-7-carbonsäureethylester

Man löst 10 g (0,12 mol) Methylhydroxylamin-Hydrochlorid in 50 ml Methanol kühlt im Eisbad und tropft 22 g (0,12 mol) 30%ige Natriummethylatlösung in Methanol hinzu. Man saugt von Kochsalz ab und wäscht das Salz mit 80 ml Toluol. Die Lösung von Methylhydroxylamin tropft man innerhalb einer Stunde zu 20 g (0,117 mol) N-Allyl-N-(2-(oxoe- thyl)-carbamidsäureethylester, der in 160 ml Toluol am Wasserabscheider unter Rückfluß erhitzt wird. Man erhitzt über Nacht unter Rückfluß und extrahiert das Produkt zweimal mit je 80 ml 10%iger Salzsäure. Die salzsauren Lösungen werden mit Kaliumcarbonat gesättigt und sechsmal mit je 200 ml Chloroform extrahiert. Man trocknet über K₂CO₃, engt ein und destilliert den Rückstand.
Ausbeute: 18,6 g (79,5% der Theorie)
Siedepunkt: 93°C/0,09 mbar

### e) 2-Methyl-3-oxa-2,7-diazabicyclo[3.3.0)octan

Man erhitzt 13 g (65 mmol) 2-Methyl-3-oxa-2,7-diazabicyclo[3.3.0]octan-7-carbonsäureethylester in 300 ml Wasser mit 41 g Ba(OH)₂.BH₂0 über Nacht unter Rückfluß. Man setzt Kaliumcarbonat hinzu, saugt ausgefallenes Bariumcarbonat ab und extrahiert das Filtrat zehnmal mit je 100 ml Chloroform. Man trocknet über Kaliumcarbonat, engt ein und destilliert den Rückstand.
Ausbeute: 5,4 g (65% der Theorie)
Siedepunkt: 80°C/10 mbar

### Beispiel G

### 1-Methyl-octahydropyrrolo[3,4-b)pyrrol (2-Methyl-2,7-diazabicyclo[3.3.0)octan)

### a) 1-Benzyl-3-(2-chlorethyl-methyl-amino)-pyrrolidin-2,5-dion

74,8 g (0,4 mol) N-Benzylmaleinimid [Arch, Pharm, 308, 489 (1975)] und 52,0 g (0,4 mol) 2-Chlorethyl-methylamin-Hydrochlorid werden in 400 ml Dioxan vorgelegt und 40,4 g (0,4 mol) Triethylamin bei 20°C zugetropft. Anschließend wird 5 Stunden unter Rückfluß gekocht. Dann wird der Ansatz auf 2 I Eiswasser gegossen, mit 3 mal 400 ml Chloroform extrahiert, der Extrakt mit Wasser gewaschen, über Natriumsulfat getrocknet und am Rotationsverdampfer eingeengt. Bei der Chromatographie des Rückstands (101,1 g) auf Kieselgel mit Essigester:Petrolether (1:2) werden 56,8 g (51% der Theorie) eines Öls erhalten.
R_{F}-Wert: 0,33 (Kieselgel, Essigester/Petrolether 1:2)

### b) 5-Benzyl-4,6-dioxo-1-methyl-octahydropyrrolo [3,4-b]pyrrol

7,2 g (0,24 mol) einer 80%igen Natriumhydrid-Suspension in Mineralöl werden in 150 ml absolutem Dimethylformamid (über Calciumhydrid getrocknet) suspendiert und 62 g (0,22 mol) 1-Benzyl-3-(2-chlorethyl-methylamino)-pyrrolidin-2,5-dion als Lösung in 50 ml absolutem Dimethylformamid bei Raumtemperatur zugetropft. Dabei erfolgt eine exotherme Reaktion unter Aufschäumen. Es wird mit weiteren 50 ml absolutem Dimethylformamid verdünnt, 1 Stunde bei Raumtemperatur nachgerührt, dann auf Eiswasser gegossen und mit Dichlormethan extrahiert. Der Extrakt wird mit Wasser gewaschen, mit Natriumsulfat getrocknet und am Rotationsverdampfer eingeengt. Der Rückstand wird auf Kieselgel mit Essigester:Petrolether (1:2) und später (1:1) chromatographiert. Dabei werden zunächst 16,4 g Edukt wiedergewonnen und anschließend 17,2 g (44% der Theorie, bezogen auf umgesetztes Edukt) öliges Produkt isoliert.
Rf-Wert - 0,26
(Kieselgel, Essigester:Petrolether 1: 1).

### c) 5-Benzyl-1-methyl-octahydropyrrolo[3,4-b]pyrrol

1,52 g (40 mmol) Lithiumaluminiumhydrid werden in 30 ml wasserfreiem Tetrahydrofuran vorgelegt und 4,9 g (20 mmol) 5-Benzyl-4,6-dioxo-1-methyl-octahydropyrrolo-[3,4-b]pyrrol als Lösung in 15 ml wasserfreiem Tetrahydrofuran zugetropft. Dann wird 3 Stunden bei Siedetemperatur nachgerührt. Nacheinander werden zu dem Ansatz 1,5 ml Wasser, 1,5 ml 15%ige Kalilauge und 4,5 ml Wasser getropft, dann wird der Niederschlag abgesaugt und mit Tetrahydrofuran gewaschen. Das Filtrat wird am Rotationsverdampfer eingeengt und der Rückstand destilliert. Es werden 3,1 g (72% der Theorie) eines farblosen Destillats vom Siedepunkt 80°C/0,07 mbar erhalten.

### d) 1-Methyl-octahydropyrrolo[3,4-b]pyrrol

6,49 g (30 mmol) 5-Benzyl-1-methyl-octahydropyrrolo-[3,4-b]-pyrrol werden in 100 ml absolutem Ether gelöst und 5,2 g über Phosphorpentoxid getrockneter Chlorwasserstoff eingeleitet. Die entstandene Hydrochlorid-Suspension wird im Vakuum eingeengt und der Rückstand in 100 ml Methanol aufgenommen. Dann wird mit 2 g Pd-C (5 %) 4 Stunden bei 80°C und 50 bar hydriert. Der Katalysator wird anschließend abfiltriert, das Filtrat eingeengt und der Rückstand mit 30 ml 40%iger Natronlauge und 50 ml Ether versetzt. Die etherische Phase wird abgetrennt und die wäßrige Phase mit 2 x 50 ml Ether extrahiert. Die vereinigten organischen Phasen werden über Natriumsulfat getrocknet, eingeengt und destilliert. Es werden 1,3 g (34% der Theorie) eines farblosen Öls vom Siedepunkt 65-66°C/12 mbar erhalten.
Reinheit: >99%

### Beispiel H

### Octahydropyrrolo[3,4-b]pyrrol (2,7-Diazabicyclo[3.3.0]-octan)

### a) 1-Benzyl-3-(2-chlorethylamino)-pyrrolidin-2,5-dion

Nach der Arbeitsvorschrift des Beispiels Ga werden 74,8 g (0,4 mol) N-Benzylmaleinimid mit 58 g (0,5 mol) 2-Chlorethylamin-Hydrochlorid und 50,5 g (0,5 mol) Triethylamin umgesetzt. Nach der chromatographischen Aufarbeitung werden 81,6 g (77% der Theorie) eines Öls mit einem R_{F}-Wert von 0,24 (auf Kieselgel mit Essigester:Petrolether = 1: 1) erhalten.

### b) 5-Benzyl-4,6-dioxo-octahydropyrrolo[3,4-b]pyrrol

Nach der Arbeitsvorschrift des Beispiels Gb werden 17,4 g (0,58 mol) Natriumhydridsuspension mit 119 g (0,45 mol) 1-Benzyl-3-(2-chlorethylamino)-pyrrolidin-2,5-dion in 550 ml absolutem Dimethylformamid umgesetzt. Nach dem Stehen über Nacht wird wäßrig aufgearbeitet. Bei der chromatographischen Reinigung werden Verunreinigunden zunächst mit Essigester und dann das Produkt mit Essigester:Methanol (3:1) (R_{F}-Wert 0.55) eluiert. Es werden 57,7 g Produkt (56% der Theorie) isoliert.

### c) 5-Benzyl-octahydropyrrolo[3,4-b]pyrrol

Nach der Arbeitsvorschrift des Beispiels Gc werden 57,7 g (0,25 mol) rohes 5-Benzyl-4,6-dioxo-octahydropyrrolo [3,4-b]pyrrol mit 21,4 g (0,56 mol) Lithiumaluminiumhydrid durch 10-stündiges Kochen in 700 ml abs. Tetrahydrofuran reduziert. Bei der destillativen Aufarbeitung werden 21,0 g (41,1% der Theorie) eines Öls vom Siedepunkt 95°C/0,1 mbar erhalten.

### d) Octahydropyrrolor3,4-b1pyrrol

21,0 g (0,104 mol) 5-Benzyl-octahydropyrrolo[3,4-b]pyrrol werden in 180 ml eisgekühltem Methanol vorgelegt und mit 17,3 ml (0,208 mol) konzentrierter Salzsäure versetzt. Dann wird mit 2 g Pd-C (5 %) 4 Stunden bei 90°C und 100 bar hydriert. Der Katalysator wird abfiltriert, das Filtrat mit 37,4 g(0,208 mol) 30%iger Natriummethylat-Lösung versetzt, erneut filtriert und das Filtrat eingeengt. Der Rückstand wird über eine kleine Vigreux-Kolonne destilliert. Es werden 5,6 g eines farblosen Öls (48% der Theorie) vom Siedepunkt 93-95°C/ 30 mbar erhalten, welches an der Luft raucht und in der Vorlage langsam erstarrt (Schmelzpunkt 40°C).

### Beispiel I

### Octahydropyrrolo[3,4-b]pyridin (2,8-Diazabicyclo[4.3.0] nonan

### a) 6-Benzyl-5,7-dioxo-octahydropyrrololf3,4-blpyridin

47,6 g (0,2 mol) Pyridin-2,3-dicarbonsäure-N-benzylimid (Brit. Pat. 1 086 637; Chem. Abstr. 68, 95695w) werden in 400 ml Glykolmonomethylether über 15 g Ruthenium auf Aktivkohle (5%) bei 90°C und 100 bar hydriert, bis die berechnete Wasserstoffmenge aufgenommen worden ist. Dann wird der Katalysator abfiltriert und das Filtrat am Rotationsverdampfer eingeengt. Es werden 44 g eines öligen Rohproduktes erhalten.

Die entsprechende Hydrierung mit Palladium/Aktivkohle (5 %ig) liefert in quantitativer Ausbeute ein Reinprodukt vom Schmelzpunkt 67-69°C,

### b) 6-Benzyl-octahydropyrrolo[3,4-b]pyridin

Nach der Arbeitsvorschrift des Beispiels Gc werden 44 g (ca. 0,18 mol) rohes oder reines 6-Benzyl-5,7-dioxo- octahydropyrrolo[3,4-b]pyridin mit 15,2 g (0,40 mol) Lithiumaluminiumhydrid in 390 ml absolutem Tetrahydrofuran innerhalb von 10 Stunden reduziert. Bei der Destillation werden 24,4 g eines farblosen Öls mit einem Siedepunkt von 93-95°C/0,06 mbar erhalten.

### c) Octahydropyrrolo[3,4-b]pyridin

69 g (0,32 mol) 6-Benzyl-octahydropyrrolo[3,4-b]pyridin werden in 450 ml Methanol bei 90°C/90 bar über 7 g Palladium auf Aktivkohle (5 %ig) innerhalb von 3 Stunden hydriert Der Katalysator wird anschließend abfiltriert, das Filtrat eingeengt und der Rückstand destilliert. Es werden 33,8 g (84 % der Theorie) eines farblosen Festkörpers mit einem Schmelzpunkt von 65-67°C und einem Siedepunkt von 78°C/9 mbar erhalten.

### Beispiel J

### 1-Methyl-octahydropyrrolo[3,4-b]pyridin (2-Methyl-2,8-diazabicyclof[4.3.O]nonan)

### a) 1-Methyl-pyridinium-2,3-dicarbonsäure-N-benzylimid-iodid

190,5 g (0,8 mol) Pyridin-2,3-dicarbonsäure-N-benzyiimid werden unter Erwärmen in 800 ml Nitromethan gelöst und 136 g (0,96 mol) Methyliodid zugetropft. Anschliessend wird 8 Stunden unter Rückflußkühlung (Kühlwasser 0°C) gekocht. Nach dem Erkalten wird der Feststoff abgesaugt und mit Dichlormethan gewaschen. Es werden 123 g dunkelrote Kristalle mit einem Schmelzpunkt 162-165°C (Zersetzung) erhalten.

### b) 6-Benzyl-1-methyl-5,7-dioxo-octahydropyrrolo-f3,4-blpyridin

38 g (0,1 mol) 1-Methyl-pyridinium-2,3-dicarbonsäure-N-benzylimid-iodid werden bei 30°C und 70 bar über 1 g Platinoxid in 450 ml Glykolmonmethylether bis zur Beendigung der Wasserstoffaufnahme (51 Stunden) hydriert. Der Katalysator wird dann abfiltriert, das Filtrat eingeengt, der Rückstand in 300 ml Chloroform aufgenommen und die Lösung 2 x mit je 300 ml 10%iger Sodaiösung sowie 300 ml Wasser gewaschen. Nach dem Trocknen über Natriumsulfat wird eingeengt, Es bleiben 27 g eines öligen Rückstands zurück.

### c) 6-Benzyl-1-methyl-octahydropyrrolo[3,4-b]pyridin

Nach der Arbeitsvorschrift des Beispiels G c werden 19,2 g (0,08 mol) rohes 6-Benzyl-1-methyl-5,7-dioxo-octa- hydropyrrolo[3,4-b]pyridin mit 6,1 g (0,16 mol) Lithiumaluminiumhydrid im absolutem Tetrahydrofuran reduziert.
Ausbeute: 9,5 g (52% der Theorie),
Siedepunkt: 93-96°C/0,1 mbar.

### d) 1-Methyl-octahydropyrrolo[3,4-b]pyridin

Nach der Arbeitsvorschrift des Beispiels Gd werden 11,7 g (54 mmol) 6-Benzyl-1-methyl-octahydropyrrolo-[3,4-b] pyridin als Dihydrochlorid in 100 ml Methanol über Palladium auf Aktivkohle hydriert. Bei der destillativen Aufarbeitung werden 2,6 g (34% der Theorie) eines farblosen Öls vom Siedepunkt 83-85°/12 mbar erhalten.

### Beispiel K

### 2,5-Dimethyl-3-oxa-2,7-diazabicyclo[3.3.0]octan

### a) N-(2-Methylprop-2-enyl)-N-(2,2-dimethoxyethyl)-urethan

Zu 20 g Natriumhydrid (80 %ig) in 500 ml absolutem Toluol tropft man bei 90°C 89 g (0,5 mol) N-(2,2-Dimethoxye- thyl)-urethan. Wenn kein Wasserstoff mehr entsteht tropft man 54 g (0,6 mol) Methallylchlorid hinzu und rührt über Nacht bei 90°C. Das ausgeschiedene Kochsalz wird mit wenig Wasser gelöst, die organische Phase abgetrennt, über K₂CO₃ getrocknet, eingeengt und destilliert.
Ausbeute: 71,3 g (61,7 % der Theorie)
Siedepunkt: 60°C/0,08 mbar

### b) N-(2-Methylprop-2-enyl)-N-(2-oxoethyl)-urethan

Man erhitzt 11,5 g (50 mmol) N-(2-Methylprop-2-enyl)-N-(2,2-dimethoxyethyl)-urethan und 1,25 g (5 mmol) Pyridinium-p-toluolsulfonat in 100 ml Aceton und 10 ml Wasser zwei Tage unter Rückfluß. Man engt ein und destilliert den Rückstand
Ausbeute: 5,3 g (61,2 % der Theorie)
Siedepunkt: 73°C/0,1 mbar

### c) 2,5-Dimethyl-3-oxa-2,7-diazabicyclo[3.3.0]octan-7-carbonsäureethylester

Zu 10 g (0,12 mol) N-Methylhydroxylamin-Hydrochlorid in 26 ml Methanol tropft man 21,7 g 30 %ige Natriummethylatlösung, Man saugt das Kochsalz ab und wäscht mit 8 ml Methanol und 80 ml Toluol. Diese Lösung tropft man zu 19,2 g (0,11 mol) N-(2-Methylprop-2-enyl)-N-(2-oxoethyl)-urethan, das in 160 ml Toluol am Wasserabscheider unter Rückfluß erhitzt wird, Man erhitzt über Nacht unter Rückfluß, extrahiert das Produkt mit 160 ml 10 %iger Salzsäure, stellt die salzsäure Lösung mit Kaliumcarbonat alkalisch und extrahiert mit sechsmal 200 ml CHCI3 Man trocknet die Extrakte über K₂C0₃, engt ein und destilliert.
Ausbeute: 13 g (55 % der Theorie)
Siedepunkt: 88-95°C/0,08 mbar

### d) 2,5-Dimethyl-3-oxa-2,7-diazabicyclo[3.3.0]octan

Man erhitzt 13 g (60,6 mmol) 2,5-Dimethyl-3-oxa-2,7-diazabicyclo[3.3.0]octan-7-carbonsäureethylester mit 33 g Ba(OH)₂·8H₂0 in 330 ml Wasser über Nacht unter Rückfluß. Man saugt BaCO₃ ab, setzt K₂CO₃ zum Fitrat, saugt erneut ab und extrahiert das Filtrat zehnmal mit je 100 ml CHC1₃. Man trocknet die Extrakte über K₂C0₃, engt ein und destilliert
Ausbeute: 5,9 g (63,7 % der Theorie)
Siedepunkt: 64°C/5 mbar

### Beispiel

### 2,8-Dimethyl-3-oxa-2,7-diazabicyclo[3.3.0]octan

### a) N-(1,1-Dimethoxyprop-2-yl)-urethan

Zu 86,2 g (0,72 mol) 2-Aminopropionaldehyddimethylacetal in 350 ml Toluol und 32 g (0,8 mol) NaOH in 300 ml Wasser tropft man unter Eiskühlung 80 g (0,73 mol) Chlorameisensäureethylester. Man rührt noch zwei Stunden bei Raumtemperatur, trennt die organische Phase ab, extrahiert die wäßrige Phase mit Toluol und trocknet die Toluollösungen über K₂CO₃. Man engt ein und destilliert.
Ausbeute: 132 g (95 % der Theorie)
Siedepunkt: 55°C/0,06 mbar

### b) N-Allyl-N-(1,1-dimethoxyprop-2-yl)-urethan

Zu 25 g Natriumhydrid (80 %ig) in 700 ml absolutem Toluol tropft man bei 90°C 131 g (0,686 mol) N-(1,1-Dime- thoxyprop-2-yl)-urethan. Nach Beendigung der Wasserstoffentwicklung tropft man bei 90°C-61,2 g (0,8 mol) Allylchlorid hinzu und rührt über Nacht bei 90°C. Ausgeschiedenes Kochsalz wird mit Wasser aufgelöst, die organische Phase abgetrennt, über K₂C0₃ getrocknet, eingeengt und destilliert.
Ausbeute: 78 g (31,7 % der Theorie)
Siedepunkt: 62-69°C/0,06 mbar.
Gehalt: 64,5 µig (gaschchromatographisch bestimmt)

### c) N-Allyl-N-(1-oxoprop-2-yl)-urethan

Man erhitzt 76,5 g (0,213 mol) 64,5 %iges N-Allyl-N-(1,1-dimethoxyprop-2-yl)-urethan in 180 ml Ameisensäure eine Stunde auf 100°C. Man gießt auf Eiswasser, extrahiert mit CH₂Cl₂, wäscht die Extrakte mit NaHC0₃-Lösung neutral, trocknet über MgS0₄, engt ein und destilliert.
Ausbeute: 36 g (80,9 % der Theorie)
Siedepunkt: 97-102°C/8 mbar
Gehalt: 88,8 %ig (gaschromatographisch bestimmt)

### d) 2,8-Dimethyl-3-oxa-2,7-diazbicyclo[3.3.0)octan-7-carbonsäureethylester

Man stellt aus 16,4 g (0,2 mol) N-MethylhydroxylaminHydrochlorid in 33 ml absolutem Methanol und 36 g (0,2 mol) 30 %iger Natriummethylatlösung eine methanolische Methylhydroxylaminlösung her, verdünnt sie mit 130 ml Toluol und tropft sie zu 354 g (0,17 mol) N-Allyl-N-(1-oxoprop-2-yl)-urethan in 250 ml Toluol, welches am Wasserabscheider unter Rückfluß erhitzt wird. Man erhitzt über Nacht unter Rückfluß, extrahiert das Produkt mit verdünnter Salzsäure, stellt die salzsäure Lösung mit K₂C0₃ alkalisch und extrahiert mit CHCI₃. Man trocknet über K₂C0₃, engt ein und destilliert.
Ausbeute: 18,5 g (50,8 % der Theorie)
Siedepunkt: 95-105°C/0,1 mbar

### e) 2,8-Dimethyl-3-oxa-2,7-diazabicyclo[3.3.0]octan

Man erhitzt 9,2 g (42,9 mmol) 2,8-Dimethyl-3-oxa-2,7-diazabicyclo[3.3.0]octan-7-carbonsäureethylester mit 23,5 g Ba(OH)₂·8H₂O in 235 ml Wasser über Nacht unter Rückfluß. Man saugt BaC0₃ ab, versetzt das Filtrat mit K₂C0₃ und saugt erneut ab. Das Filtrat wird zehnmal mit je 50 ml CHCl₃ extrahiert, die Extrakte über K₂CO₃ getrocknet, eingeengt und destilliert.
Ausbeute: 1,7 g
Siedepunkt: 87-92°C/10 mbar

Es handelt sich um ein Gemisch der möglichen Stereoisomeren im Verhältnis 3:1 1 H-NMR).
Im Nachlauf konnten 4 g Ausgangsmaterial zurückgewonnen werden.

### Beispiel M

### 2-Methyl-4-oxa-2,8-diazabicyclo[4.3.0]nonan

### a) 4-Hydroxymethyl-3-methylaminopyrrolidin-1-carbonsäureethylester

Man hydriert 10 g (50 mmol) 2-Methyl-3-oxa-2,7-diazabicyclo[3.3.0]octan-7-carbonsäureethylester (Beispiel H d)) in 200 ml Ethanol an 3 g Pd-Aktivkohle (10 % Pd) bei 50°C und 50 bar. Der Katalysator wird abfiltriert, das Filtrat eingeengt und der Rückstand destilliert.
Ausbeute 8,1 g (80 % der Theorie)
Siedepunkt: 135-140°C/0,1 mbar

### b) 2-Methyl-4-oxa-2,8-diazabicyclo[4.3.0]nonan-8-carbonsäureethylester

Man löst 10,1 g (50 mmol) 4-Hydroxymethyl-3-methylamino-pyrrolidin-1-carbonsäureethylester und 8 g (0,1 mol) 37 %ige Formaldehydlösung in 100 ml Butanol und rührt über Nacht bei Raumtemperatur. Anschließend engt man ein und destilliert den Rückstand.
Ausbeute: 9,5 g (88,7 % der Theorie)
Siedepunkt: 110°C/0,1 mbar

### c) 2-Methyl-4-oxa-2,8-diazabicyclo[4.3.0]nonan

Man erhitzt 9 g (42 mmol) 2-Methyl-4-oxa-2,8-diazabicyclo[4.3.0]nonan-8-carbonsäureethylester mit 28 g Ba (OH)₂·8H₂O in 280 ml Wasser über Nacht unter Rückfluß. Man saugt BaC0₃ ab, engt ein und kocht den Rückstand mit Dioxan aus. Die Dioxanlösung wird eingeengt und der Rückstand destilliert.
Ausbeute: 1,3 g (21,8 % der Theorie)
Siedepunkt: 115°C/8 mbar.

### d) 4-Hydroxymethyl-3-methylaminopyrrolidin

Man erhitzt 34 g (0,168 mol) 4-Hydroxymethyl-3-methylaminopyrrolidin-1-carbonsäureethylester mit 100 g Ba (OH)₂·8H₂O in 400 ml Wasser über Nacht unter Rückfluß. Man saugt BaC0₃ ab, engt das Filtrat ein und kocht den Rückstand zehnmal mit je 100 ml Dioxan aus. Man filtriert die Dioxanlösungen, engt ein und destilliert.
Ausbeute: 13 g (60,3 % der Theorie)
Siedepunkt: 85-88°C/0,08 mbar

### e) 2-Methyl-4-oxa-2,8-diazabicyclo[4.3.0]nonan

Zu 13 g (0,101 mol) 4-Hydroxymethyl-3-methylaminopyrrolidin in 100 ml n-Butanol tropft man bei Raumtemperatur 8,1 g (0,1 mol) 37 %ige Formaldehydlösung in 20 ml n-Butanol hinzu. Man rührt über Nacht bei Raumtemperatur, engt ein und destilliert.
Ausbeute: 8,7 g (61,2 % der Theorie)
Siedepunkt: 84°C/6 mbar

### Beispiel N

### 3-Oxa-2,7-diazabicyclo[3.3.0]octan

### a) 2-(Tetrahydropyran-2-yl)-3-oxa-2,7-diazabicyclo-[3.3.0]octan-7-carbonsäureethylester

Man erhitzt 18,1 g (0,106 mol) N-Allyl-N-(2-oxo-ethyl)-carbamidsäureethylester (Beispiel F c)) in 220 ml Toluol unter Rückfluß und tropft 14,2 g (0,12 mol) 5-Hydroxypentanaloxim (Acta Chim. Acad. Sci. Hung., 4, 333 (1958)) in 55 ml heißem Toluol gelöst hinzu. Man erhitzt über Nacht unter Rückfluß, engt ein und destilliert.
Ausbeute: 15,5 g (54 % der Theorie)
Siedepunkt: 160°C/0,01 mbar

### b) 3-Oxa-2,7-diazabicyclo[3.3.0]octan-7-carbonsäureethylester

Man erhitzt 15 g (55,5 mmol) 2-(Tetrahydropyran-2-yl)-3-oxa-2,7-diazabicyclo[3.3.0]octan-7-carbonsäureethyle- ster mit 8,25 g (56 mmol) 70 %iger Perchlorsäure in 100 ml Ethanol 30 Minuten unter Rückfluß. Man setzt 10,5 g (58 mmol) 30 %ige Natriummethylatlösung hinzu, engt ein, nimmt in Wasser auf, sättigt mit K₂C0₃ und extrahiert mit CHCl₃. Man trocknet über K₂C0₃, engt ein und destilliert.
Ausbeute: 7,6 g (73,5 % der Theorie)
Siedepunkt: 125-130°C/0,1 mbar

### c) 3-Oxa-2,7-diazabicyclo[3.3.0]octan-7-carbonsäureethylester

Man erhitzt 8,5 g (50 mmol) N-(2-Oxoethyl)-N-allyl-carbamidsäureethylester mit 5,5 g (50 mmol) o-Trimethylsilylhydroxylamin in 100 ml Xylol über Nacht unter Rückfluß. Man engt ein und destilliert,
Ausbeute: 6,8 g (73 % der Theorie)
Siedepunkt: 120-122°C/0,05 mbar

### d) 3-Oxa-2,7-diazabicyclo[3.3.0]octan

Man erhält diese Substanz analog Beispiel Kd) durch Verseifen von 3-Oxa-2,7-diazabicyclo[3.3.0]-octan-7-car- bonsäureethylester mit Ba(OH)₂·8H₂O. Siedepunkt: 75°C/10 mbar.

### Beispiel

### 3-Methyl-2,7-diazabicyclo[3.3.0]octan

Analog Beispiel G erhält man 3-Methyl-2,7-diazabicyclo[3.3.0]octan.
Siedepunkt: 68-70°C/6 mbar.

### Beispiel P

### 2,3-Dimethyl-2,7-diazabicyclo[3.3.0]octan

Analog Beispiel G erhält man 2,3-Dimethyl-2,7-diazabicyclo[3.3.0.]octan.
Siedepunkt: 72-74°C/10 mbar.

### Beispiel Q

### 1,2-Dimethyl-3-oxa-2,7-diazabicyclo[3.3.0]octan

### a) N-Allyl-N-(2,2-dimethoxypropyl)-acetamid

Zu 29,6 g (0,987 mol) Natriumhydrid (80 %ig in Paraffinöl) in 750 ml absolutem Toluol tropft man bei 80°C 119 g (74 mol) 2,2-Dimethoxypropylacetamid. Anschließend rührt man eine Stunde und tropft dann 100 g (0,83 mol) Allylbromid bei 80°C hinzu. Man rührt über Nacht bei 80°C, kühlt ab und löst die Salze mit Wasser. Man trennt die wäßrige Phase ab und extrahiert sie zweimal mit je 100 ml Toluol. Man trocknet die Toluollösungen über K₂C0₃, engt ein und destilliert.
Ausbeute: 112 g (75,6 % der Theorie)
Siedepunkt: 70°C/0,08 mbar.

### b) N-Allyl-N-(2-oxopropyl)-acetamid

Man erhitzt 85,5 g (0,425 mol) N-Allyl-N-(2,2-dimethoxypropyl)-acetamid mit 212 ml Ameisensäure eine Stunde unter Rückfluß. Man gießt auf 500 g Eis, extrahiert mehrfach mit Methylenchlorid, wäscht die organischen Phasen mit Natriumhydrogencarbonatlösung, trocknet über Magnesiumsulfat, engt ein und destilliert.
Ausbeute: 50 g (75,8 % der Theorie)
Siedepunkt: 79°C/0,25 mbar.

### c) 7-Acetyl-1,2-dimethyl-3-oxa-2,7-diazabicyclo-[3.3.0]octan

Man löst 15,5 g (0,1 mol) N-Allyl-N-(2-oxopropyl)-acetamid in 100 ml Dioxan und setzt 9 g wasserfreies Natriumacetat sowie 9 g (0,108 mol) N-Methylhydroxylaminhydrochlorid in 10 ml Wasser hinzu. Man erhitzt über Nacht unter Rückfluß, kühlt ab, saugt Salze ab und wäscht sie mit Dioxan. Das Filtrat wird eingeengt, der Rückstand in 100 ml Wasser aufgenommen und mit K₂CO₃ versetzt. Man extrahiert mit CHCl₃, trocknet über K₂CO₃, engt ein und destilliert.
Ausbeute: 15,9 g (86,3 % der Theorie)
Siedepunkt: 75°C/0,1 mbar.

### d) 1,2-Dimethyl-3-oxa-2,7-diazabicyclo[3.3.0]octan

Man erhitzt 11,8 g (64 mmol) 7-Acetyl-1,2-dimethyl-3-oxa-2,7-diazabicyclo[3.3.0]octan mit 12 g NaOH in 36 ml Wasser über Nacht unter Rückfluß. Man sättigt mit K₂C0₃, extrahiert mehrfach mit CHCI₃, trocknet über K₂C0₃, engt ein und destilliert,
Ausbeute: 4,7 g (51,6 % der Theorie)
Siedepunkt: 40°C/0,2 mbar.

### Beispiel R

### 2,4-Dimethyl-3-oxa-2,7-diazabicyclo[3.3.0]octan

### a) N-(But-2-enyl)-N-(2,2-dimethoxyethyl)-carbamidsäureethylester

Zu 17,5 g (0,58 mol) NaH (80 %ig in Paraffinöl) in 500 ml absolutem Toluol tropft man bei 80°C 89 g (0,5 mol) N-(2,2-Dimethoxyethyl)-carbamidsäureethylester, Anschließend rührt man eine Stunde und tropft dann bei 80°C 80 g (0,59 mol) 1-Brom-2-buten hinzu. Man rührt über Nacht bei 80°C, kühlt ab, bringt die Salze mit Wasser in Lösung, trennt die wäßrige Phase ab und extrahiert sie mit Toluol. Die Toluollösungen werden über K₂C0₃ getrocknet, eingeengt und destilliert.
Ausbeute 90 g (77,8 % der Theorie)
Siedepunkt: 65°C/0,1 mbar.

### b) N-(But-2-enyl)-N-(2-oxoethyl)-carbamidsäureethylester

Man erhitzt 90 g (0,39 mol) N-(But-2-enyl)-N-(2,2-dimethoxyethyl)-carbamidsäureethylester mit 200 ml Ameisensäure eine Stunde unter Rückfluß. Man gießt auf 500 g Eis, extrahiert mit Methylenchlorid, wäscht die organischen Phasen mit Natriumhydrogencarbonatlösung, trocknet über Magnesiumsulfat, engt ein und destilliert.
Ausbeute: 33,6 g (46,5 % der Theorie)
Siedepunkt: 65°C/0,1 mbar.

### c) 2,4-Dimethyl-3-oxa-2,7-diazabicyclo[3.3.0]octan-7-carbonsäureethylester

Man löst 18,4 g (0,1 mol) N-(But-2-enyl)-N-(2-oxo-ethyl)-carbamidsäureethylester in 100 ml Dioxan und setzt 9 g wasserfreies Natriumacetat sowie 9 g (0,108 mol) N-Methylhydroxylaminhydrochlorid in 10 ml Wasser hinzu. Man erhitzt über Nacht unter Rückfluß, kühlt ab, saugt Salze ab und wäscht sie mit Dioxan. Das Filtrat wird eingeengt, der Rückstand in 100 ml Wasser aufgenommen und mit K₂C0₃ versetzt. Man extrahiert mit CHCl₃, trocknet über K₂C0₃, engt ein und destilliert.
Ausbeute: 15,0 g (70 % der Theorie)
Siedepunkt: 74-87°C/0,1 mbar.

### d) 2,4-Dimethyl-3-oxa-2,7-diazabicyclo[3.3.0]octan

Man erhitzt 13,2 g (61,6 mmol) 2,4-Dimethyl-3-oxa-2,7-diazabicyclo[3.3.0]octan-7-carbonsäureethylester mit 39 g Ba(OH)₂· 8H₂O in 200 ml Wasser über Nacht unter Rückfluß. Man versetzt-mit K₂CO₃, saugt BaC0₃ ab und extrahiert das Filtrat mehrfach mit CHCl₃. Man trocknet über K₂C0₃, engt ein und destilliert.
Ausbeute: 4,8 g (54,8 % der Theorie)
Siedepunkt: 74°C/8 mbar.

### Beispiel S

### 2,7-Diazabicyclo[3.3.0]octan-2-carbonsäureethylester

Analog Beispiel La) wird 7-Benzyl-2,7-diazabicyclo[3.3.0]octan (Beispiel Hc) mit Chlorameisensäureethylester zu 7-Benzyl-2,7-diazabicyclo[3.3.0]octan-2-carbonsäureethylester umgesetzt und dieser anschließend analog Beispiel Hd) hydrogenolytisch debenzyliert. Es wird ein farbloses Öl vom Siedepunkt 90°C/0,1 mbar erhalten.

### Beispiel T

### 2-Phenyl-2,7-diazabicyclo[3.3.0]octan

Die Herstellung erfolgt analog Beispiel G);
Siedepunkt: 103°C/0,08 mbar.

### Beispiel U

### 4-Oxa-2,8-diazabicyclo[4.3.0]nonan

### a) 3-Amino-4-hydroxymethyl-pyrrolidin-1-carbonsäureethylester

Analog Beispiel M a) wird 3-Oxa-2,7-diazabicyclo[3.3.0)octan-7-carbonsäureethylester (Beispiel Mc) hydriert. Siedepunkt: 163-168°C/0,8 mbar

### b) 3-Amino-4-hydroxymethyl-pyrrolidin

Analog Beispiel Md) wird 3-Amino-4-hydroxymethylpyrrolidin-1-carbonsäureethylester verseift.
Siedepunkt: 78°C/0,06 mbar

### c) 4-Oxa-2,8-diazabicyclo[4.3.0]nonan

Analog Beispiel Me) wird 3-Amino-4-hydroxymethylpyrrolidin mit Formaldehydlösung umgesetzt.
Siedepunkt: 50-60°C/0,07 mbar

### Beispiel V

### 4-Methyl-2,8-diazabicyclo[4.3.0]nonan

### a) 5-Methyl-1,4-dihydropyridin-2,3-dicarbonsäure-N-benzylimid

33 g (0,29 mol) 2-Methyl-2-propenal-dimethylhydrazon und 55 g (0,29 mol) N-Benzylmaleinimid werden in 225 ml Acetonitril 3 Stunden bei 60°C gerührt. Dann wird das Lösungsmittel am Rotationsverdampfer entfernt, der Rückstand in 600 ml Toluol aufgenommen und unter Zusatz von 150 g Kieselgel 1 Stunde unter Rückflußkühlung gekocht. Dann wird heiß filtriert und das Kieselgel mehrmals mit Ethanol ausgekocht Die vereinigten organischen Phasen werden am Rotationsverdampfer eingeengt. Es werden 17,5 g (24% der Theorie) rote Kristalle vom Schmelzpunkt 184-186°C erhalten

### b) 5-Methyl-hexahydropyridin-2,3-dicarbonsäure-N-benzylimid

17,5 g (70 mmol) 5-Methyl-1,4-dihydropyridin-2,3-dicarbonsäure-N-benzylimid werden in 150 ml Tetrahydrofuran bei 70°C und 100 bar über Palladium auf Aktivkohle hydriert. Dann wird der Katalysator abfiltriert und das Filtrat eingeengt. Der ölig-feste Rückstand (13,0 g) wird als Rohprodukt in die nächste Stufe eingesetzt.

### c) 8-Benzyl-4-methyl-2,8-diazabicyclo[4.3.0]nonan

13,0 g rohes 5-Methyl-hexahydropyridin-2,3-dicarbonsäure-N-benzylimid werden als Lösung in 50 ml absolutem Tetrahydrofuran zu vorgelegten 4,6 g (0,12 mol) Lithiumaluminiumhydrid in 100 ml absolutem Tetrahydrofuran getropft. Dann wird 17 Stunden unter Rückflußkühlung gekocht. Nacheinander werden 4,6 g Wasser in 14 ml Tetrahydrofuran, 4,6 g 10%ige Natronlauge sowie 13,8 g Wasser zugetropft. Die Salze werden abgesaugt, das Filtrat eingeengt und der Rückstand destilliert Ausbeute: 8,7 g (54 % bezogen auf 5-Methyl-1,4-dihydropyridin-2,3-dicarbonsäure-N-ben- zylimid)
Siedepunkt: 95-98C/0,1 mbar.

### d) 4-Methyl-2,8-diazabicyclo[4.3.0]nonan

8,0 g (35 mmol) 8-Benzyl-4-methyl-2,8-diazabicyclo-[4.3.0]nonan werden in 60 ml Methanol gelöst und bei 100°C und 100 bar über Palladium auf Aktivkohle hydriert. Dann wird der Katalysator abfiltriert, das Filtrat eingeengt und der Rückstand destilliert
Ausbeute: 3,3 g (67 % der Theorie),
Siedepunkt: 88-89°C/11 mbar.

Das H-NMR-Spektrum weist die Verbindung als Gemisch zweier Stereoisomere im Verhältnis 7:2 aus.

### Beispiel AA

### 5,6,7,8-Tetrafluor-1-(2,4-difluorphenyl)-1,4-dihydro-4-oxo-3-chinolincarbonsäure

### a) 2-(2,3,4,5,6-Pentafluorbenzoyl)-3-(2,4-difluorphenylamino)-acrylsaureethylester

Zu einer Lösung von 115 g 3-Ethoxy-2-(2,3,4,5,6-pentafluorbenzoyl)-acrylsäureethylester in 380 ml Ethanol gibt man unter Eiskühlung und Rühren tropfenweise 44,3 g 2,4-Difluoranilin. Man rührt 1 Stunde bei Raumtemperatur, versetzt unter Eiskühlung mit 380 ml Wasser, saugt den Niederschlag ab, wäscht mit Ethanol/H₂0 (1:1) und trocknet. Man erhält 135,4 g der Titelverbindung vom Schmelzpunkt 97-99°C.

### b) 5,6,7,8-Tetrafluor-1-(2,4-difluorphenyl)-1,4-dihydro-4-oxo-3-chinolincarbonsäureethylester

Ein Gemisch von 135,4 g 2-(2,3,4,5,6-Pentafluorbenzoyl)-3-(2,4-difluorphenylamino)-acrylsäureethylester, 20,6 g Natriumfluorid und 300 ml wasserfreiem Dimethylformamid wird 3 Stunden auf 140-150°C erhitzt. Die Suspension wird heiß auf 2 kg Eis gegossen, der Niederschlag abgesaugt, mit Wasser gewaschen und getrocknet. Man erhält 122 g der Titelverbindung vom Schmelzpunkt 160-162°C.

### c) 5,6,7,8-Tetrafluor-1-(2,4-difluorphenyl)-1,4-dihydro-4-oxo-3-chinalincarbonsäure

Zu einem Gemisch von 28,5 ml konz. Schwefelsäure, 250 ml Eisessig und 200 ml Wasser gibt man 40,1 g 5,6,7,8-Tetrafluor-1-(2,4-difluorphenyl)-1,4-dihydro-4-oxo-3-chinolincarbonsäureethylester und erhitzt 2 Stunden unter Rückfluß. Man gießt die heiße Lösung auf 2 kg Eis, saugt den Niederschlag ab, wäscht mit Wasser und trocknet. Es werden 34,5 g der Titelverbindung vom Schmelzpunkt 250-252°C erhalten.

### Beispiel AB

### 5,7-Dichlor-1-cyclopropyl-6-fluor-1,4-dihydro-4-oxo-3-chinolincarbonsäure

### a) (2,4-Dichlor-3,6-difluorbenzoyl)-essigsäureethylester

2,1 g Magnesiumspäne werden in 5 ml wasserfreiem Ethanol suspendiert. Man versetzt mit 0,5 ml Tetrachlorkohlenstoff und tropft, wenn die Reaktion in Gang gekommen ist, ein Gemisch von 14 g Malonsäureethylester, 10 ml abs. Ethanol und 41 ml Toluol zu. Dann wird noch 1,5 Stunden auf 70°C erhitzt, mit Aceton/Trockeneis auf -5°C bis -10°C gekühlt und bei dieser Temperatur eine Lösung von 21,5 g 2,4-Dichlor-3,6-difluorbenzoylchlorid in 30 ml Toluol langsam zugetropft. Man rührt 1 Stunde bei 0°C, läßt über Nacht auf Raumtemperatur kommen und läßt unter Eiskühlung ein Gemisch von 35 ml Eiswasser und 5 ml konzentrierter Schwefelsäure zulaufen. Die Phasen werden getrennt und zweimal mit Toluol nachextrahiert. Die vereinigten Toluollösungen werden einmal mit gesättigter Kochsalzlösung gewaschen, mit Na₂S0₄ getrocknet und das Lösungsmittel im Vakuum abgezogen. Man erhält 34,7 g (2,4-Dichlor-3,6-difluorbenzoyl)-malonsäurediethylester als Rohprodukt.

Eine Emulsion von 34,7 g rohem (2,4-Dichlor-3,6-difluorbenzoyl)-malonsäurediethylester in 40 ml Wasser wird mit 0,04 g p-Toluolsulfonsäure versetzt. Man erhitzt unter gutem Rühren 3 Stunden zum Sieden, extrahiert die erkaltete Emulsion mehrmals mit Methylenchlorid, wäscht die vereinigten CH₂C'₂₋Lösungen einmal mit gesättigter Kochsalzlösung, trocknet mit Na₂S0₄ und destilliert das Lösungsmittel im Vakuum ab. Die Fraktionierung des Rückstandes (33,9 g) im Vakuum liefert 13,9 g (2,4-Dichlor-3,6-difluorbenzoyl)-essigsäureethylester vom Siedepunkt 110-115°C/0,05 mbar; n 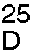 1,5241.

### b) 2-(2,4-Dichlor-3,6-difluorbenzoyl)-3-ethoxy-acrylsäureethylester

13,7 g (2,4-Dichlor-3,6-difluorbenzoyl)-essigsäureethylester werden mit 10,25 g Orthoameisensäuretriethylester und 11,8 g Essigsäureanhydrid 2 Stunden unter Rückfluß erhitzt. Anschließend wird bis 140°C Badtemperatur im Vakuum eingeengt und 15,7 g 2-(2,4-Dichlor-3,6-difluorbenzoyl)-3-ethoxy-acrylsäureethylester als Öl erhalten; n 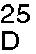 1,5302.

### c) 2-(2,4-Dichlor-3,6-difluorbenzoyl)-3-cyclopropylamino-acrylsäureethylester

15,6 g (2,4-Dichlor-3,6-difluorbenzoyl)-3-ethoxyacrylsäureethylester werden in 50 ml Ethanol gelöst und unter Kühlung 2,75 g Cyclopropylamin zugetropft. Man rührt 1 Stunde bei Raumtemperatur, versetzt unter Eiskühlung mit 50 ml Wasser, saugt ab, wäscht mit Ethanol/H₂0 (1:1) nach und trocknet. Es werden 14,1 g 2-(2,4-Dichlor-3,6-difluorben- zoyl)-3-cyclopropylamino-acryloäureethylester vom Schmelzpunkt 106-107°C erhalten.

### d) 5,7-Dichlor-1-cyclopropyl-6-fluor-1,4-dihydro-4-oxo-3-chinolincarbonsäureethylester

6 g 2-(2,4-Dichlor-3,6-difluorbenzoyl)-3-cyclopropylamino-acrylsäureethylester werden in 100 ml Dimethylformamid mit 2,75 g Kaliumcarbonat 2,5 Stunden auf 150°C erhitzt. Die Mischung wird auf 600 ml Eiswasser gegossen, der Niederschlag abgesaugt, mit Wasser gewaschen und getrocknet. Es werden 5,2 g 5;7-Dichlor-1-cyclopropyl-6-fluor-1,4-dihydro-4-oxo-3-chinolincarbonsäureethylestervom Schmelzpunkt 227-229°C erhalten.

### e) 5,7-Dichlor-1-cyclopropyl-6-fluor-1,4-dihydro-4-oxo-3-chinolincarbonsäure

5,2 g 5,7-Dichior-1-cyclopropyl-6-fluor-1,4-dihydro-4-oxo-3-chinolincarbonsäureethylester werden in einer Mischung aus 38 ml Essigsäure, 30 ml Wasser und 4,3 ml konzentrierter Schwefelsäure 2,5 Stunden unter Rückfluß erhitzt. Nach dem Abkühlen wird auf 250 ml Eiswasser gegossen, der Niederschlag abgesaugt, mit Wasser gewaschen und getrocknet. Es werden 4,8 g 5,7-Dichlor-1-cyclopropyl-6-fluor-1,4-dihydro-4-oxo-3-chinolincarbonsäure vom Schmelzpunkt 277-278°C erhalten.

### Beispiel AC

### 5,7-Dichlor-6-fluor-1-(2,4-difluorphenyl)-1,4-dihydro-4-oxo-3-chinolincarbonsäure

### a) 2-(2,4-Dichlor-3,6-difluorbenzoyl)-3-(2,4-difluorphenylamino)-acrylsäureethylester

35,3 g 2-(2,4-Dichlor-3,6-difluorbenzoyl)-3-ethoxy-acrylsäureethylester werden in 120 ml Ethanol gelöst und unter Eiskühlung 12,9 g 2,4-Difluoranilin zugetropft. Man rührt 1,5 Stunden bei Raumtemperatur, versetzt unter Kühlung mit 120 ml Wasser saugt ab, wäscht mit Ethanol/H₂0 (1:1) nach und trocknet. Es werden 40,5 g 2-(2,4-Dichlor-3,6-difluor- benzoyl)-3-(2,4-difluorphenylamino)-acrylsäureethylester erhalten. Schmelzpunkt: 84-86°C.

### b) 5,7-Dichlor-6-fluor-1-(2,4-difluorphenyl)-1,4-dihydro-4-oxo-3-chinolincarbonsäureethylester

43,6 g 2-(2,4-Dichlor-3,6-difluorbenzoyl)-3-(2,4-difluorphenylamino)-acrylsäureethylester werden in 260 ml Dimethylformamid mit 15,2 g Kaliumcarbonat 2,5 Stunden auf 150°C erhitzt. Die Mischung wird auf 1 Liter Eiswasser gegossen, der Niederschlag abgesaugt, mit Waser gewaschen und getrocknet. Es werden 38,6 g 5,7-Dichlor-6-fluor-1-(2,4-difluorphenyl)-1,4-dihydro-4-oxo-3-chinolincarbonsäureethylester erhalten.

### c) 5,7-Dichlor-6-fluor-1 -(2,4-difluorphenyl)-1,4-dihydro-4-oxo-3-chinolincarbonsäure

41,6 g 5,7-Dichlor-6-fluor-1-(2,4-difluorphenyl)-1,4-dihydro-4-oxo-3-chinolincarbonsäureethylester werden mit 250 ml Essigsäure, 200 ml Wasser und 28,5 ml konzentrierter Schwefelsäure 3 Stunden unter Rückfluß erhitzt. Nach dem Abkühlen wird auf 2 Liter Eiswasser gegossen, der Niederschlag abgesaugt, mit Wasser gewaschen und getrocknet. Es werden 35,5 g 5,7-Dichlor-6-fluor-1-(2,4-difluorphenyl)-1,4-dihydro-4-oxo-3-chinolincarbonsäure erhalten. Schmelzpunkt: 244-246°C.

### Beispiei 1

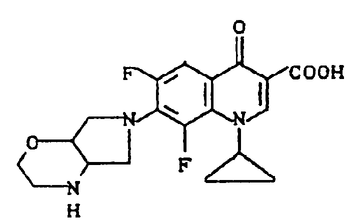

850 mg (3 mmol) 1-Cyclopropyl-6,7,8-trifluor-1,4-dihydro-4-oxo-3-chinolincarbonsäure werden in 9 ml Pyridin mit 630 mg (3,1 mmol) 2-Oxa-5,8-diazabicyclo[4.3.0]nonan-Dihydrochlorid und 500 mg (4,5 mmol) 1,4-Diazabicyclo[2.2.2] octan 1 Stunde unter Rückfluß erhitzt. Die Mischung wird eingeengt, der Rückstand mit Wasser verrührt, der Niederschlag abgesaugt, mit Wasser gewaschen, getrocknet und aus Glykolmonomethylether umkristallisiert.
Ausbeute: 840 mg (72% der Theorie) 1-Cyclopropyl-6,8-difluor-1,4-dihydro-7-(2-oxa-5,8-diazabicyclo[4.3.0]non-8-yl)-4-oxo-3-chinolincarbonsäure,
Schmelzpunkt: 289-291 °C (unter Zersetzung);
Massenspektrum: ^{m}/e 391 (M⁺), 347 (H⁺⁻C0₂), 331, 306, 294, 262, 234, 98, 41 (C3H5)

### Beispiel 2

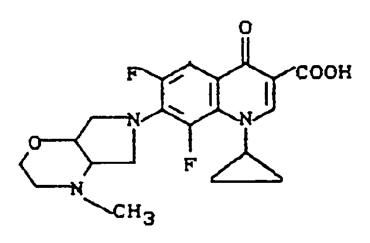

Analog Beispiel 1 setzt man mit 5-Methyl-2-oxa-5,8-diazabicyclo[4.3.0]nonan-Dihydrochlorid um und erhält:
1-Cyclopropyl-6,8-difluor-1,4-dihydro-7-(5-methyl-2-oxa-5, 8-diazabicyclo[4.3.0] non-8-yl )-4-oxo-3-chinolincar-bonsäure, Schmelzpunkt: ab 270°C (unter Zersetzung);
Massenspektrum: m/e 405 (M⁺), 361 (M⁺⁻C0₂), 331, 112, (100%).

### Beispiel 3

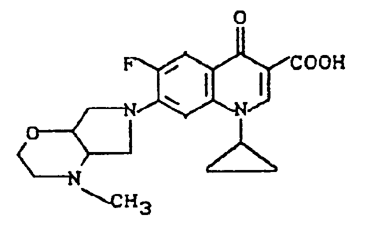

795 mg (3 mmol) 1-Cyclopropyl-6,7-difluor-1,4-dihydro-4-oxo-3-chinolincarbonsäure werden in einer Mischung aus 9 ml Acetonitril und 4,5 ml Dimethylformamid mit 890 mg (4,1 mmol) 5-Methyl-2-oxa-5,8-diazabicyclo[4.3.0]nonan-Dihydrochlorid und 860 mg (7,8 mmol) 1,4-Diazabicyclo-[2.2.2]octan 2 Stunden unter Rückfluß erhitzt. Die Mischung wird eingedampft, mit Wasser verrührt, das ungelöste Produkt wird abgesaugt, mit Wasser gewaschen, getrocknet und aus Dimethylformamid umkristallisiert. Ausbeute: 0,8 g (69% der Theorie) 1-Cyclopropyl-6-fluor-1,4-dihydro-7-(5-methyl-2-oxa-5,8-diazabicyclo[4.3.0]-non-8-yl)-4-oxo-3-chinolincarbonsäure, Schmelzpunkt 340°C (unter Zersetzung) (die Substanz wird bereits beim Aufheizen ab etwa 300° dunkel).
Massenspektrum: ^{m}/e (M⁺), 343 (M⁺⁻C0₂), 313, 244, 112, (100%).

### Beispiel 4

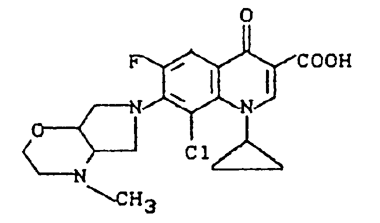

Analog Beispiel 3 setzt man mit 8-Chlor-1-cyclopropyl-6,7-difluor-1,4-dihydro-4-oxo-3-chinolincarbonsäure um und erhält 8-Chlor-1-cyclopropyl-6-fluor-1,4-dihydro-7-(5-methyl-2-oxa-5,8-diazabicyclo[4.3.0]non-8-yl)-4-oxo-3-chi- nolincarbonsäure, Schmelzpunkt 258-262°C (unter Zersetzung) (umkristallisiert aus Dimethylformamid).

### Beispiel 5

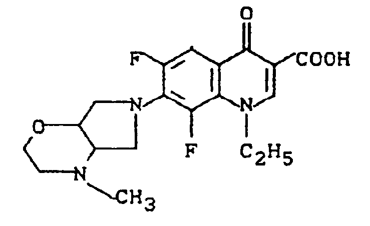

Analog Beispiel 3 setzt man mit 1-Ethyl-6,7,8-trifluor-1,4-dihydro-4-oxo-3-chinolincarbonsäure um und erhält 1-Ethyl-6,8-difluor-1,4-dihydro-7-(5-methyl-2-oxa-5,8-diazabicyclo[4.3.0]non-8-yl)-4-oxo-3-chinolincarbonsäure, Schmelzpunkt 279-281 °C (unter Zersetzung).

### Beispiel 6

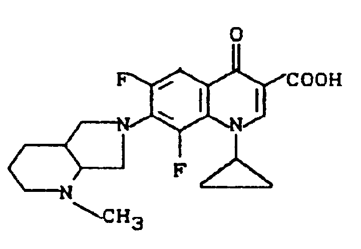

0,84 g (3 mmol) 1-Cyclopropyl-6,7,8-trifluor-1,4-dihydro-4-oxo-3-chinolincarbonsäure werden in einer Mischung aus 6 ml Acetonitril und 3 ml Dimethylformamid mit 0,66 g (6 mmol) 1,4-Diazabicyclo[2.2.2]octan und 0,49 g (3,5 mmol) 2-Methyl-2,8-diazabicyclo[4.3.0]nonan 2 Stunden unter Rückfluß erhitzt. Die Suspension wird eingeengt, mit 20 ml Wasser verrührt, mit 2n-Salzsäure auf pH 7 eingestellt, der Niederschlag wird abgesaugt, mit Wasser gewaschen, getrocknet und aus Glykolmonomethylether umkristallisiert.

Ausbeute: 0,7 g (58% der Theorie) 1-Cyclopropyl-6,8-difluor-1,4-dihydro-7-(2-methyl-2,8-diazabicyclo[4.3.0]-non-8-yl)-4-oxo-3-chinolincarbonsäure, Schmelzpunkt 204-207°.

### Beispiel 7

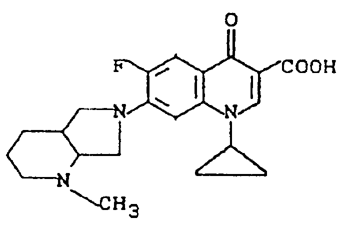

Analog Beispiel 6 erhält man mit 1-Cyclopropyl-6,7-difluor-1,4-dihydro-4-oxo-3-chinolincarbonsäure 1-Cyclopropyl-6-fluor-1,4-dihydro-7-(2-methyl-2,8-diazabicyclo[4.3.0]non-8-yl)-4-oxo-3-chinolincarbonsäure, Schmelzpunkt 234-236°.

### Beispiel 8

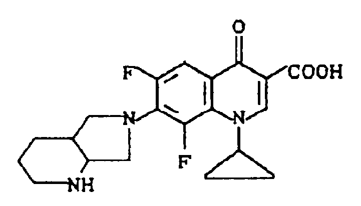

A Man setzt analog Beispiel 6 1-Cyclopropyl-6,7,8-trifluor-1,4-dihydro-4-oxo-3-chinalincarbonsäure mit 2,8-Diazabicyclo[4.3.0]nonan um und erhält 1-Cyclopropyl-7-(2,8-diazabicyclo[4.3.0]non-8-yl)-6,8-difluor-1,4-dihydro-4-oxo-3-chinolincarbonsäure, Schmelzpunkt 265-267° (unter Zersetzung) (umkristallisiert aus Dimethylformamid).
B. Führt man die Umsetzung des Beispiels 8 A) in einer Mischung aus Acetonitril/1-Methyl-2-pyrrolidinon durch und kristallisiert das Rohprodukt aus Dimethylformamid um, dann erhält man 1-Cyclopropyl-7-(2,8-diazabicyclo [4.3.0]non-8-yl)-6,8-difluor-1,4-dihydro-4-oxo-3-chinolincarbonsäure vom Schmelzpunkt 269-271 °C (unter Zersetzung). Das Produkt ist nach chromatographischem und spektroskopischem Vergleich identisch mit dem nach Verfahren A) hergestelltem Produkt.
C. 65 g (167 mmol) des Betains (Stufe A) werden in 330 ml halbkonzentrierter Salzsäure durch Erwärmen gelöst, die Lösung wird eingeengt und der Rückstand mit 300 ml Ethanol verrührt. Der ungelöste Niederschlag wird abgesaugt, mit Ethanol gewaschen und bei 100°C in Vakuum getrocknet.

Ausbeute: 66,3 g (93 % der Theorie) 1-Cyclopropyl-7-(2,8-diazabicyclo[4.3.0]non-8-yl)-6,8-difluor-1,4-dihydro-4-oxo-3-chinolincarbonsäure-hydrochlorid,
Schmelzpunkt: 303-305°C (unter Zersetzung).

### Beispiel 9

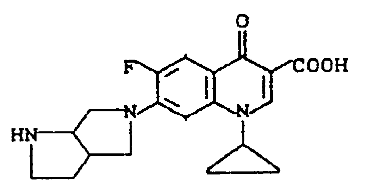

Analog Beispiel 6 erhält man mit 1-Cyclopropyl-6,7-difluor-1,4-dihydro-4-oxo-3-chinolincarbonsäure und 2,7-Diazabicyclo[3.3.0]octan 1-Cyclopropyl-7-(2,7-diazabicyclo[3.3.0]oc-7-yl)-6-fluor-1,4-dihydro-4-oxo-3-chinolincarbon- säure, Schmelzpunkt: 260-282° (unter Zersetzung).
Massenspektrum: ^{m}/e 357 (M⁺), 313 (100%, M⁺⁻C0₂), 269, 257, 244, 82, 28.

### Beispiel 10

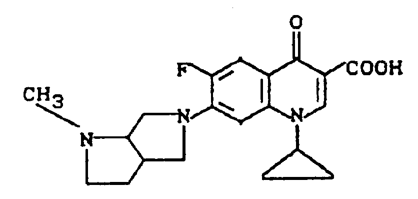

Analog Beispiel erhält man mit 1-Cyclopropyl-6,7-difluor-1,4-dihydro-4-oxo-3-chinolincarbonsäure und 2-Methyl-2,7-diazabicyclo[3.3.0]octan 1-Cyclopropyl-6-fluor-1,4-dihydro-7-(2-methyl-2,7-diazabicyclo[3.3.0]-oct-7-yl)-4-oxo-3-chinolincarbonsäure, Schmelzpunkt: 206-208°C (unter Zersetzung).

### Beispiel 11

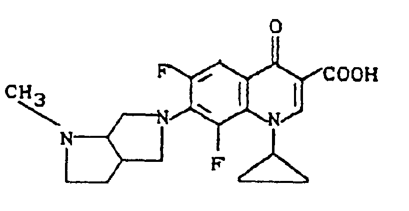

Analog Beispiel 6 erhält man mit 2-Methyl-2,7-diazabicyclo[3.3.0]octan 1-Cyclopropyl-6,8-difluor-1,4-dihydro-7-12-methyl-2,7-diazabicyclo[3.3.0]oct-7-yl)-4-oxo-3-chinolincarbonsäure, Schmelzpunkt 198-200°C (unter Zersetzung).

### Beispiel 12

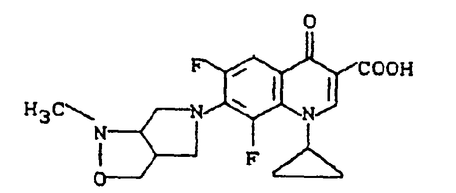

Eine Mischung aus 2,83 g (10 mmol) 1-Cyclopropyl-6,7,8-trifluor-1,4-dihydro-4-oxo-3-chinolincarbonsäure, 1,1 g (10 mmol) 1,4-Diazabicyclo[2.2.2]octan und 1,4 g (11 mmol) 2-Methyl-3-oxa-2,7-diazabicyclo[3.3.0]octan in 20 ml Acetonitril und 10 ml 1-Methyl-2-pyrrolidinon wird 1 Stunde unter Rückfluß erhitzt. Man engt im Vakuum ein, verrührt den Rückstand mit Wasser (pH 7), saugt den Niederschlag ab, wäscht mit Wasser und trocknet bei 60° im Vakuum. Das Rohprodukt (3,7 g) wird aus Dimethylformamid umkristallisiert.

Ausbeute: 1,9 g (49% der Theorie) 1-Cyclopropyl-6,8-difluor-1,4-dihydro-7-(2-methyl-3-oxa-2,7-diazabicyclo [3.3.0]oct-7-yl)-4-oxo-3-chinolincarbonsäure, Schmelzpunkt 221-223°C (unter Zersetzung).

### Beispiel 13

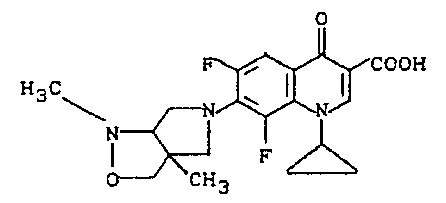

Analog Beispiel 12 wird mit 2,5-Dimethyl-3-oxa-2,7-diazabicyclo[3.3.0]octan zu 1-Cyclopropyl-6,8-difluor-1,4-di- hydro-7-(2,5-dimethyl-3-oxa-2,7-diazabicyclo[3.3.0]-oct-7-yl)-4-oxo-3-chinolincarbonsäure vom Schmelzpunkt 237-238°C (unter Zersetzung) umgesetzt.

### Beispiel 14

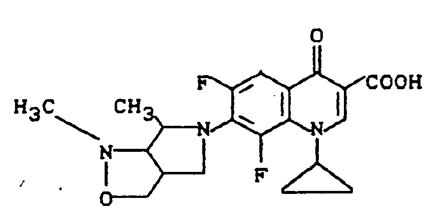

Analog Beispiel 12 wird mit 2,8-Dimethyl-3-oxa-2,7-diazabicyclo[3.3.0]octan zu 1-Cyclopropyl-6,8-difluor-1,4-di- hydro-7-(2,8-dimethyl-3-oxa-2,7-diazabicyclo[3.3.0]-oct-7-yl]-4-oxo-3-chinolincarbonsäure vom Schmelzpunkt 197-199°C umgesetzt.

### Beispiel 15

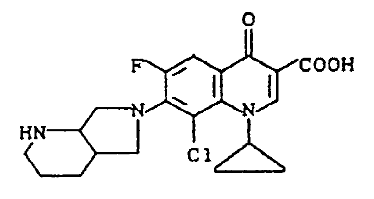

A 3 g (10 mmol) 8-Chlor-1-cyclopropyl-6,7-difluor-1,4-dihydro-4-oxo-3-chinolincarbonsäure werden in einer Mischung aus 30 ml Acetonitril und 15 ml 1-Methyl-2-pyrrolidinon mit 1,4 g (11 mmol) 2,8-Diazabicyclo-[4.3.0]nonan und 1,65g(15 mmol) 1,4-Diazabicyclo[2.2.2]-octan 1 Stunde unter Rückfluß erhitzt. Die Suspension wird nach dem Abkühlen mit etwa 150 ml Wasser verrührt, der ungelöste Niederschlag abgesaugt, mit Wasser und Ethanol gewaschen und bei 80°C/12 mbar getrocknet. Das Rohprodukt wird aus 40 ml Glykolmonomethylether umkristallisiert.
   Ausbeute 2,3 g (57 % der Theorie) 8-Chlor-1-cyclopropyl-7-(2,8-diazabicyclo[4.3.0]non-8-yl)-6-fluor-1,4-dihydro-4-oxo-3-chinolincarbonsäure, Schmelzpunkt: 224-226°C (unter Zersetzung).
B. Man stellt analog Beispiel 15 A. das rohe Betain her, suspendiert dieses in 50 ml Wasser und bringt es durch Zugabe von 17 ml 1 n-Salzsäure und Erwärmen in Lösung. Nach dem Abkühlen im Eisbad wird der ausgefallene Niederschlag abgesaugt, mit Ethanol gewaschen und bei 100°C im Vakuum getrocknet.
   Ausbeute 2,7 g (61 % der Theorie) 8-Chlor-1-cyclopropyl-7-(2,8-diazabicyclo[4.3.0]non-8-yl)-6-fluor-1,4-dihydro-4-oxo-3-chinolincarbonsäure-hydrochlorid,
   Schmelzpunkt: ab 225°C Zersetzung.

### Beispiel 16

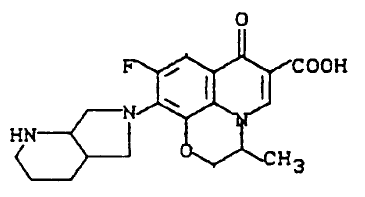

Analog Beispiel 15 wird die Umsetzung mit 9,10-Difluor-2,3-dihydro-3-methyl-7-oxo-7H-pyridol[1,2,3-de][1,4]ben- zoxacin-6-carbonsäure durchgeführt und das erhaltene Reaktionsprodukt durch Chromatographie an Kieselgel mit Dichlormethan/Methanol/17 %iger wäßriger Ammoniaklösung (30:8:1) als Laufmittel gereinigt. Man erhält 10-(2,8-Diazabicyclo[4.3.0]non-8-yl)-9-fluor-2,3-dihydro-3-methyl-7-oxo-7H-pyrido[1,2,3-de][1,4]benzoxacin-6-carbonsäure vom Schmelzpunkt 291-292°C (unter Zersetzung).

### Beispiel 17

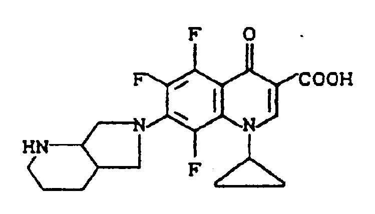

6 g (20 mmol) 1 -Cyclopropyl-5,6,7,8-tetrafluor-1,4-dihydro-4-oxo-3-chinolincarbonsäure werden in 30 ml 1-Methyl-2-pyrrolidinon und 60 ml Acetonitril mit 2,2 g (20 mmol) 1,4-Diazabicyclo[2.2.2]octan und 2,7 g (21,4 mmol) 2,8-Diazabicyclo[4.3.0]nonan 1 Stunde unter Rückfluß erhitzt. Die Mischung wird im Vakuum weitgehend eingeengt, der Rückstand mit 200 ml Wasser verrührt, das ungelöste Kristallisat abgesaugt,mit Wasser gewaschen und getrocknet.
Ausbeute 6,3 g (77,4 % der Theorie) 1-Cyclopropyl-7-(2,8-diazabicyclo[4.3.0]non-8-yl]-5,6,8-trifluor-1,4-dihydro-4-oxo-3-chinolincarbonsäure
Schmelzpunkt: 266-269°C (unter Zersetzung); nach Umkristallisation aus Dimethylformamid : Schmelzpunkt: 272-273°C (unter Zersetzung).

### Beispiel 18

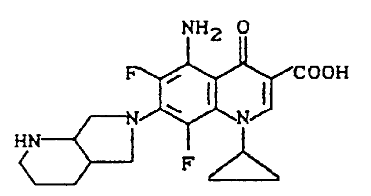

4,1 g (10 mmol) des Produktes aus Beispiel 17 werden in 40 ml Pyridin mit 20 ml gesättigter ethanolischer Ammoniak-Lösung versetzt und die Mischung im Autoklaven 12 Stunden auf 120°C erhitzt. Die Suspension wird eingedampft, der Rückstand mit Wasser verrührt und mit 2n-Salzsäure auf pH 7 eingestellt. Der ausgefallene Niederschlag wird abgesaugt und aus Glykolmonomethylether umkristallisiert.
Ausbeute 0,7 g (17 % der Theorie) 5-Amino-1-cyclopropyl-7-(2,8-diazabicyclo[4.3.0]non-8-yl)-6,8-difluor-1,4-di- hydro-4-oxo-3-chinolincarbonsäuro, Schmelzpunkt: 275-277°C (unter Zersetzung).
Massenspektrum: ^{m}/e 404 (M₊), 384 (M₊-HF), 290, 249, 96 (100 %).

### Beispiel 19

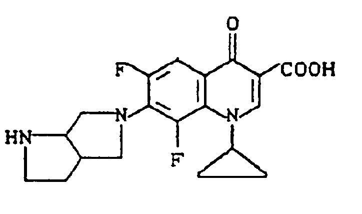

A. Analog Beispiel 6 erhält man mit 2,7-Diazabicyclo[3.3.0]octan 1-Cyclopropyl-7-(2,7-diazabicyclo-[3.3.0]oct-7-yl)-6,8-difluor-1,4-dihydro-4-oxo-3-chinolincarbonsäure, Schmelzpunkt: 277-280° (unter Zersetzung).
B. 370 mg des Betains werden in 13 ml halbkonzentrierter Salzsäure gelöst, die Lösung eingeengt und der Rückstand mit 10 ml Ethanol behandelt. Das ungelöste Produkt wird abgesaugt, mit Ethanol gewaschen und getrocknet Ausbeute: 290 mg 1-Cyclopropyl-7-(2,7-diazabicyclo-[3.3.0]oct-7-yl)-6,8-difluor-1,4-dihydro-4-oxo-3-chinolincar- bonsäure-hydrochlorid, Schmelzpunkt: 269-271 °C (unter Zersetzung).

### Beispiel 20

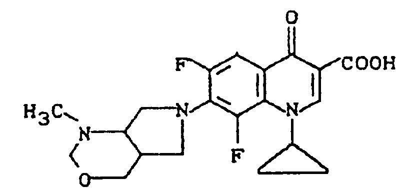

Analog Beispiel 6 erhält man mit 2-Methyl-4-oxo-2,8-diazabicyclo[4.3.0]nonan 1 -Cyclopropyl-6,8-difluor-1,4-dihy- dro-7-(2-methyl-4-oxa-2,8-diazabicyclo[4.3.0]non-8-yl)-4-oxo-3-chinolincarbonsäure, Schmelzpunkt 258-260°C (unter Zersetzung).

### Beispiel 21

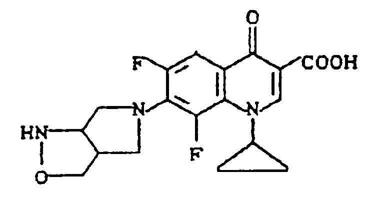

Analog Beispiel 12 erhalt man mit 3-Oxa-2,7-diazabicyclo[3.3.0]octan 1-Cyclopropyl-6,8-difluor-1,4-dihydro-7-(3-oxa-2,7-diazabicyclo[3.3.0]octan-7-yl)-4-oxo-3-chinolincarbonsäure.

### Beispiel 22

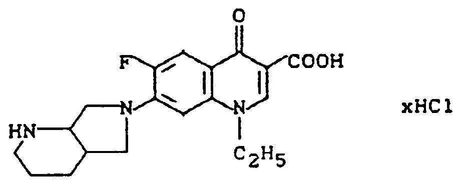

A 2,53 g (10 mmol) 1-Ethyl-6,7-difluor-1,4-dihydro-4-oxo-3-chinolincarbonsäure werden in 30 ml Acetonitril und 15 ml Dimethylformamid mit 1,1 g (10 mmol) 1,4-Diazabicyclo[2.2.2]octan und 1,4 g (11 mmol) 2,8-Diazabicyclo [4.3.0]nonan versetzt und 1 Stunde unter Rückfluß erhitzt. Die Mischung wird eingeengt, mit Wasserver rührt und der Niederschlag abgesaugt, mit Wasser gewaschen und getrocknet. Ausbeute: 3,1 g (86 % der Theorie) 7-(2,8-Diazabicyclo(4.3.0]non-8-yl)-1-ethyl-6-fluor-4-oxo-3-chinolincarbonsäure, Schmelzpunkt: 259-261°C (unter Zersetzung).
B. 2,9 g (8 mmol) des Betains aus Stufe A werden in 20 ml halbkonzentrierter Salzsäure in der Wärme gelöst, die Lösung heiß filtriert und aus dem Filtrat durch Zusatz von Ethanol das Hydrochlorid ausgefällt. Dieses wird abgesaugt, mit Ethanol gewaschen und bei 120°C/12 mbar getrocknet.
   Ausbeute: 1,8 g (57 der Theorie) 7-(2,8-Diazabicyclo-[4.3.0]non-8-yl)-1-ethyl-6-fluor-4-oxo-3-chinolincarbonsäu- re-Hydrochlorid, Schmelzpunkt unter Zersetzung: 299°C (bereits ab ca. 215°C beginnende Dunkelfärbung).

### Beispiel 23

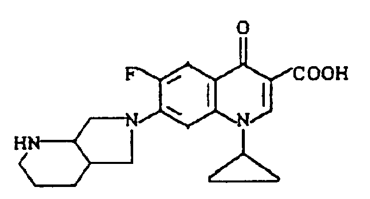

Analog Beispiel 22 erhält man mit 1-Cyclopropyl-6,7-difluor-1,4-dihydro-4-oxo-3-chinolincarbonsäure:
A. 1-Cyclopropyl-7-(2,8-diazabicyclo[4.3.0]non-8-yl)-6-fluor-4-oxo-3-chinolincarbonsäure, Schmelzpunkt: 249-257°C (unter Zersetzung)
B. 1-Cyclopropyl-7-(2,8-diazabicyclo[4.3.0]non-8-yl)-6-fluor-4-oxo-3-chinolincarbonsäure-Hydrochlorid, Schmelzpunkt unter Zersetzung: 320°C (bereits ab ca. 288°C beginnende Dunkelfärbung).

### Beispiel 24

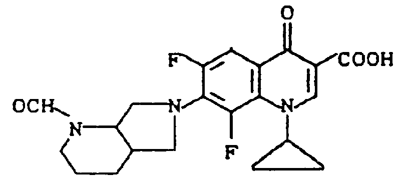

1,1 g (3 mmol) 1-Cyclopropyl-7-(2,8-diazabicyclo[4.3.0]-non-8-yl)-6,8-difluor-1,4-dihydro-4-oxo-3-chinolincarbon- säure werden in 10 ml Dimethylformamid und 1 ml Ameisensäure 4 Stunden unter Rückfluß erhitzt. Die Mischung wird eingedampft, der Rückstand mit 4 ml Wasser verrührt, der Niederschlag abgesaugt, getrocknet (Rohausbeute: 1 g, Gehalt: 99,5 %) und aus Dimethylformamid umkristallisiert. Ausbeute: 0,8 g (64 % der Theorie) 1-Cyclopropyl-6,8-difluor-7-(2-formyl-2,8-diazabicyclo[4.3.0]non-8-yl)-1,4-dihydro-4-oxo-3-chinolincarbonsäure, Schmelzpunkt: 276-278°C.

### Beispiel 25

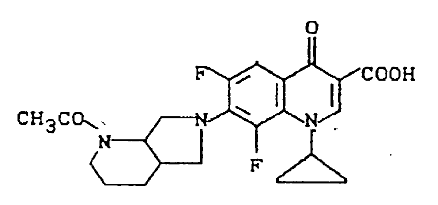

1,1 g (3 mmol) 1-Cyclopropyl-7-(2,8-diazabicyclo[4.3.0]-non-8-yl)-6,8-difluor-1,4-dihydro-4-oxo-3-chinolincarbon- säure werden in einer Mischung aus 8 ml Dioxan und einer Lösung von 120 mg Natriumhydroxid in 1 ml Wasser gelöst und unter Eiskühlung gleichzeitig mit 3 ml 1 n-Natronlauge und 260 mg Acetylchlorid versetzt. Man läßt 2 Stunden bei Raumtemperatur nachrühren, verdünnt mit 30 ml Wasser und saugt den ausgefallenen Niederschlag ab, Das Rohprodukt wird aus Glykolmonomethylether umkristallisiert.
Ausbeute: 0,6 g (46 % der Theorie) 7-(2-Acetyl-2,8-diazabicyclo[4.3.0]non-8-yl)-1-cyclopropyl-6,8-difluor-1,4-dihydro-4-oxo-3-chinolincarbonsäure, Schmelzpunkt: 261-263°C (unter Zersetzung)

### Beispiel 26

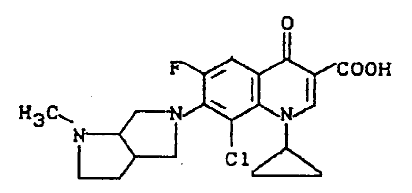

A Analog Beispiel 6 erhält man mit 8-Chlor-1-cyclopropyl-6,7-difluor-1,4-dihydro-4-oxo-3-chinolincarbonsäure und 2-Methyl-2,7-diazabicyclo[3.3.0]octan 8-Chlor-1 -cyclopropyl-6-fluor-1,4-dihydro-7-(2-methyl-2,7-diazabicyclo [3.3.0]oct-7-yl)-4-oxo-3-chinolincarbonsäure, Schmelzpunkt: 222-227°C (unter Zersetzung).
B. 2,3 g (5,8 mmol) des Betains aus Stufe A werden in 15 ml 1n-Salzsäure in der Wärme gelöst, die Lösung eingedampft und der Rückstand mit Ethanol behandelt. Der Niederschlag wird abgesaugt, mit Ethanol gewaschen und getrocknet.
   Ausbeute: 2,2 g (87,7 % der Theorie) 8-Chlor-1-cyclopropyl-6-fluor-1,4-dihydro-7-(2-methyl-2,7-diazabicyclo-[3.3.0]oct-7-yl)-4-oxo-3-chinolincarbonsäure-hydrochlorid; Schmelzpunkt: 303-305°C (unter Zersetzung).

### Beispiel 27

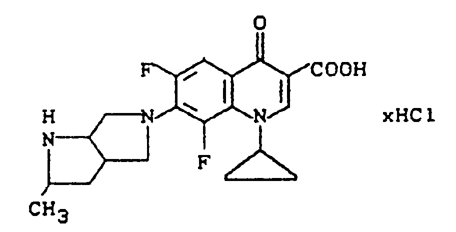

Analog Beispiel 6 wird mit 3-Methyl-2,7-diazabicyclo-[3.3.0]octan 1-Cyclopropyl-6,8-difluor-1,4-dihydro-7-(3-methyl-2,7-diazabicyclo[3.3.0]oct-7-yl)-4-oxo-3-chinolincarbonsäure erhalten und analog Beispiel 8 C. mit halbkonzentrierter Salzsäure in 1 -Cyclopropyl-6,8-difluor-1,4-dihydro-7-(3-methyl-2,7-diazabicyclo[3.3.0]-oct-7-yl)-4-oxo-3-chino- lincarbonsäure-Hydrochlorid, Schmelzpunkt 216-221°C (unter Zersetzung) überführt.

### Beispiel 28

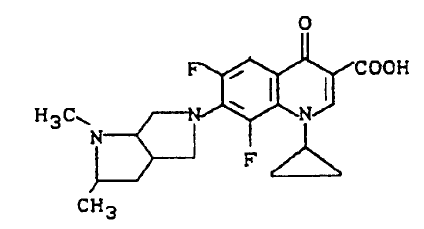

A. Eine Mischung aus 1,45 g (5 mmol) 1-Cyclopropyl-6,7,8-trifluor-1,4-dihydro-4-oxo-3-chinolincarbonsäure, 0,85 g (7,5 mmol) 1,4-Diazabicyclo[2.2.2]octan und 0,77 g (5,5 mmol) 2,3-Dimethyl-2,7-diazabicyclo[3.3.0]-octan in 15 ml Acetonitril und 7,5 ml Dimethylformamid wird 1 Stunde unter Rückfluß erhitzt. Nach dem Abkühlen wird der Niederschlag abgesaugt, mit Wasser gewaschen und aus Glykolmonomethylether umkristallisiert. Ausbeute: 1 g (47 % der Theorie) 1-Cyclopropyl-7-(2,3-dimethyl-2,7-diazabicyclo[2.2.2]oct-7-yl)-6,8-difluor-1,4-dihydro-4-oxo-3-chinolincarbonsäure, Schmelzpunkt: 208-209°C (unter Zersetzung).
B. 0,7 g (1,7 mmol) des Betains aus Stufe A werden in 6 ml halbkonzentrierter Salzsäure heiß gelöst, die Lösung filtriert und im Vakuum weitgehend konzentriert. Man versetzt mit etwa 15 ml Ethanol, kühlt im Eisbad, saugt das Salz ab, wäscht mit Ethanol und trocknet bei 100°C/1 mbar.
   Ausbeute: 0,64 g (84 % der Theorie) 1-Cyclopropyl-7-(2,3-dimethyl-2,7-diazabicyclo[2.2.2]oct-7-yl)-6,8-difluor-1,4-dihydro-4-oxo-3-chinolincarbonsäure-Hydrochlorid, Schmelzpunkt: 233-236°C (unter Zersetzung).

### Beispiel 29

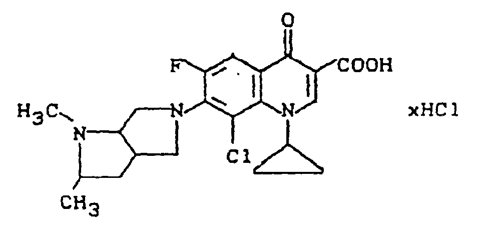

Analog Beispiel 28 A. und B. erhält man mit 8-Chlor-1-cyclopropyl-6,7-difluor-1,4-dihydro-4-oxo-3-chinolincarbon- säure 8-Chlor-1-cyclopropyl-7-(2,3-dimethyl-2,7-diazabicyclo[2.2.2]oct-7-yl)-6-fluor-1,4-dihydro-4-oxo-3-chinolincar- bonsäure-Hydrochlorid, Schmelzpunkt: 240-241 °C (unter Zersetzung).

### Beispiel 30

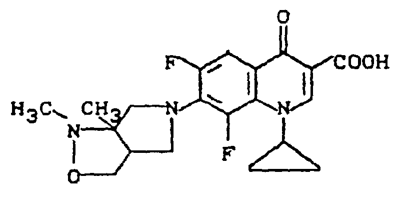

Analog Beispiel 12 wird mit 1,2-Dimethyl-3-oxa-2,7-diazabicyclo[3.3.0]octan zu 1-Cyclopropyl-6,8-difluor-1,4-di- hydro-7-(1,2-dimethyl-3-oxa-2,7-diazabicyclo[3.3.0]-oct-7-yl)-4-oxo-3-chinolincarbonsäure vom Schmelzpunkt 269-271 °C (unter Zersetzung) umgesetzt

### Beispiel 31

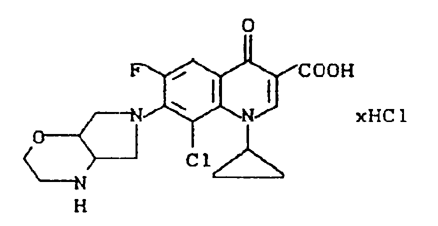

2,6 g (8,7 mmol) 8-Chlor-1-cyclopropyl-6,7-difluor-1,4-dihydro-4-oxo-3-chinolincarbonsäure werden in einer Mischung aus 25 ml Acetonitril und 12,5 ml Dimethylformamid mit 1,45 g (13 mmol) 1,4-Diazabicyclo[2.2.2]octan und 1,23 g (9,6 mmol) 2-Oxa-5,8-diazabicyclo[4.3.0]nonan versetzt und 1 Stunde unter Rückfluß erhitzt. Die Mischung wird eingeengt, der Rückstand mit Wasser verrührt und der ungelöste Niederschlag abgesaugt und mit Wasser gewaschen. Diese rohe 1-Cyclopropyl-8-chlor-6-fluor-1,4-dihydro-7-(2-oxa-5,8-diazabicyclo[4.3.0]non-8-yl)-4-oxo-3-chinolincar- bonsäure wird in 85 ml 1 n-Salzsäure eingetragen und mit 6 ml konzentrierter Salzsäure versetzt. Das ausgefallene Hydrochlorid wird abgesaugt, mit Ethanol gewaschen und getrocknet.
Ausbeute: 3,0 g (77,7 % der Theorie) 8-Chlor-1-cyclopropyl-6-fluor-1,4-dihydro-7-(2-oxa-5,8-diazabicyclo-[4.3.0]non-8-yl)-4-oxo-3-chinolincarbonsäuro-Hydrochlorid, Schmelzpunkt: ab 290°C Zersetzung.

### Beispiel 32

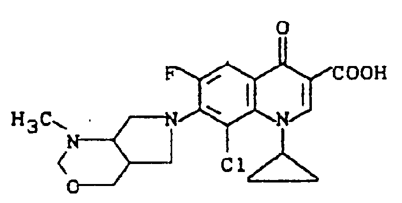

Analog Beispiel 6 erhält man mit 8-Chlor-l-cyclopropyl-6,7-difluor-1,4-dihydro-4-oxo-3-chinolincarbonsäure und 2-Methyl-4-oxa-2,8-diazabicyclo[4.3.0]nonan 8-Chlor-1-cyclopropyl-6-fluor-7-(2-methyl-4-oxa-2,8-diazabicyclo-[4.3.0]non-8-yl)-4-oxo-3-chinolincarbonsäure, Schmelzpunkt: 202-203°C (unter Zersetzung)
FAB-Massenspektrum : ^{m}/e 422 ([M+H]⁺), 404 (422-H₂0).

### Beispiel 33

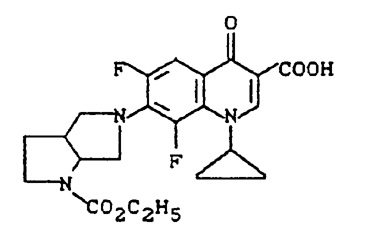

A. Analog Beispiel 6 wird mit 2,7-Diazabicyclo-[3.3.0]octan-2-carbonsäureethylester zu 1-Cyclopropyl-7-(2-ethoxycarbonyl-2,7-diazabicyclo[3.3.0]oct-7-yl)-6,8-difluor-1,4-dihydro-4-oxo-3-chinolincarbonsäure vom Schmelzpunkt 191-192°C umgesetzt.
B. 1,8 g(4 mmol) des Produktes aus Beispiel 33A werden in 30 ml konzentrierter Salzsäure unter leichtem Rückfluß während 15 Stunden erhitzt Die Lösung wird eingeengt, der Rückstand mit Ethanol verrührt, der Niederschlag abgesaugt, mit Ethanol gewaschen und bei 120°C/12 mbar getrocknet.
   Ausbeute: 1,1 g (67 % der Theorie) 1-Cyclopropyl-7-(2,7-diazabicyclo[3.3.0]oct-7-yl)-6,8-difluor-1,4-dihydro-4-oxo-3-chinolincarbonsäure-hydrochlorid, Schmelzpunkt: 273-275°C (unter Zersetzung). Das Produkt ist identisch mit der nach Beispiel 26B erhaltenen Verbindung.

### Beispiel 34

A 7,8 g (20 mmol) 1-Cyclopropyl-7-(2,8-diazabicyclo-[4.3.0]non-8-yl)-6,8-difluor-1;4-dihydro-4-oxo-3-chinolincar- bonsäure werden in 175 ml Ethanol eingetragen und bei etwa 70°C mit 2,4 g (25 mmol) Methansulfonsäure versetzt. Das Betain löst sich auf und beim Abkühlen fällt das Salz aus, das abgesaugt, mit Ethanol gewaschen und bei 120°C/12 mbar getrocknet wird. Es ist in Wasser leicht löslich.
   Ausbeute: 8,6 g (88,6 % der Theorie) 1-Cyclopropyl-7-(2,8-diazabicyclo[4.3.0]non-8-yl)-6,8-difluor-1,4-dihydro-4-oxo-3-chinolincarbonsäure-Mesylat, Schmelzpunkt: 262-265°C (unter Zersetzung).
   Analog erhält man:
B 1-Cyclopropyl-7-(2,8-diazabicyclo[4.3.0]non-8-yl)-6,8-difluor-1,4-dihydro-4-oxo-3-chinolincarbonsäure-Tosylat, Schmelzpunkt: 248-250°C (unter Zersetzung).
C. 1-Cyclopropyl-7-(2,8-diazabicyclo[4.3.0]non-8-yl)-6,8-difluor-1,4-dihydro-4-oxo-3-chinolincarbonsäure-Lactat, Schmelzpunkt: 205°C-215°C nach vorhergehendem Sintern.

### Beispiel 35

3,9 g (10 mmol) 1-Cyclopropyl-7-(2,8-diazabicyclo-[4.3.0]non-8-yl)-6,8-difluor-1,4-dihydro-4-oxo-3-chinolincar- bonsäure werden in 50 ml Wasser suspendiert und bei Raumtemperatur mit 10 ml 1 n-Natronlauge versetzt, wobei sich das Produkt weitgehend auflöst. Von einer schwachen Trübung wird durch Filtration über ein Membranfilter abgetrennt, das Filtrat im Hochvakuum eingeengt und der Rückstand mit Ethanol verrührt, abgesaugt und getrocknet. Ausbeute: 3,4 g (82,7 % der Theorie) 1-Cyclopropyl-7-(2,8-diazabicyclo[4.3.0]non-8-yl)-6,8-difluor-1,4-dihydro-4-oxo-3-chinolincarbonsäure-Natriumsalz; das Salz zersetzt sich langsam oberhalb 210°C ohne zu schmelzen.

### Beispiel 36

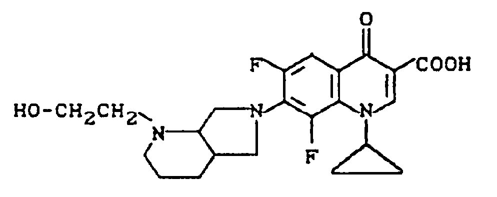

Eine Mischung von 3,9 g (10 mmol) 1-Cyclopropyl-7-(2,8-diazabicyclo[4.3.0]non-8-yl)-6,8-difluor-1,4-dihydro-4-oxo-3-chinolincarbonsäure in 100 ml Dimethylformamid mit 4,2 g Triethylamin und 2,8 g 2-Bromethanol wird 20 Stunden auf 80-100°C erhitzt. Danach wird die Lösung im Vakuum eingeengt und der erhaltene Rückstand an 200 g Kieselgel chromatographisch gereinigt (Laufmittel: CH₂Cl₂/ CH₃OH/17 % -NH₃ = 30:8: 1). Das Eluat wird eingeengt, mit Ethanol verrührt, abgesaugt und getrocknet.
Ausbeute: 1,8 g (41,6 % der Theorie) 1-Cyclopropyl-6,8-difluor-1,4-dihydro-7-[2-(2-hydroxyethyl)-2,8-diazabicyclo [4.3.0]non-8-yl]-4-oxo-3-chinolincarbonsäure,
Schmelzpunkt: 200-206°C (unter Zersetzung).
Massenspektrum: ^{m}/e 433 (M⁺), 402 (M⁺ -CH₂OH), 140, 110 (100 %), 96

### Beispiel 37

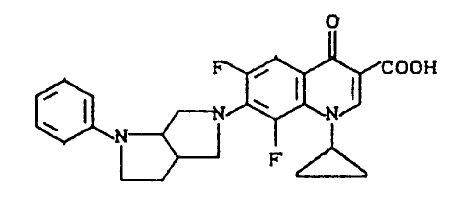

Analog Beispiel 6 wird mit 2-Phenyl-2,7-diazabicyclo-[3.3.0]octan zu 1-Cyclopropyl-6,8-difluor-1,4-dihydro-4-oxo-7-(2-phenyl-2,7-diazabicyclo[3.3.0]oct-7-yl)-3-chinolincarbonsäure umgesetzt, Schmelzpunkt: 259-260°C (unter Zersetzung).

### Beispiel 38

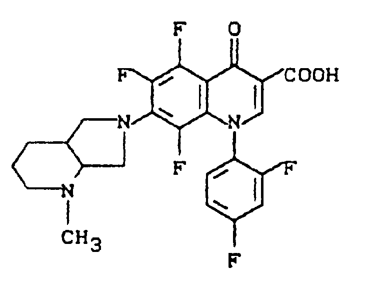

Analog Beispiel 6 erhält man mit 5,6,7,8-Tetrafluor-1-(2,4-difluorphenyl)-1,4-dihydro-4-oxo-3-chinolincarbonsäure 5,6,8-Trifluor-1-(2,4-difluorphenyl)-1,4-dihydro-7-(2-methyl-2,8-diazabicyclo[4.3.0]non-8-yl)-4-oxo-3-chinolincarbon- säure.

### Beispiel 39

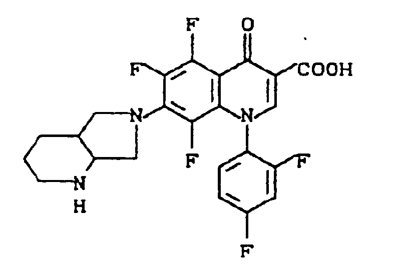

Analog Beispiel 17 erhält man mit 5,6,7,8-Tetrafluor-1-(2,4-difluorphenyl)-1,4-dihydro-4-oxo-3-chinolincarbonsäu- re 7-(2,8-Diazabicyclo[4.3.0]non-8-yl)-5,6,8-trifluor-1-(2,4-difluorphenyl)-1,4-dihydro-4-oxo-3-chinolincarbonsäure.

### Beispiel 40

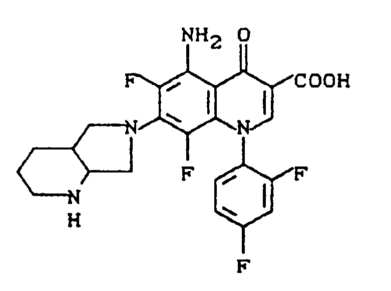

Analog Beispiel 18 erhält man mit7-(2,8-Diazabicyclo-[4.3.0]non-8-yl)-5,6,8-trifluor-1-(2,4-difluorphenyl)-1,4-dihy- dro-4-oxo-3-chinolincarbonsäure 5-Amino-7-(2,8-diazabicyclo[4.3.0]non-8-yl)-6,8-difluor-1-(2,4-difluorphenyl)-1,4-di- hydro-4-oxo-3-chinolincarbonsäure.

### Beispiel 41

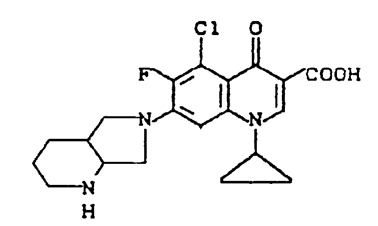

Analog Beispiel 8 A erhält man mit 5,7-Dichlor-1-cyclopropyl-6-fluor-1,4-dihydro-4-oxo-3-chinolincarbonsäure (5 Stunden Rückfluß) 5-Chlor-1-cyclopropyl-7-(2,8-diazabicyclo[4.3.0]non-8-yl)-6-fluor-1,4-dihydro-4-oxo-3-chinolincar- bonsäure, Schmelzpunkt: 270°C (Zersetzung).

### Beispiel 42

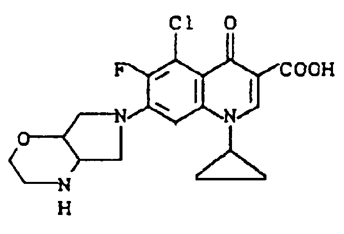

Analog Beispiel 1 erhält man mit 5,7-Dichlor-1-cyclopropyl-6-fluor-1,4-dihydro-4-oxo-3-chinolincarbonsäure (5 Stunden Rückfluß) 5-Chlor-1-cyclopropyl-6-fluor-1,4-dihydro-7-(2-oxa-5,8-diazabicyclo[4.3.0]non-8-yl)-4-oxo-3-chi- nolincarbonsäure.

### Beispiel 43

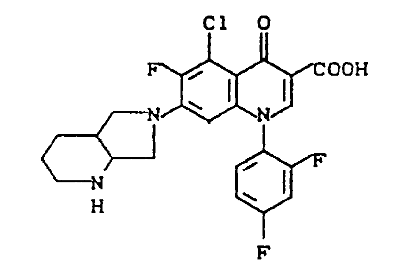

Analog Beispiel 8 A erhält man mit 5,7-Dichlor-6-fluor-1-(2,4-difluorphenyl)-1,4-dihydro-4-oxo-3-chinolincarbon- säure (5 Stunden Rückfluß) 5-Chlor-7-(2,8-diazabicyclo[4.3.0]non-8-yl)-6-fluor-1-(2,4-difluorphenyl)-1,4-dihydro-4-oxo-3-chinolincarbonsäure.

### Beispiel 44

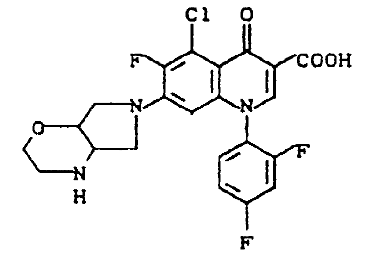

Analog Beispiel 1 erhält man mit 5,7-Dichlor-6-fluor-1-(2,4-difluorphenyl)-1,4-dihydro-4-oxo-3-chinolincarbonsäu- re (5 Stunden Rückfluß) 5-Chlor-6-fluor-1-(2,4-difluorphenyl)-1,4-dihydro-7-(2-oxa-5,8-diazabicyclo [4.3.0]non-8-yl)-4-oxo-3-chinolincarbonsäure.

### Beispiel 45

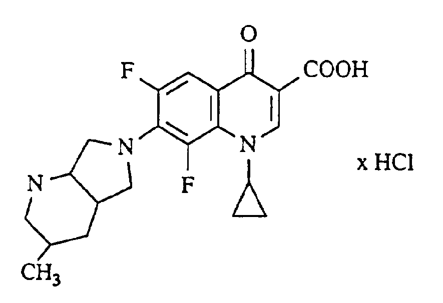

Analog Beispiel 6 und 8 erhält man mit 4-Methyl-2,8-diazabicyclo[4.3.0]-nonan 1-Cyclopropyl-6,B-difluor-1,4-dihy- dro-7-(4-methyl-2,8-diazabicyclo[4.3.0]non-8-yl)-4-oxo-3-chinolincarbonsäure, Schmelzpunkt: 213-215°C (unter Zersetzung) (umkristallisiert aus Glykolmonomethylether) und 1-Cyclopropyl-6,8-difluor-1,4-dihydro-7-(4-methyl-2,8-diazabicyclo[4.3.0]non-8-yl)-4-oxo-3-chinolincarbonsäure-Hydrochlorid, Schmelzpunkt: 217-218°C (unter Zersetzung).

Das Produkt besteht aus einem Gemisch von 2 Stereoisomeren.

## Patentansprüche (Patentansprüche für folgende(n) Vertragsstaat(en) : AT, BE, CH, DE, ES, FR, GB, IT, LI, LU, NL, SE)

1. 7-(1-Pyrrolidinyl)-3-chinolon- und -naphthyridoncarbonsäure-Derivate der Formel (I) 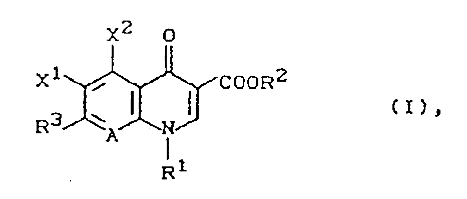 in welcher
X¹ für Halogen,
X² für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen,
R¹ für Alkyl mit 1 bis 4 Kohlenstoffatomen, Alkenyl mit 2 bis 4 Kohlenstoffatomen, Cycloalkyl mit 3 bis 6 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,
R² für Wasserstoff, Alkyl mit 1 bis 4 Kohlenstoffatomen oder (5-Methyl-2-oxo-1,3-dioxol-4-yl)-methyl und
R³ für einen Rest der Struktur 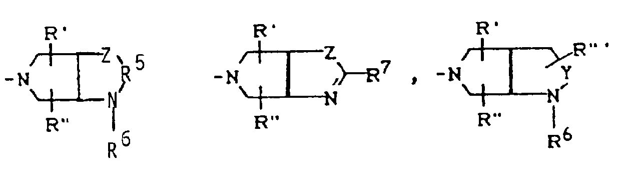 steht, worin
R⁵ eine gegebenenfalls durch Methyl ein- oder zweifach substituierte C₁-C₃-Alkylenbrücke bedeutet,
R⁶ für H, gegebenenfalls durch Hydroxy substituiertes C₁-C₄₋Alkyl, sowie Aryl, Heteroaryl, Benzyl, C₁-C₄-Alkoxycarbonyl, C₁-C₄-Acyl, (5-Methyl-2-oxo-1,3-dioxol-4-yl)-methyl oder C₃-C₆-Cycloalkyl,
R⁷ für H oder C₁-C₄-Alkyl,
R' für H, CH₃ oder Phenyl,
R" für H, CH₃ oder Phenyl,
R"' für H oder CH₃,
Y für O; CH₂, CH₂CH₂ oder CH₂-0 stehen kann, wobei die Verknüpfung der CH₂-0-Gruppe zum Stickstoff sowohl über O als auch über CH₂ erfolgen kann, und
Z für O oder S stehen kann,
A für N oder C-R⁸ steht, worin
R⁸ für H, Halogen, Methyl, Cyano, Nitro, Hydroxy oder Methoxy steht oder auch gemeinsam mit R¹ eine Brücke der Struktur 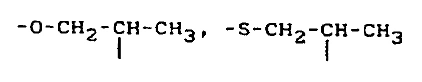 oder 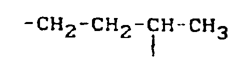 bilden kann,
und deren pharmazeutisch verwendbare Hydrate und Säureadditionssalze sowie die Alkali-, Erdalkali-, Silber- und Guanidiniumsalze der zugrundeliegenden Carbonsäuren.

2. Verbindungen der Formel (I) gemäß Anspruch 1, in welcher
X¹ für Fluor oder Chlor,
X² für Wasserstoff, Amino, Alkylamino mit 1 bis 2 Kohlenstoffatomen, Dimethylamino, Hydroxy, Methoxy, Mercapto, Methylthio, Phenylthio, Fluor, Chlor,
R¹ für Alkyl mit 1 bis 3 Kohlenstoffatomen, Alkenyl mit 2 bis 3 Kohlenstoffatomen, Cycloalkyl mit 3 bis 5 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,
R² für Wasserstoff, Alkyl mit 1 bis 3 Kohlenstoffatomen oder (5-Methyl-2-oxo-1,3-dioxol-4-yl)-methyl,
R³ für einen Rest der Struktur 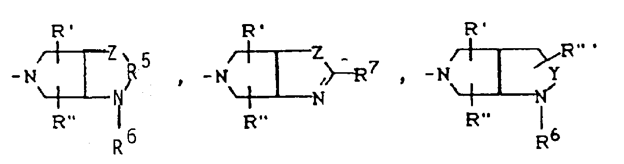 steht, worin
R⁵ eine gegebenenfalls durch Methyl ein - oder zweifach substituierte C₁-C₂-Alkylenbrücke bedeutet,
R⁶ für H, gegebenenfalls durch Hydroxy substituiertes C₁-C₃-Alkyl, sowie Phenyl, Benzyl, C₁-C₄-Alkoxycarbonyl, C₁-C₂-Acyl, (5-Methyl-2-oxo-1,3-dioxol-4-yl)-methyl oder C₃-C₅-Alkyl,
R⁷ für H oder C₁-C₂-Alkyl,
R' für H oder CH₃,
R" für H oder CH₃,
R"' für H oder CH₃,
Y für O; CH₂, CH₂CH₂ oder CH₂-0 stehen kann, wobei die Verknüpfung der CH₂-0-Gruppe zum Stickstoff sowohl über O als auch über CH₂ erfolgen kann, und
Z für O oder S stehen kann,
A für N oder C-R⁸ steht, worin
R⁸ für, Fluor, Chlor Brom, Hydroxy oder Methoxy steht oder auch gemit R¹ eine Brücke der Struktur 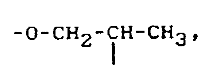 bilden kann.

3. Verbindungen der Formel (I) gemäß Anspruch 1, in welcher
X¹ für Fluor
X² für Wasserstoff, Amino, Methylamino, Fluor
R¹ für Alkyl mit 1 bis 2 Kohlenstoffatomen, Vinyl, Cyclopropyl, 2-Hydroxyethyl, 2-Fluorethyl, Methoxy, Methylamino, 4-Fluorphenyl, 2,4-Difluorphenyl,
R² für Wasserstoff, Alkyl mit 1 bis 2 Kohlenstoffatomen,
R³ für einen Rest der Struktur 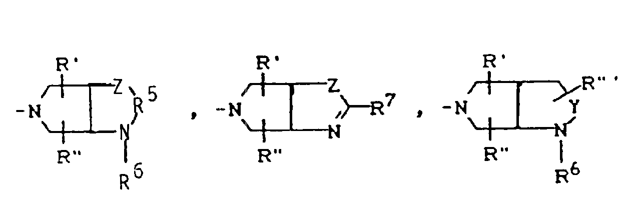 steht, worin
R⁵ eine gegebenenfalls durch Methyl substituierte C₁-C₂-Alkylenbrücke bedeutet,
R⁶ für H, CH₃, C₂H₅, HOCH₂CH₂, Benzyl, C₁-C₄-Alkoxycarbonyl, C₁-C₂-Acyl,
_{R}7 für H oder CH₃,
R' für H oder CH₃,
R" für H oder CH₃,
R"' für H oder CH₃,
Y für O, CH₂, CH₂CH₂ oder CH₂-O stehen kann, wobei die Verknüpfung der CH₂-O-Gruppe zum Stickstoff sowohl über O als auch über CH₂ erfolgen kann, und
Z für O oder S stehen kann,
A für N oder C-R⁸ steht, worin
R⁸ für H, Fluor, Chlor oder Methoxy steht, oder auch gemeinsam mit R¹ eine Brücke der Struktur 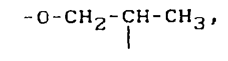 bilden kann.

4. Verfahren zur Herstellung von Verbindungen gemäß Anspruch 1 der Formel (I), 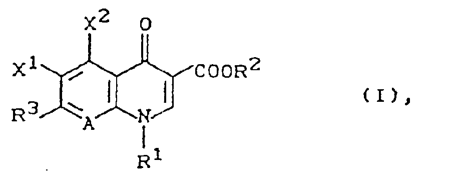 in welcher
X¹ für Halogen,
X² für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Hercapto, Alkylthio mit 1 bis 4 Kohlenstoffatomen, Arylthio, Halogen,
R¹ für Alkyl mit 1 bis 4 Kohlenstoffatomen, Alkenyl mit 2 bis 4 Kohlenstoffatomen, Cycloalkyl mit 3 bis 6 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,
R² für Wasserstoff, Alkyl mit 1 bis 4 Kohlenstoffatomen oder (5-Methyl-2-oxo-1,3-dioxol-4-yl)-methyl und
R³ für einen Rest der Struktur 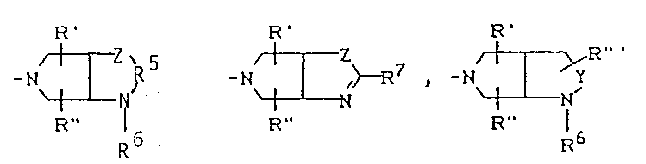 steht, worin
R⁵ eine gegebenenfalls durch Methyl ein - oder zweifach substituierte C₁-C₃-Alkylenbrücke bedeutet,
R⁶ für H, gegebenenfalls durch Hydroxy substituiertes C₁-C₄-Alkyl, sowie Aryl, Heteroaryl, Benzyl, C₁-C₄-Alkoxycarbonyl, C₁-C₄-Acyl, (5-Methyl-2-oxo-1,3-dioxol-4-yl)-methyl oder C3-C6-Cycloalkyl,
R⁷ für H oder C₁-C₄-Alkyl,
R' für H, CH₃ oder Phenyl,
R" für H, CH₃ oder Phenyl,
R"' für H oder CH₃,
Y für O, CH₂, CH₂CH₂ oder CH₂-0 stehen kann, wobei die Verknüpfung der CH₂-0-Gruppe zum Stickstoff sowohl über 0 als auch über CH₂ erfolgen kann, und
Z für O oder S stehen kann,
A für N oder C-R⁸ steht, worin 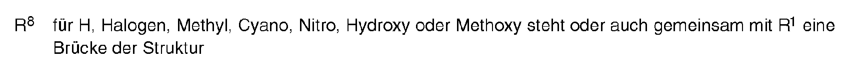 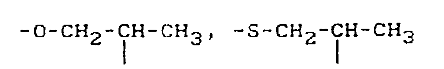
oder 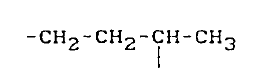 bilden kann,
dadurch gekennzeichnet, daß man Verbindungen der Formel (II) 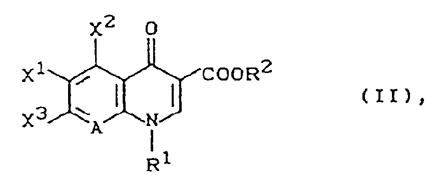 in welcher
A, R¹, R², X¹ und X² die oben angegebene Bedeutung haben und
X³ für Halogen, insbesondere Fluor oder Chlor steht, mit Verbindungen der Formel (III) 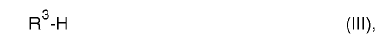 in welcher
R³ die oben angegebene Bedeutung hat,
gegebenenfalls in Gegenwart von Säurefängern umsetzt, und gegebenenfalls in R³ enthaltene Schutzgruppen abspaltet.

5. Verfahren zur Herstellung von Verbindungen gemäß Anspruch 1 der Formel (I) 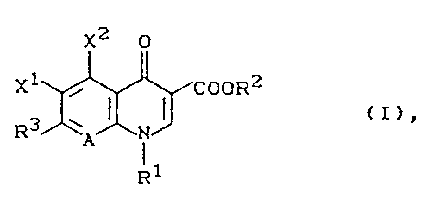 in welcher
X¹ für Halogen,
R¹ für Alkyl mit 1 bis 4 Kohlenstoffatomen, Alkenyl mit 2 bis 4 Kohlenstoffatomen, Cycloalkyl mit 3 bis 6 Kohlenstoffatomen, 2-Hydroxyethyl, 2-Fluorethyl, Hethoxy, Amino, Methylamino, Ethylamino, Dimethylamino, gegebenenfalls durch 1 oder 2 Fluoratome substituiertes Phenyl,
R² für Wasserstoff, Alkyl mit 1 bis 4 Kohlenstoffatomen oder (5-Methyl-2-oxo-1,3-dioxol-4-yl)-methyl und
R³ für einen Rest der Struktur 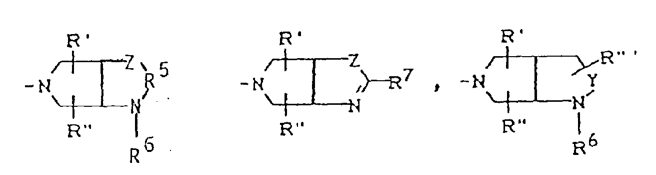 steht, worin
R⁵ eine gegebenenfalls durch Methyl ein - oder zweifach substituierte C₁-C₃-Alkylenbrücke bedeutet,
R⁶ für H, gegebenenfalls durch Hydroxy substituiertes C₁-C₄ Alkyl, sowie Aryl, Heteroaryl, Benzyl, C₁-C₄ Alkoxycarbonyl, C₁-C₄-Acyl, (5-Methyl-2-oxo-1,3-dioxol-4-yl)-methyl oder C₃-C₆-Cycloalkyl,
R⁷ für H oder C₁-C₄-Alkyl,
R' für H, CH₃ oder Phenyl,
R" für H, CH₃ oder Phenyl,
R"' für H oder CH₃,
Y für O; CH₂, CH₂CH₂ oder CH₂-O stehen kann, wobei die Verknüpfung der CH₂-O-Gruppe zum Stickstof sowohl über O als auch über CH₂ erfolgen kann, und
Z für O oder S stehen kann,
A für N oder C-R⁸ steht, worin
R⁸ für H, Halogen, Methyl, Cyano, Nitro, Hydroxy oder Methoxy steht oder auch gemeinsam mit R¹ eine Brücke der Struktur 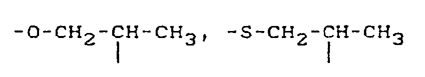 oder 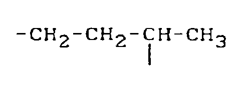 bilden kann,
X² für Amino, Alkylamino mit 1 bis 4 Kohlenstoffastomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen oder Arylthio steht,
dadurch gekennzeichnet, daß man eine Verbindung der Formel (IV) 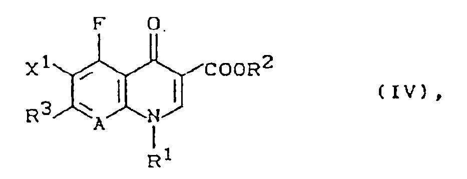 in welcher
X¹, R¹, R², R³ und A die oben angegebene Bedeutung haben,
mit Verbindungen der Formel (V) 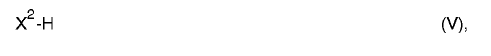 in welcher
X² die oben angegebene Bedeutung hat, gegebenenfalls in Gegenwart von Säurefängern umsetzt.

6. Verfahren zur Herstellung von Verbindungen gemäß Anspruch 1 der Formel (la) 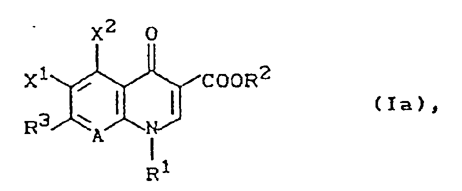 in welcher
X¹ für Halogen,
X² für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Hercapto, Alkylthio mit 1 bis 4 Kohlenstoffatomen, Arylthio, Halogen,
R¹ für Alkyl mit 1 bis 4 Kohlenstoffatomen, Alkenyl mit 2 bis 4 Kohlenstoffatomen, Cycloalkyl mit 3 bis 6 Kohlenstoffatomen, 2-Hydroxyethyl, 2-FluoreLhyl, Methoxy, Amino, Methylamino, Ethylamino, Dimethylamino, gegebenenfalls durch 1 oder 2 Fluoratome substituiertes Phenyl,
R² für Wasserstoff, Alkyl mit 1 bis 4 Kohlenstoffatomen oder (5-Methyl-2-oxo-1,3-dioxol-4-yl)-methyl,
A für N oder C-R⁸ steht, worin
R⁸ für H, Halogen, methyl, Cyano, Nitro, Hydroxy oder Methoxy steht oder auch gemeinsam mit R¹ eine Brücke der Struktur 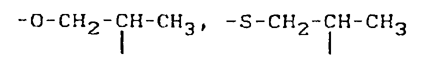 oder 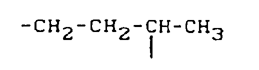 bilden kann
und R³ für einen Rest der Struktur 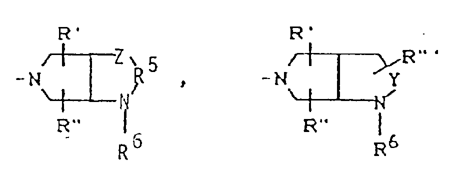 steht, worin
R⁵, R⁶, R', R", R"', Y und Z die oben angegebene Bedeutung haben, dadurch gekennzeichnet, daß man eine Verbindung der Formel (VI) 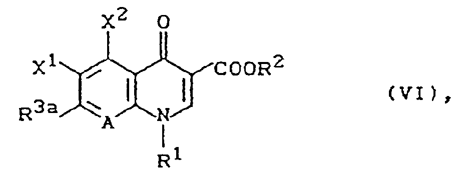 in welcher
X¹, X², R¹, R² und A die oben angegebene Bedeutung haben und
R3a für einen Rest der Struktur 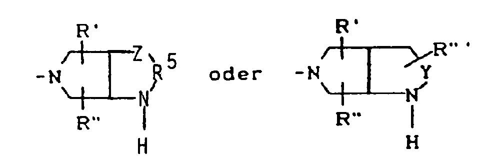 steht,
worin R⁵, R', R", R"', Y und Z die oben angegebene Bedeutung haben,
mit Verbindungen der Formel (VII) 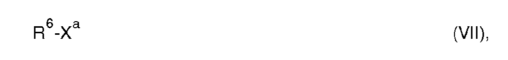 in welcher
R⁶ die oben angegebene Bedeutung hat und
X^{a} für Chlor, Brom, lod, Hydroxy oder Acyloxy steht,
gegebenenfalls in Gegenwart von Säurefängern umsetzt.

7. 7-(1-Pyrrolidinyl)-3-chinolan- und -naphthyridoncarbonsäure-Derivate der Formel (I) gemäß Anspruch 1 zur Anwendung in einem Verfahren zur Bekämpfung von Krankheiten.

8. Arzneimittel enthaltend Verbindungen der Formel (I) gemäß Anspruch 1.

9. Verwendung von Verbindungen der Formel (I) gemäß Anspruch 1 zur Herstellung von Arzneimitteln.

10. Verwendung von Verbindungen der Formel (I) gemäß Anspruch 1 als Tierfutterzusatzmittel.

11. Tierfutter bzw. Tierfutterzusatzmittel und Prämixe enthaltend Verbindungen der Formel (I) nach Anspruch 1.

12. Verbindungen aus der Gruppe bestehend aus
2-Oxa-5,8-diazabicyclo[4.3.0]nonan-dihydrochlorid, trans-2-Oxa-5,8-diazabicyclo[4.3.0]nonan,
5-Methyl-2-oxa-5,8-diazabicyclo[4.3.0]nonan-dihydrochlorid,
2,8-Diazabicyclo[4.3.0]nonan,
4-Methyl-2,8-diazabicyclo[4.3.0]nonan,
2-Methyl-2,8-diazabicyclo[4.3.0]nonan,
2,7-Diazabicyclo[3.3.0]octan,
2-Methyl-2,7-diazabicyclo[3.3.0]octan,
3-Oxa-2,7-diazabicyclo[3.3.0]octan,
2-Methyl-3-oxa-2,7-diazabicyclo[3.3.0]octan,
2,5-Dimethyl-3-oxa-2,7-diazabicyclo[3.3.0]octan,
2,8-Dimethyl-3-oxa-2,7-diazabicyclo[3.3.0]octan,
2-Methyl-4-oxa-2,8-diazabicyclo[4.3.0]nonan,
3-Methyl-2,7-diazabicyclo[3.3.0]octan,
2,3-Dimethyl-2,7-diazabicyclo[3.3.0]octan,
2,7-Diazabicyclo[3.3.0]octan-2-carbonsäureethylester,
2-Phenyl-2,7-diazabicyclo[3.3.0]octan,
4-Oxa-2,8-diazabicyclo[4.3.0]nonan,

13. Verbindungen aus der Gruppe bestehend aus
5,6,7,8-Tetrafluor-1-(2,4-difluorphenyl)-1,4-dihydro-4-oxo-3-chinolincarbonsäure,
5,7-Dichlor-1-cyclopropyl-6-fluor-1,4-dihydro-4-oxo-3-chinolincarbonsäure und
5,7-Dichlor-6-fluor-1-(2,4-difluorphenyl)-1,4-dihydro-4-oxo-3-chinolincarbonsäure,

14. Carbonsäurederivate der Formel (I) gemäß Anspruch 1, worin
X¹ für Fluor,
X² für Wasserstoff,
R¹ für Cyclopropyl,
_{R}² für Wasserstoff,
R³ für einen Rest mit der Formel 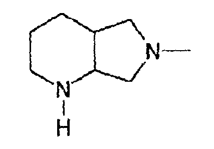 und
A für C-Cl, C-F oder C-OCH₃ stehen,
und deren pharmazeutisch verträgliche Salze.

15. Carbonsäurederivate gemäß Anspruch 14 zur Verwendung als antibakterielle Mittel.

16. Verwendung von Verbindungen gemäß Anspruch 14 zur Herstellung von Medikamenten gegen bakterielle Erkrankungen.

17. Verwendung von Verbindungen gemäß Anspruch 14 in Tierfutter.

## Patentansprüche (Patentansprüche für folgende(n) Vertragsstaat(en) : ES)

1. Verfahren zur Herstellung von 7-(1-Pyrrolidinyl)-3-chinolon- und -naphthyridoncarbonsäure-Derivaten der Formel (I) 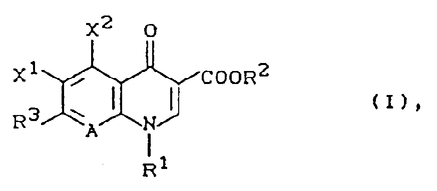 in welcher
X¹ für Halogen,
X² für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen,
R¹ für Alkyl mit 1 bis 4 Kohlenstoffatomen, Alkenyl mit 2 bis 4 Kohlenstoffatomen, Cycloalkyl mit 3 bis 6 Kohlenstoffatomen, 2-Hydroxyethyl, 2-Fluorethyl, Hethoxy, Amino, Hethylamino, Ethylamino, Dimethylamino, gegebenenfalls durch 1 oder 2 Fluoratome substituiertes Phenyl,
R² für Wasserstoff, Alkyl mit 1 bis 4 Kohlenstoffatomen oder (5-Methyl-2-oxo-1,3-dioxol-4-yl)-methyl und
R³ für einen Rest der Struktur 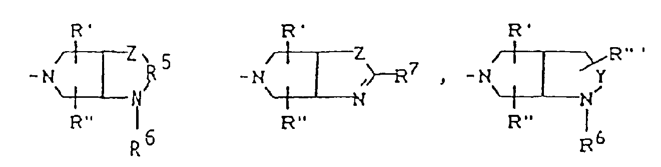 steht, worin
R⁵ eine gegebenenfalls durch Methyl ein - oder zweifach substituierte C₁-C₃-Alkylenbrücke bedeutet,
R⁶ für H, gegebenenfalls durch Hydroxy substituiertes C₁-C₄ Alkyl, sowie Aryl, Heteroaryl, Benzyl, C₁-C₄-Alkoxycarbonyl, C₁-C₄-Acyl, (5-Methyl-2-oxo-1,3-dioxol-4-yl)-methyl oder C₃-Cₛ-Cycloalkyl,
R⁷ für H oder C₁-C₄-Alkyl,
R' für H, CH₃ oder Phenyl,
R" für H, CH₃ oder Phenyl,
R"' für H oder CH₃,
Y für O; CH₂, CH₂CH₂ oder CH₂-0 stehen kann, wobei die Verknüpfung der CH₂-0-Gruppe zum Stickstoff sowohl über O als auch über CH₂ erfolgen kann, und
Z für O oder S stehen kann,
A für N oder C-R⁸ steht, worin
R⁸ für H, Halogen, Methyl, Cyano, Nitro, Hydroxy oder Methoxy steht oder auch gemeinsam mit R¹ eine Brücke der Struktur 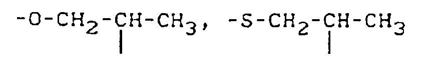 oder 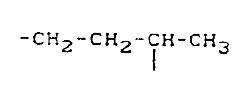 bilden kann,
und deren pharmazeutisch verwendbare Hydrate und Säureadditionssalze sowie die Alkali-, Erdalkali-, Silber- und Guanidiniumsalze der zugrundeliegenden Carbonsäuren,
umfassend die Umsetzung einer Verbindung der Formel (II) 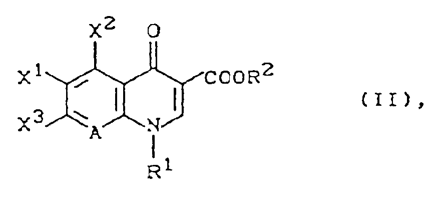 in welcher
A, R¹, R², X¹ und X² die oben angegebene Bedeutung haben und
X³ für Halogen, insbesondere Fluor oder Chlor steht, mit Verbindungen der Formel (III) 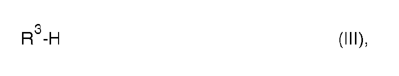 in welcher
R³ die oben angegebene Bedeutung hat,
gegebenenfalls in Gegenwart von Säurefängern und gegebenenfalls unter Abspaltung von in R³ enthaltenen Schutzgruppen.

2. Verfahren zur Herstellung von Verbindungen mit der Formel (I) gemäß Anspruch 1 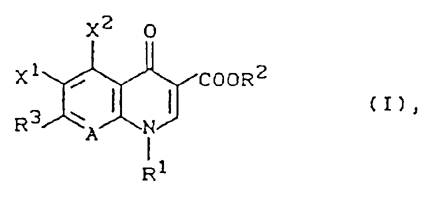 in welcher
X¹, R¹, R², R³ und A die in Anspruch 1 angegebenen Bedutungen haben und
X² für Amino, Alkylamino mit 1 bis 4 Kohlenstoffastomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 KohlensLoffatomen oder Arylthio steht,
dadurch gekennzeichnet, daß man eine Verbindung der Formel (IV) 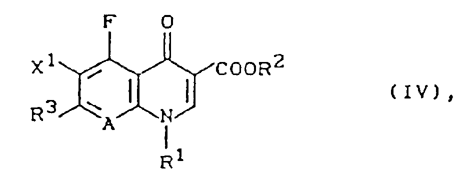 in welcher
X¹, R¹, R², R³ und A die oben angegebene Bedeutung haben,
mit Verbindungen der Formel (V) 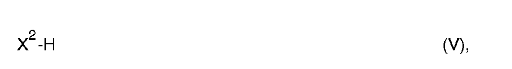 in welcher
X² die oben angegebene Bedeutung hat, gegebenenfalls in Gegenwart von Säurefängern umsetzt.

3. Verfahren zur Herstellung von Verbindungen mit der Formel (la) 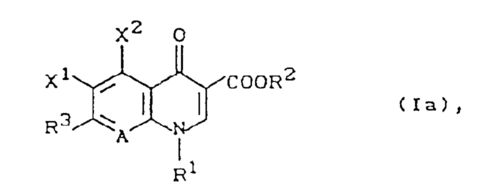 in welcher
X¹, X², R⁷, R² und A die in Anspruch 1 angegebenen Bedeutungen haben und R³ für einen Rest der Struktur 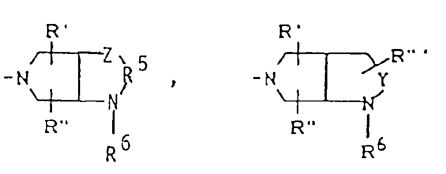 steht, worin
R⁵, R⁶, R', R", R_{"'}, Y und Z die in Anspruch 1 angegebenen Bedutungen haben,
dadurch gekennzeichnet, daß man eine Verbindung der Formel (VI) 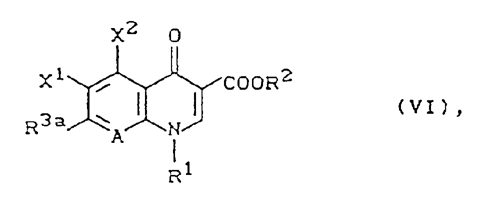 in welcher
X¹, X², R¹, R² und A die oben angegebene Bedeutung haben und
_{R}3a für einen Rest der Struktur 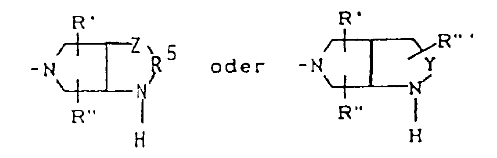 steht,
worin R⁵, R', R", R"', Y und Z die oben angegebene Bedeutung haben,
mit Verbindungen der Formel (VII) 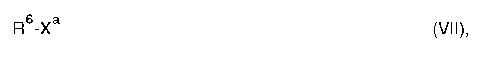 in welcher
R⁶ die oben angegebene Bedeutung hat und
X^{a} für Chlor, Brom, lod, Hydroxy oder Acyloxy steht,
gegebenenfalls in Gegenwart von Säurefängern umsetzt.

4. Verfahren gemäß einem der Ansprüche 1 bis 3, in welchem in Formel (I) oder (la)
X¹ für Fluor oder Chlor,
X² für Wasserstoff, Amino, Alkylamino mit 1 bis 2 Kohlenstoffatomen, Dimethylamino, Hydroxy, Methoxy, Mercapto, Methylthio, Phenylthio, Fluor, Chlor,
R¹ für Alkyl mit 1 bis 3 Kohlenstoffatomen, Alkenyl mit 2 bis 3 Kohlenstoffatomen, Cycloalkyl mit 3 bis 5 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,
R² für Wasserstoff, Alkyl mit 1 bis 3 Kohlenstoffatomen oder (5-Methyl-2-oxo-1,3-dioxol-4-yl)-methyl,
R³ für einen Rest der Struktur 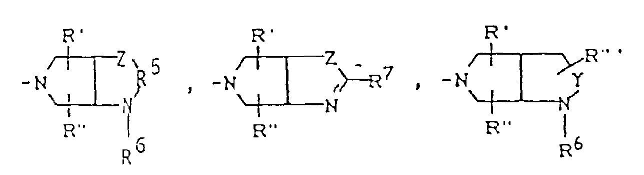 steht, worin
R⁵ eine gegebenenfalls durch Methyl ein - oder zweifach substituierte C₁-C₂-Alkylenbrücke bedeutet,
R⁶ für H, gegebenenfalls durch Hydroxy substituiertes C₁-C₃-Alkyl, sowie Phenyl, Benzyl C₁-C₄-Alkoxycarbonyl, C₁-C₂-Acyl, (5-Methyl-2-oxo-1,3-dioxol-4-yl)-methyl oder C₃-C₅-Alkyl,
R⁷ für H oder C₁-C₂-Alkyl,
R' für H oder CH₃,
R" für H oder CH₃,
R"' für H oder CH₃,
Y für O, CH₂, CH₂CH₂ oder CH₂-0 stehen kann, wobei die Verknüpfung der CH₂-0-Gruppe zum Stickstoff sowohl über 0 als auch über CH₂ erfolgen kann, und
Z für 0 oder S stehen kann,
A für N oder C-R⁸ steht, worin
R⁸ für H, Fluor, Chlor, Brom, Hydroxy oder Methoxy steht oder auch gemit R¹ eine Brücke der Struktur 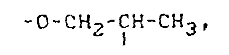 bilden kann.

5. Verfahren gemäß einem der Ansprüche 1 bis 3, in welchem in Formel (I) oder (la)
X¹ für Fluor
X² für Wasserstoff, Amino, Hethylamino, Fluor
R¹ für Alkyl mit 1 bis 2 Kohlenstoffatomen, Vinyl, Cyclopropyl, 2-Hydroxyethyl, 2-Fluorethyl, Methoxy, Methylamino, 4-Fluorphenyl, 2,4-Difluorphenyl,
R² für Wasserstoff, Alkyl mit 1 bis 2 Kohlenstoffatomen,
R³ für einen Rest der Struktur 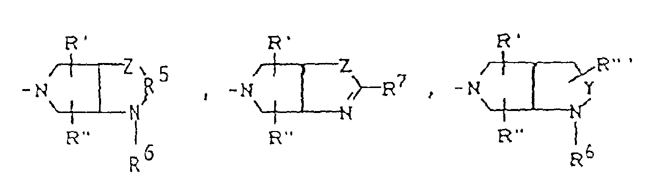 steht, worin
R⁵ eine gegebenenfalls durch Methyl substituierte C₁-C₂-Alkylenbrücke bedeutet,
R⁶ für H, CH₃, C₂H₅, HOCH₂CH₂, Benzyl, C₁-C₄-Alkoxycarbonyl, C₁-C₂-Acyl,
_{R}7 für H oder CH₃,
R' für H oder CH₃,
R" für H oder CH₃,
R"' für H oder CH₃,
Y für O, CH₂, CH₂CH₂ oder CH₂-0 stehen kann, wobei die Verknüpfung der CH₂-0-Gruppe zum Stickstoff sowohl über O als auch über CH₂ erfolgen kann, und
Z für O oder S stehen kann,
A für N oder C-R⁸ steht, worin
R⁸ für H, Fluor, Chlor oder Methoxy steht, oder auch gemeinsam mit R¹ eine Brücke der Struktur 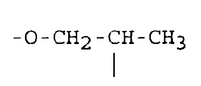 bilden kann.

6. Verfahren gemäß Anspruch 1, in dem eine der folgenden Verbindungen mit der Formel (III) umgesetzt wird:
2-Oxa-5,8-diazabicyclo[4.3.0]nonan-dihydrochlorid, trans-2-Oxa-5,8-diazabicyclo[4.3.0]nonan,
5-Methyl-2-oxa-5,8-diazabicyclo[4.3.0]nonan-dihydrochlorid,
2,8-Diazabicyclo[4.3.0]nonan,
4-Methyl-2,8-diazabicyclo[4.3.0]nonan,
2-Methyl-2,8-diazabicyclo[4.3.0]nonan,
2,7-Diazabicyclo[3.3.0]octan,
2-Methyl-2,7-diazabicyclo[3.3.0]octan,
3-Oxa-2,7-diazabicyclo[3.3.0]octan,
2-Methyl-3-oxa-2,7-diazabicyclo[3.3.0]octan,
2,5-Dimethyl-3-oxa-2,7-diazabicyclo[3.3.0]octan,
2,8-Dimethyl-3-oxa-2,7-diazabicyclo(3.3.0]octan,
2-Methyl-4-oxa-2,8-diazabicyclo[4.3.0]nonan,
3-Methyl-2,7-diazabicyclo[3.3.0]octan,
2,3-Dimethyl-2,7-diazabicyclo[3.3.0]octan,
2,7-Diazabicyclo[3.3.0]octan-2-carbonsäureethylester,
2-Phenyl-2,7-diazabicyclo[3.3.0]octan,
4-Oxa-2,8-diazabicyclo[4.3.0]nonan.

7. Verfahren gemäß Anspruch 1, in dem eine der folgenden Verbindungen mit der Formel (II) umgesetzt wird:
5,6,7,8-Tetrafluor-1-(2,4-difluorphenyl)-1,4-dihydro-4-oxo-3-chinolincarbonsäure,
5,7-Dichlor-1-cyclopropyl-6-fluor-1,4-dihydro-4-oxo-3-chinolincarbonsäure und
5,7-Dichlor-6-fluor-1-(2,4-difluorphenyl)-1,4-dihydro-4-oxo-3-chinolincarbonsäure.

8. Verfahren gemäß Anspruch 1 zur Herstellung von Carbonsäurederivaten der Formel (I), worin
X¹ für Fluor,
X² für Wasserstoff,
R¹ für Cyclopropyl,
R² für Wasserstoff,
R³ für einen Rest mit der Formel 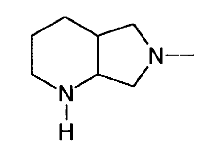 und
A für C-CI, C-F oder C-OCH₃ stehen.

9. 7-(1-Pyrrolidinyl)-3-chinalon- und -naphthyridoncarbonsäure-Derivate der Formel (I) 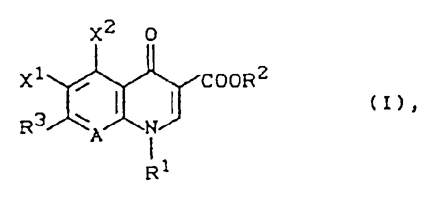 in welcher
X¹ für Halogen,
X² für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen,
R¹ für Alkyl mit 1 bis 4 Kohlenstoffatomen, Alkenyl mit 2 bis 4 Kohlenstoffatomen, Cycloalkyl mit 3 bis 6 Kohlenstoffatomen 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,
R² für Wasserstoff, Alkyl mit 1 bis 4 Kohlenstoffatomen oder (5-Methyl-2-oxo-1,3-dioxol-4-yl)-methyl und
R³ für einen Rest der Struktur 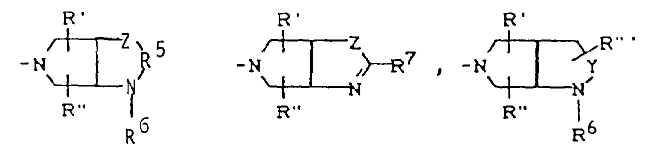 steht, worin
R⁵ eine gegebenenfalls durch Methyl ein - oder zweifach substituierte C₁-C₃-Alkylenbrücke bedeutet,
R⁶ für H, gegebenenfalls durch Hydroxy substituiertes C₁-C₄-Alkyl, sowie Aryl, Hetero aryl, Benzyl, C₁-C₄-Alkoxycarbonyl, C₁-C₄-Acyl, (5-Methyl-2-oxo-1,3-dioxol-4-yl)-methyl oder C₃-C₆-Cycloalkyl,
R⁷ für H oder C₁-C₄-Alkyl,
R' für H, CH₃ oder Phenyl,
R" für H, CH₃ oder Phenyl,
R"' für H oder CH₃,
Y für O, CH₂, CH₂CH₂ oder CH₂-0 stehen kann, wobei die Verknüpfung der CH₂-0-Gruppe zum Stickstoff sowohl über O als auch über CH₂ erfolgen kann, und
Z für O oder S stehen kann,
A für N oder C-R⁸ steht, worin
R⁸ für H, Halogen, Methyl, Cyano, Nitro, Hydroxy oder Methoxy steht oder auch gemeinsam mit R¹ eine Brücke der Struktur 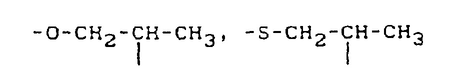 oder 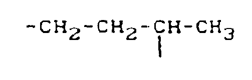 bilden kann,
und deren pharmazeutisch verwendbare Hydrate und Säureadditionssalze sowie die Alkali-, Erdalkali-, Silber- und Guanidiniumsalze der zugrundeliegenden Carbonsäuren.

10. Verbindungen der Formel (I) gemäß Anspruch 9, in welcher
X¹ für Fluor oder Chlor,
X² für Wasserstoff, Amino, Alkylamino mit 1 bis 2 Kohlenstoffatomen, Dimethylamino, Hydroxy, Methoxy, Mercapto, Methylthio, Phenylthio, Fluor, Chlor,
R¹ für Alkyl mit 1 bis 3 Kohlenstoffatomen, Alkenyl mit 2 bis 3 Kohlenstoffatomen, Cycloalkyl mit 3 bis 5 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,
R² für Wasserstoff, Alkyl mit 1 bis 3 Kohlenstoffatomen oder (5-Methyl-2-oxo-1,3-dioxol-4-yl)-methyl,
R³ für einen Rest der Struktur 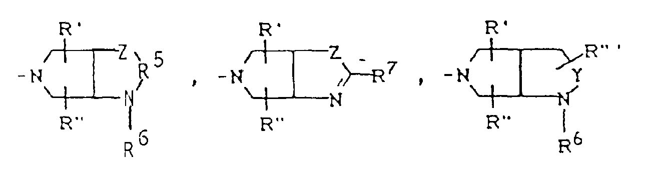 steht, worin
R⁵ eine gegebenenfalls durch Methyl ein - oder zweifach substituierte C₁-C₂-Alkylenbrücke bedeutet,
R⁶ für H, gegebenenfalls durch Hydroxy substituiertes C₁-C₃-Alkyl, sowie Phenyl, Benzyl C₁-C₄-Alkoxycarbonyl, C₁-C₂-Acyl, (5-Methyl-2-oxo-1,3-dioxol-4-yl)-methyl oder C₃-C_{S}-Alkyl,
R⁷ für H oder C₁-C₂-Alkyl,
R' für H oder CH₃,
R" für H oder CH₃,
R"' für H oder CH₃,
Y für O, CH₂, CH₂CH₂ oder CH₂-0 stehen kann, wobei die Verknüpfung der CH₂-0-Gruppe zum Stickstoff sowohl über O als auch über CH₂ erfolgen kann, und
Z für O oder S stehen kann,
A für N oder C-R⁸ steht, worin
R⁸ für H, Fluor, Chlor, Brom, Hydroxy oder Methoxy steht oder auch gemit R¹ eine Brücke der Struktur 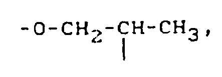 bilden kann.

11. Verbindungen der Formel (I) gemäß Anspruch 9, in welcher
X¹ für Fluor
X² für Wasserstoff, Amino, Methylamino, Fluor
R¹ für Alkyl mit 1 bis 2 Kohlenstoffatomen, Vinyl, Cyclopropyl, 2-Hydroxyethyl, 2-Fluorethyl, Methoxy, Methylamino, 4-Fluorphenyl, 2,4-Difluorphenyl,
R² für Wasserstoff, Alkyl mit 1 bis 2 Kohlenstoffatomen,
R³ für einen Rest der Struktur 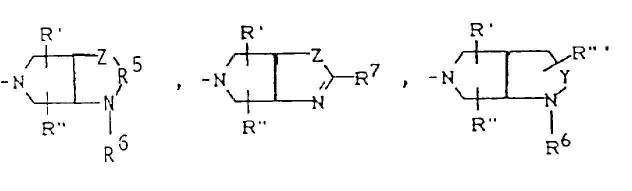 steht, worin
R⁵ eine gegebenenfalls durch Methyl substituierte C₁-C₂-Alkylenbrücke bedeutet,
R⁶ für H, CH₃, C₂H₅, HOCH₂CH₂, Benzyl, C₁-C₄-Alkoxycarbonyl, C₁-C₂-Acyl,
_{R}7 für H oder CH₃,
R' für H oder CH₃,
R" für H oder CH₃,
R"' für H oder CH₃,
Y für O, CH₂, CH₂CH₂ oder CH₂-O stehen kann, wobei die Verknüpfung der CH₂-O-Gruppe zum Stickstoff sowohl über O als auch über CH₂ erfolgen kann, und
Z für O oder S stehen kann,
A für N oder C-R⁸ steht, worin
R⁸ für H, Fluor, Chlor oder Methoxy steht, oder auch gemeinsam mit R¹ eine Brücke der Struktur 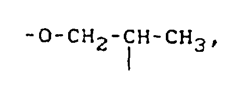 bilden kann.

12. Verwendung von Verbindungen der Formel (I) gemäß Anspruch 9 zur Herstellung von Arzneimitteln.

13. Verwendung von Verbindungen der Formel (I) gemäß Anspruch 9 als Tierfutterzusatzmittel.

14. Tierfutter bzw. Tierfutterzusatzmittel und Prämixe enthaltend Verbindungen der Formel (I) nach Anspruch 9.

15. Verbindungen aus der Gruppe bestehend aus
2-Oxa-5,8-diazabicyclo[4.3.0]nonan-dihydrochlorid, trans-2-Oxa-5,8-diazabicyclo[4.3.0]nonan,
5-Methyl-2-oxa-5,8-diazabicyclo[4.3.0]nonan-dihydrochlorid,
2,8-Diazabicyclo[4.3.0]nonan,
4-Methyl-2,8-diazabicyclo[4.3.0]nonan,
2-Methyl-2,8-diazabicyclo[4.3.0]nonan,
2,7-Diazabicyclo[3.3.0]octan,
2-Methyl-2,7-diazabicyclo[3.3.0]octan,
3-Oxa-2,7-diazabicyclo[3.3.0]octan,
2-Methyl-3-oxa-2,7-diazabicyclo[3.3.0]octan,
2,5-Dimethyl-3-oxa-2,7-diazabicyclo[3.3.0]octan,
2,8-Dimethyl-3-oxa-2,7-diazabicyclo[3.3.0]octan,
2-Methyl-4-oxa-2,8-diazabicyclo[4.3.0]nonan,
3-Methyl-2,7-diazabicyclo[3.3.0]octan,
2,3-Dimethyl-2,7-diazabicyclo[3.3.0]octan,
2,7-Diazabicyclo[3.3.0]octan-2-carbonsäureethylester
2-Phenyl-2,7-diazabicyclo[3.3.0]octan,
4-Oxa-2,8-diazabicyclo[1.3.0]nonan.

16. Verbindungen aus der Gruppe bestehend aus
5,6,7,8-Tetrafluor-1-(2,4-difluorphenyl)-1,4-dihydro-4-oxo-3-chinolincarbonsäure,
5,7-Dichlor-1-cyclopropyl-6-fluor-1,4-dihydro-4-oxo-3-chinolincarbonsäure und
5,7-Dichlor-6-fluor-1 -(2,4-difluorphenyl)-1,4-dihydro-4-oxo-3-chinolincarbonsäure.

17. Carbonsäurederivate der Formel (I) gemäß Anspruch 1, worin
X¹ für Fluor,
X² für Wasserstoff,
R¹ für Cyclopropyl,
_{R}² für Wasserstoff,
R³ für einen Rest mit der Formel 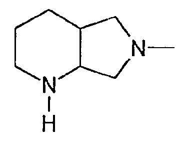 und
A für C-CI, C-F oder C-OCH₃ stehen,
und deren pharmazeutisch verträgliche Salze.

18. Carbonsäurederivate gemäß Anspruch 17 zur Verwendung als antibakterielle Mittel.

19. Verwendung von Verbindungen gemäß Anspruch 17 zur Herstellung von Medikamenten gegen bakterielle Erkrankungen.

20. Verwendung von Verbindungen gemäß Anspruch 17 in Tierfutter.

## Claims (Claims for the following Contracting State(s) : AT, BE, CH, DE, ES, FR, GB, IT, LI, LU, NL, SE)

1. 7-(1-Pyrrolidinyl)-3-quinolone- and -naphthyridonecarboxylic acid derivatives of the formula (I) 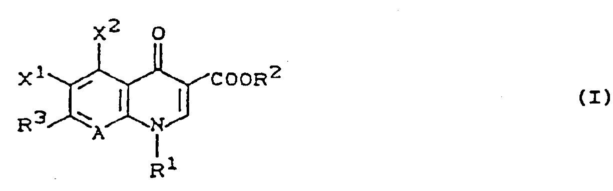 in which
X¹ represents halogen,
X² represents hydrogen, amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms, arylthio or halogen,
R¹ represents alkyl having 1 to 4 carbon atoms, alkenyl having 2 to 4 carbon atoms, cycloalkyl having 3 to 6 carbon atoms, 2-hydroxyethyl, 2-fluoroethyl, methoxy amino, methylamino, ethylamino, dimethylamino or phenyl which is optionally substituted by 1 or 2 fluorine atoms,
R² represents hydrogen, alkyl having 1 to 4 carbon atoms or (5-methyl-2-Oxo-1,3 -dioxol-4-yl)-methyl,
R³ represents a radical of the structure 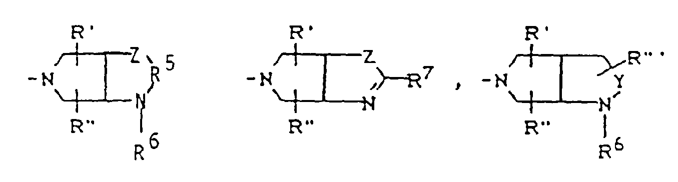 wherein
R⁵ denotes a C₁-C₃-alkylene bridge which is optionally mono- or disubstituted by methyl,
R⁶ can represent H, optionally hydroxyl-substituted C₁-C₄-alkyl, and aryl, heteroaryl, benzyl, C1-C4-alkoxycarbonyl, C₁-C₄-acyl, (5-methyl-2-oxo-1,3-dioxol-4-yl)-methyl, or C₃-C₆-cycloalkyl,
R⁷ can represent H or C₁-C₄-alkyl,
R' can represent H, CH₃ or phenyl,
R" can represent H, CH₃ or phenyl,
R"' can represent H or CH₃,
Y can represent O, CH₂, CH₂CH₂ or CH₂-0, it being possible for the CH₂-0 group to be linked to the nitrogen either via O or via CH₂, and
Z can represent O or S, and
A represents N or C-R⁸, wherein
R⁸ represents H, halogen, methyl, cyano, nitro, hydroxyl or methoxy or, together with R¹, also can form a bridge of the structure 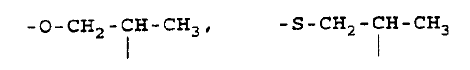 or 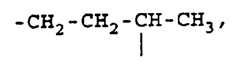 and pharmaceutically usable hydrates and acid addition salts thereof and the alkali metal, alkaline earth metal, silver and guanidinium salts of the underlying carboxylic acids.

2. Compounds of the formula (I) according to Claim 1, in which
X¹ represents fluorine or chlorine,
X² represents hydrogen, amino, alkylamino having 1 or 2 carbon atoms, dimethylamino, hydroxyl, methoxy mercapto, methylthio, phenylthio, fluorine or chlorine,
R¹ represents alkyl having 1 to 3 carbon atoms, alkenyl having 2 or 3 carbon atoms, cycloalkyl having 3 to 5 carbon atoms, 2-hydroxyethyl, 2-fluoroethyl, methoxy, amino, methylamino, ethylamino, dimethylamino or phenyl which is optionally substituted by 1 or 2 fluorine atoms,
R² represents hydrogen, alkyl having 1 to 3 carbon atoms or (5-methyl-2-oxo-1,3-dioxol-4-yl)-methyl,
R³ represents a radical of the structure 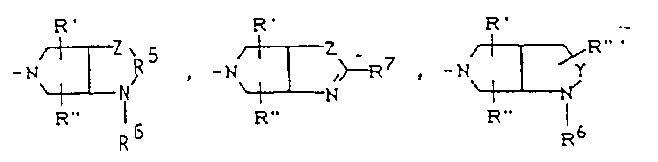 wherein
R⁵ denotes a C₁-C₂-alkylene bridge which is optionally mono- or disubstituted by methyl,
R⁶ can represent H, optionally hydroxyl-substituted C₁-C₃-alkyl and phenyl, benzyl, C₁-C₄-alkoxycarbonyl, C₁-C₂-acyl, (5-methyl-2-oxo-1,3-dioxol-4-yl)-methyl or C₃-C₅-alkyl,
R⁷ can represent H or C₁-C₂-alkyl,
R' can represent H or CH₃,
R" can represent H or CH₃,
R"' can represent H or CH₃,
Y can represent O, CH₂, CH₂CH₂ or CH₂-0, it being possible for the CH₂-0 group to be linked to the nitrogen either via O or via CH₂, and
Z can represent O or S, and
A represents N or C-R⁸, wherein
R⁸ represents H, fluorine, chlorine, bromine, hydroxyl or methoxy or, together with R¹, also can form a bridge of the structure

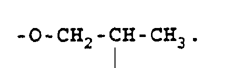

3. Compounds of the formula (I) according to Claim 1, in which
X¹ represents fluorine,
X² represents hydrogen, amino, methylamino or fluorine,
R¹ represents alkyl having 1 or 2 carbon atoms, vinyl, cyclopropyl, 2-hydroxyethyl, 2-fluoroethyl, methoxy, methylamino, 4-fluorophenyl or 2,4-difluorophenyl,
R² represents hydrogen or alkyl having 1 or 2 carbon atoms,
R³ represents a radical of the structure 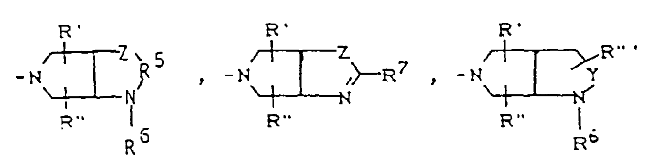 wherein
R⁵ denotes a C₁-C₂-alkylene bridge which is optionally substituted by methyl,
R⁶ can represent H, CH₃, C₂H₅, HOCH₂CH₂, benzyl, C₁-C₄-alkoxycarbonyl or C₁-C₂-acyl,
R⁷ can represent H or CH₃,
R' can represent H or CH₃,
R" can represent H or CH₃,
R"' can represent H or CH₃,
Y can represent O, CH₂, CH₂CH₂ or CH₂-0, it being possible for the CH₂-0 group to be linked to the nitrogen either via O or via CH₂, and
Z can represent O or S, and
A represents N or C-R⁸, wherein
R⁸ represents H, fluorine, chlorine or methoxy or, together with R¹ , also can form a bridge of the structure

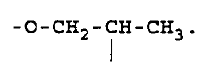

4. Process for the preparation of compounds according to Claim 1 of the formula (I) 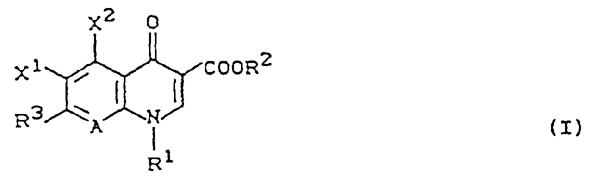 in which
X¹ represents halogen,
X² represents hydrogen, amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms, arylthio or halogen,
R¹ represents alkyl having 1 to 4 carbon atoms, alkenyl having 2 to 4 carbon atoms, cycloalkyl having 3 to 6 carbon atoms, 2-hydroxyethyl, 2-fluoroethyl, methoxy, amino, methylamino, ethylamino, dimethylamino or phenyl which is optionally substituted by 1 or 2 fluorine atoms,
R² represents hydrogen, alkyl having 1 to 4 carbon atoms or (5-methyl-2-oxo-1,3-dioxol-4-yl)-methyl,
R³ represents a radical of the structure 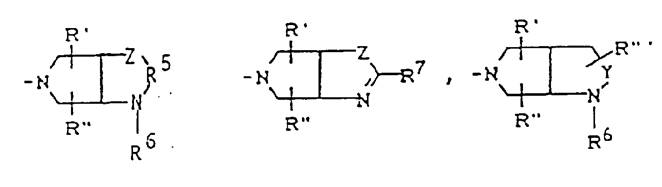 wherein
R⁵ denotes a C₁-C₃-alkylene bridge which is optionally mono- or disubstituted by methyl,
R⁶ can represent H, optionally hydroxyl-substituted C₁-C₄-alkyl, and aryl, heteroaryl, benzyl, C₁-C₄-alkoxycarbonyl, C₁-C₄-acyl, (5-methyl-2-oxo-1,3-dioxol-4-yl)-methyl, or C₃-C₆ cycloalkyl,
R⁷ can represent H or C₁-C₄-alkyl,
R' can represent H, CH₃ or phenyl,
R" can represent H, CH₃ or phenyl,
R"' can represent H or CH₃,
Y can represent O, CH₂, CH₂CH₂ or CH₂-0, it being possible for the CH₂-0 group to be linked to the nitrogen either via 0 or via CH₂, and
Z can represent O or S, and
A represents N or C-R⁸, wherein
R⁸ represents H, halogen, methyl, cyano, nitro, hydroxyl or methoxy or, together with R⁷, also can form a bridge of the structure 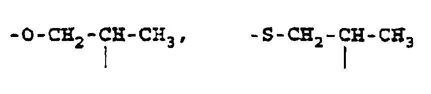 or 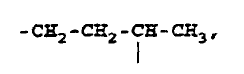
characterised in that a compound of formula (II) 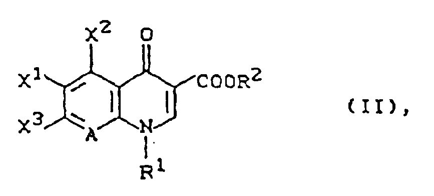 in which
A R¹, R², X¹ and X² have the abovementioned meaning and
X³ represents halogen, in particular fluorine or chlorine,
is reacted with compounds of the formula (III)
in which
R³ has the abovementioned meaning,
if appropriate in the presence of acid scavengers, and if appropriate protective groups contained in R³ are removed.

5. Process for the preparation of compounds according to Claim 1 of the formula (I) 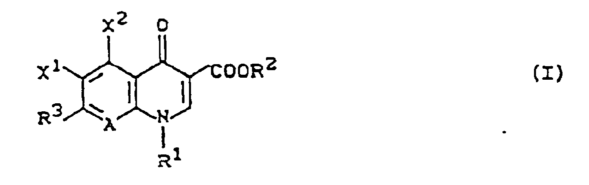 in which
X¹ represents halogen,
R¹ represents alkyl having 1 to 4 carbon atoms, alkenyl having 2 to 4 carbon atoms, cycloalkyl having 3 to 6 carbon atoms, 2-hydroxyethyl, 2-fluoroethyl, methoxy, amino, methylamino, ethylamino, dimethylamino or phenyl which is optionally substituted by 1 or 2 fluorine atoms,
R² represents hydrogen, alkyl having 1 to 4 carbon atoms or (5-methyl-2-oxo-1,3-dioxol-,4-yl)-methyl),
R³ represents a radical of the structure 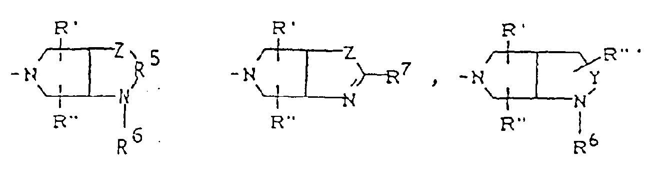 wherein
R⁵ denotes a C₁-C₃-alkylene bridge which is optionally mono- or disubstituted by methyl,
R⁶ can represent H, optionally hydroxyl-substituted C₁-C₄-alkyl, and aryl, heteroaryl, benzyl, C₁-C₄-alkoxycarbonyl, C₁-C₄-acyl, (5-methyl-2-oxo-1,3-dioxol-4-yl)-methyl, or C₃-C₆-cycloalkyl,
R⁷ can represent H or C₁-C₄-alkyl,
R' can represent H, CH₃ or phenyl,
R" can represent H, CH₃ or phenyl,
R"' can represent H or CH₃,
Y can represent O, CH₂, CH₂CH₂ or CH₂-0, it being possible for the CH₂-0 group to be linked to the nitrogen either via O or via CH₂, and
Z can represent O or S, and
A represents N or C-R⁸, wherein
R⁸ represents H, halogen, methyl, cyano, nitro, hydroxyl or methoxy or, toqether with R¹, also can form a bridge of the structure 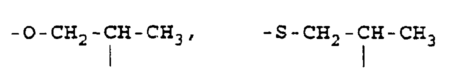 or 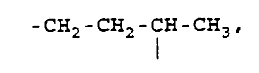
X² represents amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms or arylthio,
characterized in that a compound of the formula (IV) 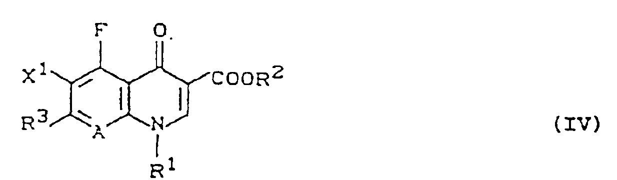 in which
X¹, R¹, R², R³ and A have the abovementioned meaning,
is reacted with compounds of the formula (V) 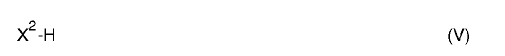 in which
X² has the abovementioned meaning, if appropriate in the presence of acid scavengers.

6. Process for the preparation of compounds according to Claim 1 of the formula (la) 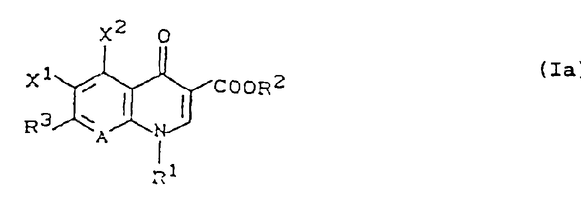 in which
X¹ represents halogen,
X² represents hydrogen, amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms, arylthio or halogen,
R¹ represents alkyl having 1 to 4 carbon atoms, alkenyl having 2 to 4 carbon atoms, cycloalkyl having 3 to 6 carbon atoms, 2-hydroxyethyl, 2-fluoroethyl, methoxy, amino, methylamino, ethylamino, dimethylamino or phenyl which is optionally substituted by 1 or 2 fluorine atoms,
R² represents hydrogen, alkyl having 1 to 4 carbon atoms or (5-methyl-2-oxo-1,3-dioxol-4-yl)-methyl,
A represents N or C-R⁸, wherein
R⁸ represents H, halogen, methyl, cyano, nitro, hydroxyl or methoxy or, together with R¹, also can form a bridge of the structure 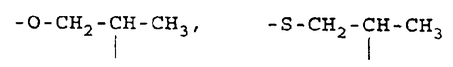 or 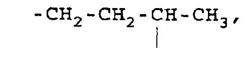 and R³ represents a radical of the structure 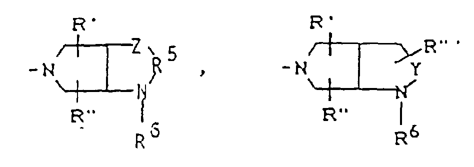 wherein
R⁵, R⁶, R', R" R"', Y and Z have the abovementioned meaning,
characterized in that a compound of the formula (VI) 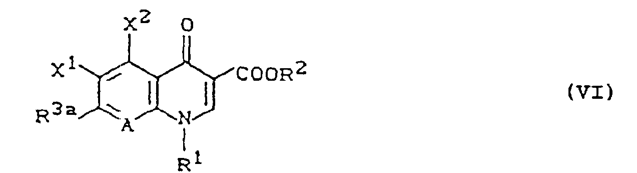 in which
X¹, X², R¹, R² and A have the abovementioned meaning and
R³ₐ represents a radical of the structure 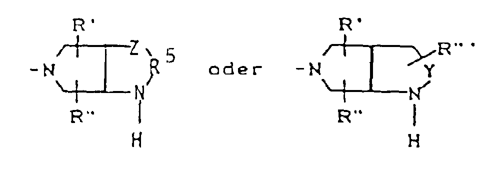 wherein
R⁵, R', R", R"', Y and Z have the abovementioned meaning,
is reacted with compounds of the formula (VII) 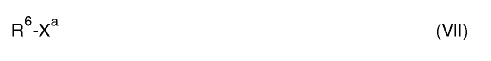 in which
R⁶ has the abovementioned meaning and
X^{a} represents chlorine, bromine, iodine, hydroxyl or acyloxy,
if appropriate in the presence of acid scavengers.

7. 7-(1-Pyrrolidinyl)-3-quinolone- and -naphthyridone-carboxylic acid derivatives of the formula (I) according to Claim 1 for use in a method for combating diseases.

8. Medicaments containing compounds of the formula (I) according to Claim 1.

9. Use of compounds of the formula (I) according to Claim 1 for the preparation of medicaments.

10. Use of compounds of the formula (I) according to Claim 1 as animal feed additives.

11. Animal feeds or animal feed additives and premixes containing compounds of the formula (I) according to Claim 1.

12. Compounds from the group consisting of
2-oxa-5,8-diazabicyclo[4.3.0]nonane dihydrochloride, trans-2-oxa-5,8-diazabicyclo[4.3.0]nonane,
5-methyl-2-oxa-5,8-diazabicyclo[4.3.0]nonane dihydrochloride,
2,8-diazabicyclo[4.3.0]nonane,
4-methyl-2,8-diazabicyclo[4.3.0]nonane,
2-methyl-2,8-diazabicyclo[4.3.0]nonane,
2,7-diazabicyclo[3.3.0]octane,
2-methyl-2,7-diazabicyclo[3.3.0]octane,
3-oxa-2,7-diazabicyclo[3.3.0]octane,
2-methyl-3-oxa-2,7-diazabicyclo[3.3.0]octane,
2,5-dimethyl-3-oxa-2,7-diazabicyclo[3.3.0]octane,
2,8-dimethyl-3-oxa-2,7-diazabicyclo[3.3.0]octane,
2-methyl-4-oxa-2,8-diazabicyclo[4.3.0]nonane,
3-methyl-2,7-diazabicyclo[3.3.0]octane,
2,3-dimethyl-2,7-diazabicyclo[3.3.0]octane, ethyl 2,7-diazabicyclo[3.3.0]octane-2-carboxylate,
2-phenyl-2,7-diazabicyclo[3.3.0]octane,
4-oxa-2,8-diazabicyclo[4.3.0]nonane.

13. Compounds from the group consisting of
5,6,7,8-tetrafluoro-1-(2,4-difluorophenyl)-1,4-dihydro-4-oxo-3-quinolinecarboxylic acid,
5,7-dichloro-1-cyc1opropyl-6-fluoro-1,4-dihydro-4-oxo-3-quinolinecarboxylic acid and
5,7-dich1oro-6-f1 uoro-1-(2,4-difluorophenyl)-1,4-dihydro-4-oxo-3-quinolinecarboxylic acid.

14. Carboxylic acid derivatives of the formula (I) according to Claim 1,
wherein
X¹ represents fluorine,
X² represents hydrogen,
R¹ represents cyclopropyl,
R² represents hydrogen,
R³ represents a radical having the formula 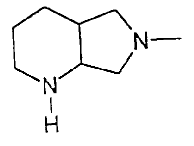 and
A represents C-CI, C-F or C-OCH₃,
and their pharmaceutically tolerable salts.

15. Carboxylic acid derivatives according to Claim 14 for use as antibacterial agents.

16. Use of compounds according to Claim 14 for the production of medicaments against bacterial disorders.

17. Use of compounds according to Claim 14 in animal feed.

## Claims (Claims for the following Contracting State(s) : GR)

1. Process for the preparation of 7-(1-pyrrolidinyl)-3-quinolone- and -naphthyridone-carboxylic acid derivatives of the formula (I) 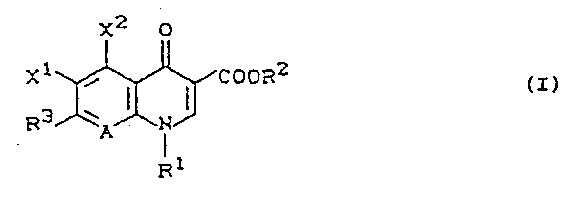 in which
X¹ represents halogen,
X² represents hydrogen, amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms, arylthio or halogen,
R¹ represents alkyl having 1 to 4 carbon atoms, alkenyl having 2 to 4 carbon atoms, cycloalkyl having 3 to 6 carbon atoms, 2-hydroxyethyl, 2-fluoroethyl, methoxy, amino, methylamino, ethylamino, dimethylamino or phenyl which is optionally substituted by 1 or 2 fluorine atoms,
R² represents hydrogen, alkyl having 1 to 4 carbon atoms or (5-methyl-2-oxo-1,3-dioxol-4-yl)-methyl,
R³ represents a radical of the structure 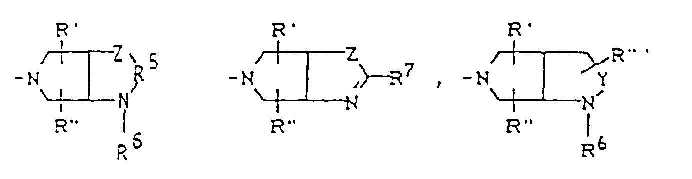 wherein
R⁵ denotes a C₁-C₃-alkylene bridge which is optionally mono- or disubstituted by methyl,
R⁶ can represent H, optionally hydroxyl-substituted C₁-C₄-alkyl and aryl, heteroaryl, benzyl, C₁-C₄-alkoxycarbonyl, C₁-C₄-acyl, (5-methyl-2-oxo-1,3-dioxol-4-yl)-methyl or C₃-C₆-cycloalkyl,
R⁷ can represent H or C₁-C₄-alkyl,
R' can represent H, CH₃ or phenyl,
R" can represent H, CH₃ or phenyl,
R"' can represent H or CH₃,
Y can represent O, CH₂, CH₂CH₂ or CH₂-0, it being possible for the CH₂-0 group to be linked to the nitrogen either via O or via CH₂, and
Z can represent O or S, and
A represents N or C-R⁸, wherein
R⁸ represents H, halogen, methyl, cyano, nitro, hydroxyl, or methoxy or, together with R¹, can form a bridge of the structure 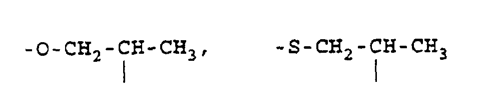 or 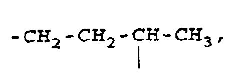 and pharmaceutically usable hydrates and acid addition salts thereof and the alkali metal, alkaline earth metal, silver and guanidinium salts of the underlying carboxylic acids, comprising the reaction of a compound of the formula (II) 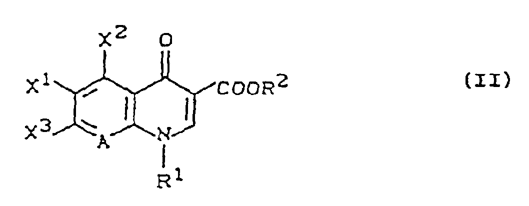 in which
A, R¹, R², X¹ and X² have the abovementioned meaning and
X³ represents halogen, in particular fluorine or chlorine,
with compounds of the formula (III) 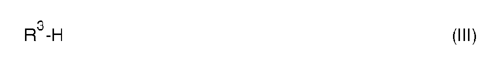 in which
R³ has the abovementioned meaning,
if appropriate in the presence of acid scavengers, and if appropriate with removal of protective groups contained in _{R3}.

2. Process for the preparation of compounds having the formula (I) according to Claim 1 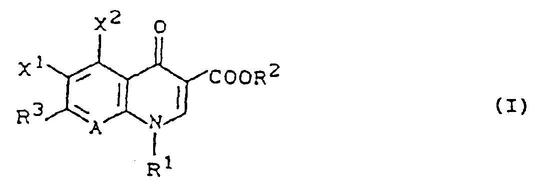 in which
X¹, R¹, R², R³ and A have the meanings given in Claim 1 and
X² represents amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms or arylthio,
characterized in that a compound of the formula (IV) 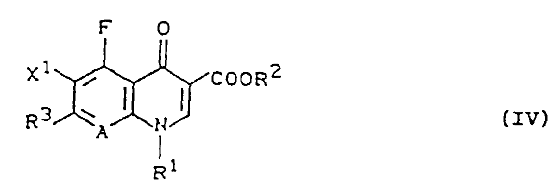 in which
X¹, R¹, R², R³ and A have the abovementioned meaning,
is reacted with compounds of the formula (V) 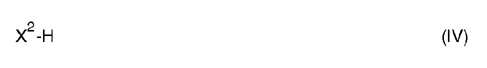 in which
X² has the abovementioned meaning, if appropriate in the presence of acid scavengers.

3. Process for the preparation of compounds having the formula (la) 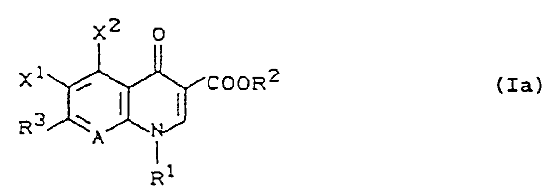 in which
X¹, X², R⁷, R² and A have the meanings given in claim 1 and R³ represents a radical of the structure 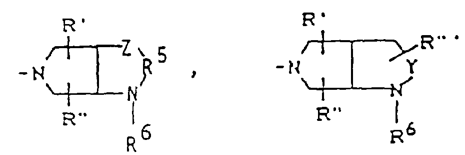 wherein
R⁵, R⁶, R', R", R"', Y and Z have the meanings given in Claim 1,
characterized in that a compound of the formula (VI) 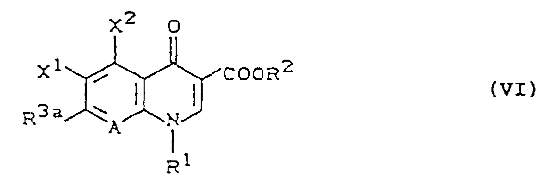 in which
X¹, X², R¹, R² and A have the abovementioned meaning and
R^{3a} represents a radical of the structure 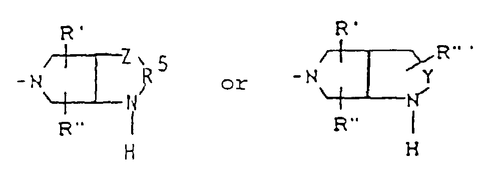 wherein
R⁵, R', R", R"', Y and Z have the abovementioned meaning,
is reacted with compounds of the formula (VII) 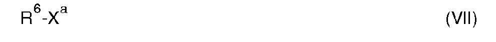 in which
R⁶ has the abovementioned meaning and
X^{a} represents chlorine, bromine, iodine, hydroxyl or acyloxy,
if appropriate in the presence of acid scavengers.

4. Process according to one of Claims 1 to 3, in which in formula (I) or (la)
X¹ represents fluorine or chlorine,
X² represents hydrogen, amino, alkylamino having 1 or 2 carbon atoms, dimethylamino, hydroxyl, methoxy mercapto, methylthio, phenylthio, fluorine or chlorine,
R¹ represents alkyl having 1 to 3 carbon atoms, alkenyl having 2 or 3 carbon atoms, cycloalkyl having 3 to 5 carbon atoms, 2-hydroxyethyl, 2-fluoroethyl, methoxy amino, methylamino, ethylamino, dimethylamino or phenyl which is optionally substituted by 1 or 2 fluorine atoms,
R² represents hydrogen, alkyl having 1 to 3 carbon atoms or (5-methyl-2-oxo-1,3-dioxol-4-yl)-methyl,
R³ represents a radical of the structure 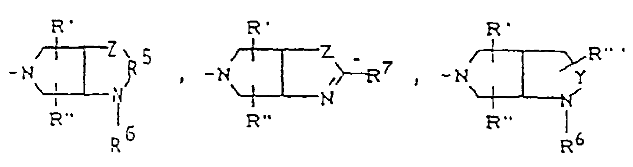 wherein
R⁵ denotes a C₁-C₂-alkylene bridge which is optionally mono- or disubstituted by methyl,
R⁶ can represent H, optionally hydroxyl-substituted C₁-C₃-alkyl and phenyl, benzyl, C₁-C₄-alkoxycarbonyl, C₁-C₂-acyl, (5-methyl-2-oxo-1,3-dioxol-4-yl)-methyl or C₃-C₅-alkyl,
R⁷ can represent H or C₁-C₂-alkyl,
R' can represent H or CH₃,
R" can represent H or CH₃,
R"' can represent H or CH₃,
Y can represent O, CH₂, CH₂CH₂ or CH₂-0, it being possible for the CH₂-0 group to be linked to the nitrogen either via O or via CH₂, and
Z can represent O or S, and
A represents N or C-R⁸, wherein
R⁸ represents H, fluorine, chlorine, bromine, hydroxyl or methoxy or, together with R¹, also can form a bridge of the structure

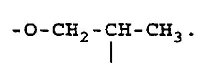

5. Process according to one of Claims 1 to 3, in which in formula (I) or (la)
X¹ represents fluorine,
X² represents hydrogen, amino, methylamino or fluorine,
R¹ represents alkyl having 1 or 2 carbon atoms, vinyl, cyclopropyl, 2-hydroxyethyl, 2-fluoroethyl, methoxy methylamino, 4-fluorophenyl or 2,4-difluorophenyl,
R² represents hydrogen or alkyl having 1 or 2 carbon atoms,
R³ represents a radical of the structure 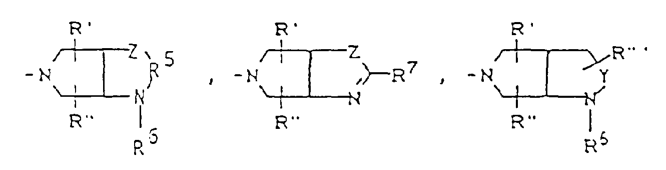 wherein
R⁵ denotes a C₁-C₂-alkylene bridge which is optionally substituted by methyl,
R⁶ can represent H, CH₃, C₂H₅, HOCH₂CH₂, benzyl, C₁-C₄-alkoxycarbonyl or C₁-C₂-acyl,
R⁷ can represent H or CH₃,
R' can represent H or CH₃,
R" can represent H or CH₃,
R"' can represent H or CH₃,
Y can represent O, CH₂, CH₂CH₂ or CH₂-0, it being possible for the CH₂-0 group to be linked to the nitrogen either via O or via CH₂, and
Z can represent O or S, and
A represents N or C-R⁸, wherein
R⁸ represents H, fluorine, chlorine or methoxy or, together with R¹, also can form a bridge of the structure

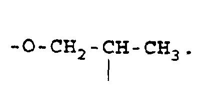

6. Process according to Claim 1, in which one of the following compounds having the formula (III) is reacted:
2-oxa-5,8-diazabicyclo[4.3.0]nonane dihydrochloride, trans-2-oxa-5,8-diazabicyclo[4.3.0]nonane,
5-methyl-2-oxa-5,8-diazabicyclo[4.3.0]nonane dihydrochloride,
2,8-diazabicyclo[4.3.0]nonane,
4-methyl-2,8-diazabicyclo[4.3.0]nonane,
2-methyl-2,8-diazabicyclo[4.3.0]nonane,
2,7-diazabicyclo[3.3.0]octane,
2-methyl-2,7-diazabicyclo[3.3.0]octane,
3-oxa-2,7-diazabicyclo[3.3.0]octane,
2-methyl-3-oxa-2,7-diazabicyclo[3.3.0]octane,
2,5-dimethyl-3-oxa-2,7-diazabicyclo[3.3.0]octane,
2,8-dimethyl-3-oxa-2,7-diazabicyclo[3.3.0]octane,
2-methyl-4-oxa-2,8-diazabicyclo[4.3.0]nonane,
3-methyl-2,7-diazabicyclo[3.3.0]octane,
2,3-dimethyl-2,7-diazabicyclo[3.3.0]octane, ethyl 2,7-diazabicyclo[3.3.0]octane-2-carboxylate,
2-phenyl-2,7-diazabicyclo[3.3.0]octane,
4-oxa-2,8-diazabicyclo[4.3.0]nonane.

7. Process according to Claim 1, in which one of the following compounds having the formula (II) is reacted:
5,6,7,8-tetrafl uoro-1 -(2,4-difluorophenyl)-1,4-dihydro-4-oxo-3-quinolinecarboxylic acid,
5,7-dichloro-1-cyclopropyl-6-fluoro-1,4-dihydro-4-oxo-3-quinolinecarboxylic acid and
5,7-dichloro-6-f1 uoro-1 -(2,4-difluorophenyl)-1,4-dihydro-4-oxo-3-quinolinecarboxylic acid.

8. Process according to Claim 1 for the preparation of carboxylic acid derivatives of the formula (I) in which
X¹ represents fluorine,
X² represents hydrogen,
R¹ represents cyclopropyl,
R² represents hydrogen,
R³ represents a radical having the formula 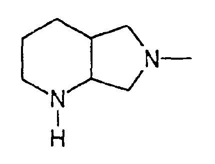 and
A represents C-CI, C-F or C-OCH₃.

9. 7-(1-Pyrrolidinyl)-3-quinolone- and -naphthyridonecarboxylic acid derivatives of the formula (I) 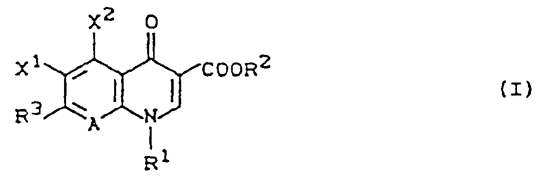 in which
X¹ represents halogen,
X² represents hydrogen, amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms, arylthio or halogen,
R¹ represents alkyl having 1 to 4 carbon atoms, alkenyl having 2 to 4 carbon atoms, cycloalkyl having 3 to 6 carbon atoms, 2-hydroxyethyl, 2-fluoroethyl, methoxy, amino, methylamino, ethylamino, dimethylamino or phenyl which is optionally substituted by 1 or 2 fluorine atoms,
R² represents hydrogen, alkyl having 1 to 4 carbon atoms or (5-methyl-2-oxo-1,3-dioxol-4-yl)-methyl, and
R³ represents a radical of the structure 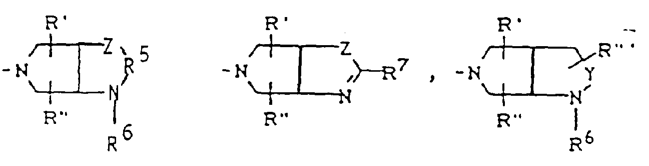 wherein
R⁵ denotes a C₁-C₃-alkylene bridge which is optionally mono- or disubstituted by methyl,
R⁶ can represent H, optionally hydroxyl-substituted C₁-C₄-alkyl and aryl, heteroaryl, benzyl, C₁-C₄-alkoxycarbonyl, C₁-C₄-acyl, (5-methyl-2-oxo-1,3-dioxol-4-yl)-methyl or C₃₋C₆-cycloalkyl,
R⁷ can represent H or C₁-C₄-alkyl,
R' can represent H, CH₃ or phenyl,
R" can represent H, CH₃ or phenyl,
R"' can represent H or CH₃,
Y can represent O, CH₂, CH₂CH₂ or CH₂-O, it being possible for the CH₂-0 group to be linked to the nitrogen either via O or via CH₂, and
Z can represent 0 or S, and
A represents N or C-R⁸, wherein
R⁸ represents H, halogen, methyl, cyano, nitro, hydroxyl or methoxy or, together with R¹, also can form a bridge of the structure 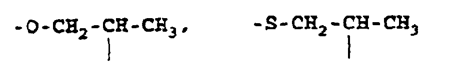 or 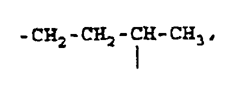 and pharmaceutically usable hydrates and acid addition salts thereof and the alkali metal, alkaline earth metal, silver and guanidinium salts of the underlying carboxylic acids.

10. Compounds of the formula (I) according to Claim 9, in which
X¹ represents fluorine or chlorine,
X² represents hydrogen, amino, alkylamino having 1 or 2 carbon atoms, dimethylamino, hydroxyl, methoxy mercapto, methylthio, phenylthio, fluorine or chlorine,
R¹ represents alkyl having 1 to 3 carbon atoms, alkenyl having 2 or 3 carbon atoms, cycloalkyl having 3 to 5 carbon atoms, 2-hydroxyethyl, 2-fluoroethyl, methoxy amino, methylamino, ethylamino, dimethylamino or phenyl which is optionally substituted by 1 or 2 fluorine atoms,
R² represents hydrogen, alkyl having 1 to 3 carbon atoms or (5-methyl-2-oxo-1,3-dioxol-4-yl)-methyl,
R³ represents a radical of the structure 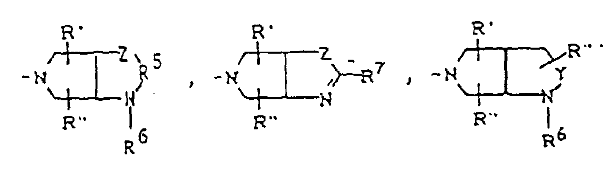 wherein
R⁵ denotes a C₁-C₂-alkylene bridge which is optionally mono- or disubstituted by methyl,
R⁶ can represent H, optionally hydroxyl-substituted C₁-C₃-alkyl and phenyl, benzyl, C₁-C₄-alkoxycarbonyl, C₁-C₂-acyl, (5-methyl-2-oxo-1,3-dioxol-4-yl)-methyl or C₃-C₅-alkyl,
R⁷ can represent H or C₁-C₂-alkyl,
R' can represent H or CH₃,
R" can represent H or CH₃,
R"' can represent H or CH₃,
Y can represent O, CH₂, CH₂CH₂ or CH₂-0, it being possible for the CH₂-0 group to be linked to the nitrogen either via O or via CH₂, and
Z can represent O or S, and
A represents N or C-R⁸, wherein
R⁸ represents H, fluorine, chlorine, bromine, hydroxyl or methoxy or, together with R¹, also can form a bridge of the structure

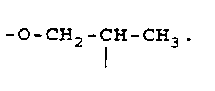

11. Compounds of the formula (I) according to Claim 9, in which
X¹ represents fluorine,
X² represents hydrogen, amino, methylamino or fluorine,
R¹ represents alkyl having 1 or 2 carbon atoms, vinyl, cyclopropyl, 2-hydroxyethyl, 2-fluoroethyl, methoxy, methylamino, 4-fluorophenyl or 2,4-difluorophenyl,
R² represents hydrogen or alkyl having 1 or 2 carbon atoms,
R³ represents a radical of the structure 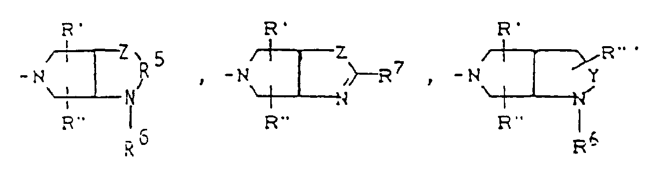 wherein
R⁵ denotes a C₁-C₂-alkylene bridge which is optionally substituted by methyl,
R⁶ can represent H, CH₃, C₂H₅, HOCH₂CH₂, benzyl, C₁-C₄-alkoxycarbonyl or C₁-C₂-acyl,
R⁷ can represent H or CH₃,
R' can represent H or CH₃,
R" can represent H or CH₃,
R"' can represent H or CH₃,
Y can represent O, CH₂, CH₂CH₂ or CH₂-0, it being possible for the CH₂-0 group to be linked to the nitrogen either via O or via CH₂, and
Z can represent O or S, and
A represents N or C-R⁸, wherein
R⁸ represents H, fluorine, chlorine or methoxy, or together with R¹ also can form a bridge of the structure

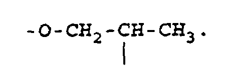

12. Use of compounds of the formula (I) according to Claim 9 for the production of medicaments.

13. Use of compounds of the formula (I) according to Claim 19 as animal feed additives.

14. Animal feeds or animal feed additives and premixes containing compounds of the formula (I) according to Claim 9.

15. Compounds from the group consisting of
2-oxa-5,8-diazabicyclo[4.3.0]nonane dihydrochloride, trans-2-oxa-5,8-diazabicyclo[4.3.0]nonane,
5-methyl-2-oxa-5,8-diazabicyclo[4.3.0]nonane dihydrochloride,
2,8-diazabicyclo[4.3.0]nonane,
4-methyl-2,8-diazabicyclo[4.3.0]nonane,
2-methyl-2,8-diazabicyclo[4.3.0]nonane,
2,7-diazabicyclo[3.3.0]octane,
2-methyl-2,7-diazabicyclo[3.3.0]octane,
3-oxa-2,7-diazabicyclo[3.3.0]octane,
2-methyl-3-oxa-2,7-diazabicyclo[3.3.0]octane,
2,5-dimethyl-3-oxa-2,7-diazabicyclo[3.3.0]octane,
2,8-dimethyl-3-oxa-2,7-diazabicyclo[3.3.0]octane,
2-methyl-4-oxa-2,8-diazabicyclo[4.3.0]nonane,
3-methyl-2,7-diazabicyclo[3.3.0]octane,
2,3-dimethyl-2,7-diazabicyclo[3.3.0]octane, ethyl 2,7-diazabicyclo[3.3.0]octane-2-carboxylate,
2-phenyl-2,7-diazabicyclo[3.3.0]octane,
4-oxa-2,8-diazabicyclo[4.3.0]nonane.

16. Compounds from the group consisting of
5,6,7,8-tetrafluoro-1 -(2,4-difluorophenyl)-1,4-dihydro-4-oxo-3-quinolinecarboxylic acid,
5,7-dichloro-1-cyclopropyl-6-fluoro-1,4-dihydro-4-oxo-3-quinolinecarboxylic acid and
5,7-dichloro-6-fluoro-1 -(2,4-difluorophenyl)-1,4-dihydro-4-oxo-3-quinolinecarboxylic acid.

17. Carboxylic acid derivatives of the formula (I) according to Claim 1,
wherein
X¹ represents fluorine,
X² represents hydrogen,
R¹ represents cyclopropyl,
R² represents hydrogen,
R³ represents a radical having the formula 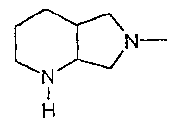 and
A represents C-CI, C-F or C-OCH₃,
and their pharmaceutically tolerable salts.

18. Carboxylic acid derivatives according to Claim 17 for use as antibacterial agents.

19. Use of compounds according to Claim 17 for the production of medicaments against bacterial disorders.

20. Use of compounds according to Claim 17 in animal feed.

## Revendications (Revendications pour l'(les) Etat(s) contractant(s) suivant(s) : AT, BE, CH, DE, ES, FR, GB, IT, LI, LU, NL, SE)

1. Dérivés d'acides 7-(1-pyrrolidinyl)-3-quinolone- et -naphtyridone-carboxyliques de formule (I) 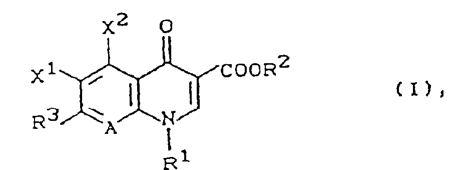 dans laquelle
X¹ représente un halogène,
X² est de l'hydrogène, un groupe amino, alkylamino ayant 1 à 4 atomes de carbone, dialkylamino ayant 1 à 3 atomes de carbone par groupe alkyle, hydroxy, alkoxy ayant 1 à 4 atomes de carbone, mercapto, alkylthio ayant 1 à 4 atomes de carbone, arylthio, un halogène,
R¹ est un groupe alkyle ayant 1 à 4 atomes de carbone, un groupe alcényle ayant 2 à 4 atomes de carbone, un groupe cycloalkyle ayant 3 à 6 atomes de carbone, un groupe 2-hydroxyéthyle, 2-fluoréthyle, méthoxy, amino, méthylamino, éthylamino, diméthylamino, phényle éventuellement substitué par un ou deux atomes de fluor,
R² est de l'hydrogène, un groupe alkyle ayant 1 à 4 atomes de carbone ou un groupe (5-méthyl-2-oxo-1,3-dioxol-4-yl)méthyle et
R³ est un reste de structure 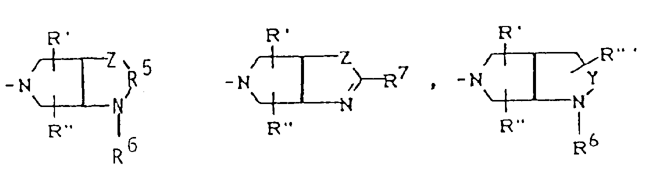 où
R⁵ est un pont alkylène en C₁ à C₃ éventuellement substitué une ou deux fois par un radical méthyle,
R⁶ peut représenter H, un groupe alkyle en C₁ à C₄ éventuellement substitué par un radical hydroxy, ainsi qu'un groupe aryle, hétéroaryle, benzyle, (alkoxy en C₁ à C₄)carbonyle, acyle en C₁ à C₄, (5-méthyl-2-oxo-1,3-dioxol-4-yl)méthyle ou cycloalkyle en C₃ à C₆,
R⁷ peut représenter H ou un groupe alkyle en C₁ à C₄,
R' peut représenter H, un groupe CH₃ ou phényle,
R" peut représenter H, CH₃ ou un groupe phényle,
R"' peut représenter H ou CH₃,
Y peut représenter O, un groupe CH₂, CH₂CH₂ ou CH₂-0, la liaison du groupe CH₂-0 à l'azote pouvant s'effectuer tant par de l'oxygène que par un groupe CH₂, et
Z peut représenter O ou S,
A représente N ou un groupe C-R⁸, dans lequel
R⁸ est de l'hydrogène, un halogène, un groupe méthyle, cyano, nitro, hydroxy ou méthoxy ou peut aussi former conjointement avec R¹ un pont de structure 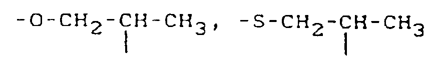 ou 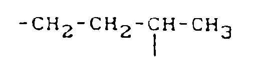 et leurs hydrates et leurs sels d'addition d'acides pharmaceutiquement utilisables ainsi que les sels de métaux alcalins, de métaux alcalino-terreux, d'argent et de guanidinium, des acides carboxyliques correspondants.

2. Composés de formule (I) suivant la revendication 1, dans lesquels
X¹ représente du fluor ou du chlore,
X² est de l'hydrogène, un groupe amino, alkylamino ayant 1 ou 2 atomes de carbone, diméthylamino, hydroxy méthoxy, mercapto, méthylthio, phénylthio, du fluor, du chlore,
R¹ est un groupe alkyle ayant 1 à 3 atomes de carbone, un groupe alcényle ayant 2 ou 3 atomes de carbone, un groupe cycloalkyle ayant 3 à 5 atomes de carbone, un groupe 2-hydroxyéthyle, 2-fluoréthyle, méthoxy, amino, méthylamino, éthylamino, diméthylamino, phényle éventuellement substitué par un ou deux atomes de fluor,
R² est de l'hydrogène, un groupe alkyle ayant 1 à 3 atomes de carbone ou (5-méthyl-2-oxo-1,3-dioxol-4-yl)méthyle,
R³ représente un reste de structure 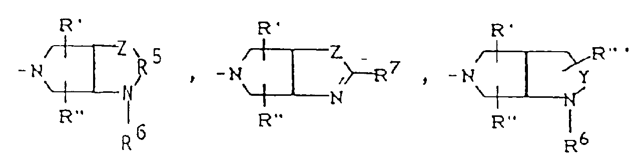 où
R⁵ représente un pont alkylène en C₁ ou C₂ éventuellement substitué une ou deux fois par un radical méthyle,
R⁶ peut représenter H, un groupe alkyle en C₁ à C₃ éventuellement substitué par un radical hydroxy, ainsi qu'un groupe phényle, benzyle, (alkoxy en C₁ à C₄)carbonyle, acyle en C₁ ou C₂, (5-méthyl-2-oxo-1,3-dioxol-4-yl) méthyle ou alkyle en C₃ à C₅,
R⁷ peut représenter H ou un groupe alkyle en C₁ ou C₂,
R' peut représenter H ou un groupe CH₃,
R" peut représenter H ou un groupe CH₃,
R"' peut représenter H ou un groupe CH₃,
Y peut représenter O, un groupe CH₂, CH₂CH₂ ou CH₂-0, la liaison du groupe CH₂-0 avec l'azote pouvant s'effectuer tant par l'intermédiaire d'oxygène que par l'intermédiaire d'un groupe CH₂, et
Z peut représenter O ou S,
A représente N ou un groupe C-R⁸, dans lequel
R⁸ représente de l'hydrogène, du fluor, du chlore, du brome, un groupe hydroxy ou méthoxy ou peut aussi former conjointement avec R¹ un pont de structure

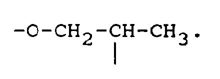

3. Composés de formule (I) suivant la revendication 1, dans lesquels
X¹ est du fluor,
X² est de l'hydrogène, un groupe amino, méthylamino, du fluor,
R¹ est un groupe alkyle ayant 1 ou 2 atomes de carbone, vinyle, cyclopropyle, 2-hydroxyéthyle, 2-fluoréthyle, méthoxy, méthylamino, 4-fluorophényle, 2,4-difluorophényle,
R² est de l'hydrogène, un groupe alkyle ayant 1 ou 2 atomes de carbone,
R³ est un reste de structure 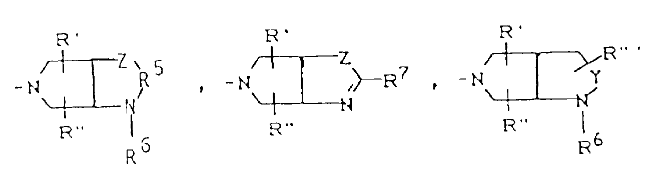 où
R⁵ représente un pont alkylène en C₁ ou C₂ substitué par un radical méthyle,
R⁶ peut être de l'hydrogène, un groupe CH₃, C₂H₅, HOCH₂CH₂, benzyle, (alkoxy en C₁ à C₄)carbonyle, acyle en C₁ ou C₂,
R⁷ peut représenter H ou CH₃,
R' peut représenter H ou CH₃,
R" peut représenter H ou CH₃,
R"' peut représenter H ou CH₃,
Y peut représenter O, un groupe CH₂, CH₂CH₂ ou CH₂-0, la liaison du groupe CH₂-0 à l'azote pouvant s'effectuer tant par l'intermédiaire d'oxygène qu'au moyen d'un groupe CH₂, et
Z peut représenter O ou S,
A représente de l'azote ou un groupe C-R⁸ dans lequel
R⁸ est de l'hydrogène, du fluor, du chlore ou un groupe méthoxy, ou peut aussi former conjointement avec R¹ un pont de structure

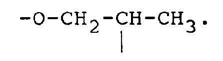

4. Procédé de production de composés de formule (I) suivant la revendication 1 de formule (I), 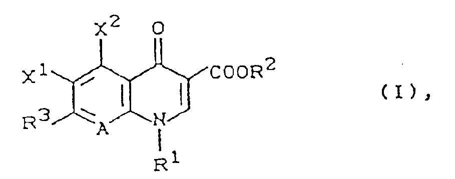 dans laquelle
X¹ représente un halogène,
X² est de l'hydrogène, un groupe amino, alkylamino ayant 1 à 4 atomes de carbone, dialkylamino ayant 1 à 3 atomes de carbone par groupe alkyle, hydroxy, alkoxy ayant 1 à 4 atomes de carbone, mercapto, alkylthio ayant 1 à 4 atomes de carbone, arylthio, un halogène,
R¹ est un groupe alkyle ayant 1 à 4 atomes de carbone, un groupe alcényle ayant 2 à 4 atomes de carbone, un groupe cycloalkyle ayant 3 à 6 atomes de carbone, un groupe 2-hydroxyéthyle, 2-fluoréthyle, méthoxy amino, méthylamino, éthylamino, diméthylamino, phényle éventuellement substitué par un ou deux atomes de fluor,
R² est de l'hydrogène, un groupe alkyle ayant 1 à 4 atomes de carbone ou un groupe (5-méthyl-2-oxo-1,3-dioxol-4-yl)méthyle et
R³ est un reste de structure 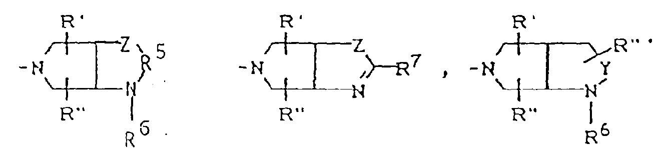 où
R⁵ est un pont alkylène en C₁ à C₃ éventuellement substitué une ou deux fois par un radical méthyle,
R⁶ peut représenter H, un groupe alkyle en C₁ à C₄ éventuellement substitué par un radical hydroxy, ainsi qu'un groupe aryle, hétéroaryle, benzyle, (alkoxy en C₁ à C₄)carbonyle, acyle en C₁ à C₄, (5-méthyl-2-oxo-1,3-dioxol-4-yl)méthyle ou cycloalkyle en C₃ à C₆,
R⁷ peut représenter H ou un groupe alkyle en C₁ à C₄,
R' peut représenter H, un groupe CH₃ ou phényle,
R" peut représenter H, CH₃ ou un groupe phényle,
R"' peut représenter H ou CH₃,
Y peut représenter O, un groupe CH₂, CH₂CH₂ ou CH₂-0, la liaison du groupe CH₂-0 à l'azote pouvant s'effectuer tant par de l'oxygène que par un groupe CH₂, et
Z peut représenter O ou S,
A représente N ou un groupe C-R⁸, dans lequel
R⁸ est de l'hydrogène, un halogène, un groupe méthyle, cyano, nitro, hydroxy ou méthoxy ou peut aussi former conjointement avec R¹ un pont de structure 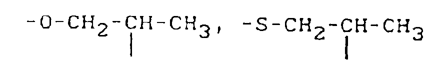 ou 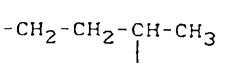
caractérisé en ce qu'on fait réagir des composés de formule (II) 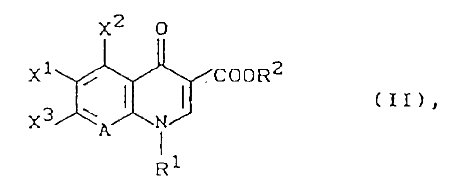 dans laquelle
A, R¹, R², X¹ et X² ont la définition indiquée ci-dessus et
X³ représente un halogène, en particulier le fluor ou le chlore, avec des composés de formule (III) 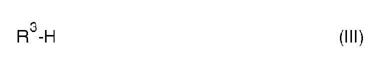 dans laquelle
R³ a la définition indiquée ci-dessus,
le cas échéant en présence d'accepteurs d'acides, et on élimine éventuellement les groupes protecteurs contenus dans R3_{.}

5. Procédé de production de composés suivant la revendication 1, de formule (I) 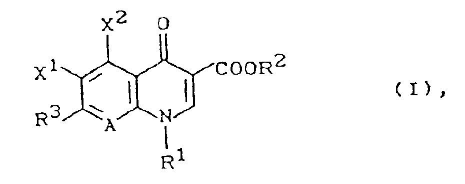 dans laquelle
X¹ est un halogène,
R¹ est un groupe alkyle ayant 1 à 4 atomes de carbone, un groupe alcényle ayant 2 à 4 atomes de carbone, un groupe cycloalkyle ayant 3 à 6 atomes de carbone, un groupe 2-hydroxyéthyle, 2-fluoréthyle, méthoxy, amino, méthylamino, éthylamino, diméthylamino, phényle éventuellement substitué par un ou deux atomes de fluor,
R² est de l'hydrogène, un groupe alkyle ayant 1 à 4 atomes de carbone ou un groupe (5-méthyl-2-oxo-1,3-dioxol-4-yl)méthyle et
R³ est un reste de structure 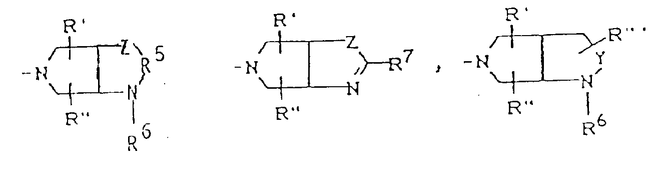 où
R⁵ est un pont alkylène en C₁ à C₃ éventuellement substitué une ou deux fois par un radical méthyle,
R⁶ peut représenter H, un groupe alkyle en C₁ à C₄ éventuellement substitué par un radical hydroxy, ainsi qu'un groupe aryle, hétéroaryle, benzyle, (alkoxy en C₁ à C₄)carbonyle, acyle en C₁ à C₄, (5-méthyl-2-oxo-1,3-dioxol-4-yl)méthyle ou cycloalkyle en C₃ à C₆,
R⁷ peut représenter H ou un groupe alkyle en C₁ à C₄,
R' peut représenter H, un groupe CH₃ ou phényle,
R" peut représenter H, CH₃ ou un groupe phényle,
R"' peut représenter H ou CH₃,
Y peut représenter O, un groupe CH₂, CH₂CH₂ ou CH₂-0, la liaison du groupe CH₂-0 à l'azote pouvant s'effectuer tant par de l'oxygène que par un groupe CH₂, et
Z peut représenter O ou S,
A représente N ou un groupe C-R⁸, dans lequel
R⁸ est de l'hydrogène, un halogène, un groupe méthyle, cyano, nitro, hydroxy ou méthoxy ou peut aussi former conjointement avec R¹ un pont de structure 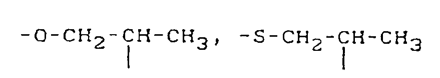 ou 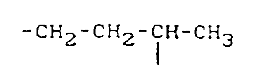 dans laquelle
X² est un groupe amino, alkylamino ayant 1 à 4 atomes de carbone, dialkylamino ayant 1 à 3 atomes de carbone par groupe alkyle, hydroxy, alkoxy ayant 1 à 4 atomes de carbone, mercapto, alkylthio ayant 1 à 4 atomes de carbone ou arylthio,
caractérisé en ce qu'on fait réagir un composé de formule (IV) 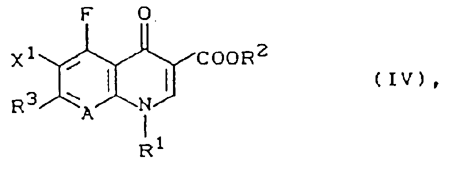 dans laquelle
X¹, R¹, R², R³ et A ont la définition indiquée ci-dessus, avec des composés de formule (V) 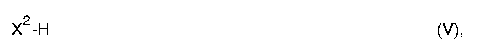 dans laquelle
X² a la définition indiquée ci-dessus, le cas échéant en présence d'accepteurs d'acides.

6. Procédé de production de composés suivant la revendication 1, de formule (la) 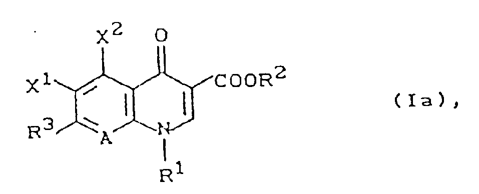 dans laquelle
X¹ est un halogène,
X² est de l'hydrogène, un groupe amino, alkylamino ayant 1 à 4 atomes de carbone, dialkylamino ayant 1 à 3 atomes de carbone par groupe alkyle, hydroxy, alkoxy ayant 1 à 4 atomes de carbone, mercapto, alkylthio ayant 1 à 4 atomes de carbone, arylthio, un halogène,
R¹ est un groupe alkyle ayant 1 à 4 atomes de carbone, un groupe alcényle ayant 2 à 4 atomes de carbone, un groupe cycloalkyle ayant 3 à 6 atomes de carbone, un groupe 2-hydroxyéthyle, 2-fluoréthyle, méthoxy, amino, méthylamino, éthylamino, diméthylamino, un groupe phényle éventuellement substitué par 1 ou 2 atomes de fluor,
R² est de l'hydrogène, un groupe alkyle ayant 1 à 4 atomes de carbone ou un groupe (5-méthyl-2-oxo-1,3-dioxol-4-yl)-méthyle,
A représente N ou un groupe C-R⁸, dans lequel
R⁸ représente H, un halogène, un groupe méthyle, cyano, nitro, hydroxy ou méthoxy ou peut aussi former conjointement avec R¹ un pont de structure 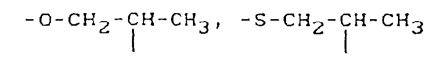 ou 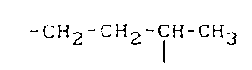 et R³ représente un reste de structure 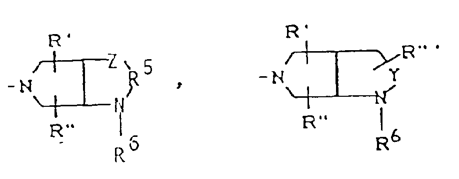 où R⁵, R⁶, R', R", R"', Y et Z ont la définition ci-dessus, caractérisé en ce qu'on fait réagir un composé de formule (VI) 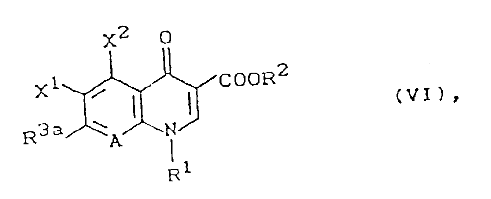 dans laquelle
X¹, X², R¹, R² et A ont la définition indiquée ci-dessus et
_{R}3a représente un reste de structure 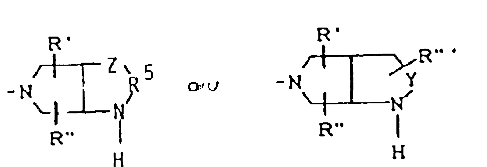 où R⁵, R', R"; R"', Y et Z ont la définition indiquée ci-dessus,
avec des composés de formule (VII) 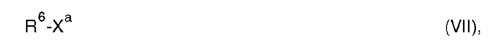 dans laquelle
R⁶ a la définition indiquée ci-dessus et
X^{a} représente du chlore, du brome, de l'iode, un groupe hydroxy ou acyloxy,
le cas échéant en présence d'accepteurs d'acides.

7. Dérivés d'acides 7-(1-pyrrolidinyl)-3-quinolone- et -naphtyridone-carboxyliques de formule (I) suivant la revendication 1, destinés à être utilisés dans un procédé pour combattre des maladies.

8. Médicament contenant des composés de formule (I) suivant la revendication 1.

9. Utilisation de composés de formule (I) suivant la revendication 1 pour la préparation de médicaments.

10. Utilisation de composés de formule (I) suivant la revendication 1 comme additifs pour l'alimentation du bétail.

11. Aliments pour le bétail ou additifs pour aliments pour le bétail et mélanges préalables contenant des composés de formule (I) suivant la revendication 1.

12. Composés du groupe comprenant :
le dichlorhydrate de 2-oxa-5,8-diazabicyclo-[4.3.0]nonane_{;}
le trans-2-oxa-5,8-diazabicyclo[4.3.0]nonane,
le dichlorhydrate de 5-méthyl-2-oxa-5,8-diazabicyclo[4.3.0]nonane,
le 2,8-diazabicycio[4.3.0]nonane,
le 4-méthyl-2,8-diazabicyclo[4.3.0]nonane,
le 2-méthyl-2,8-diazabicyclo[4.3.0]nonane,
le 2,7-diazabicyclo[3.3.0]octane,
le 2-méthyl-2,7-diazabicyclo[3.3.0]octane,
le 3-oxa-2,7-diazabicyclo[3.3.0]octane,
le 2-méthyl-3-oxa-2,7-diazabicyclo[3.3.0]octane,
le 2,5-diméthyl-3-oxa-2,7-diazabicyclo[3.3.0]-octane,
le 2,8-diméthyl-3-oxa-2,7-diazabicyclo[3.3.0]-octane,
le 2-méthyl-4-oxa-2,8-diazabicyclo[4.3.0]nonane,
le 3-méthyl-2,7-diazabicyclo[3.3.0]octane,
le 2,3-diméthyl-2,7-diazabicyclo[3.3.0]octane,
l'ester éthylique d'acide 2,7-diazabicyclo-[3.3.0]octane-2-carboxylique,
le 2-phényl-2,7-diazabicyclo[3.3.0]octane,
le 4-oxa-2,8-diazabicyclo[4.3.0]nonane.

13. Composés du groupe comprenant
l'acide 5,6,7,8-tétrafluoro-1-(2,4-difluorophényl)-1,4-dihydro-4-oxo-3-quinoléine-carboxylique,
l'acide 5,7-dichloro-1-cyclopropyl-6-fluoro-1,4-dihydro-4-oxo-3-quinoléine-carboxylique et
l'acide 5,7-dichloro-6-fluoro-l-(2,4-difluorophényl)-1,4-dihydro-4-oxo-3-quinoléine-carboxylique.

14. Dérivés d'acides carboxyliques de formule (I) suivant la revendication 1, dans lesquels
X¹ représente le fluor,
X² représente l'hydrogène,
R¹ représente le groupe cyclopropyle,
R² représente l'hydrogène,
R³ est un reste de formule 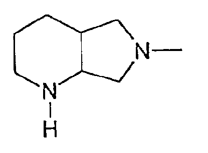
A représente C-CI, C-F OU -C-OCH₃,
et leurs sels acceptables du point de vue pharmaceutique.

15. Dérivés d'acides carboxyliques suivant la revendication 14, destinés à être utilisés comme agents antibactériens.

16. Utilisation de composés suivant la revendication 14 pour la préparation de médicaments contre des maladies bactériennes.

17. Utilisation de composés suivant la revendication 14 dans des aliments pour animaux.

## Revendications (Revendications pour l'(les) Etat(s) contractant(s) suivant(s) : GR)

1. Procédé de production de dérivés d'acides 7-(1-pyrrolidinyl)-3-quinolone- et -naphtyridone-carboxyliques de formule (I) 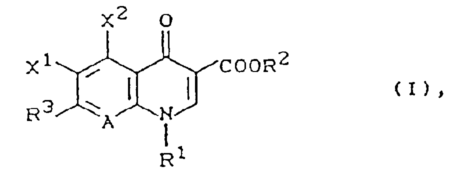 dans laquelle
X¹ représente un halogène,
X² est de l'hydrogène, un groupe amino, alkylamino ayant 1 à 4 atomes de carbone, dialkylamino ayant 1 à 3 atomes de carbone par groupe alkyle, hydroxy, alkoxy ayant 1 à 4 atomes de carbone, mercapto, alkylthio ayant 1 à 4 atomes de carbone, arylthio, un halogène,
R¹ est un groupe alkyle ayant 1 à 4 atomes de carbone, un groupe alcényle ayant 2 à 4 atomes de carbone, un groupe cycloalkyle ayant 3 à 6 atomes de carbone, un groupe 2-hydroxyéthyle, 2-fluoréthyle, méthoxy, amino, méthylamino, éthylamino, diméthylamino, phényle éventuellement substitué par un ou deux atomes de fluor,
R² est de l'hydrogène, un groupe alkyle ayant 1 à 4 atomes de carbone ou un groupe (5-méthyl-2-oxo-1,3-dioxol-4-yl)méthyle et
R³ est un reste de structure 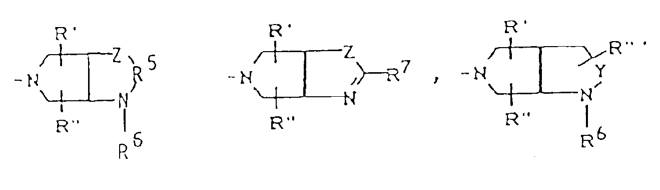 où
R⁵ est un pont alkylène en C₁ à C₃ éventuellement substitué une ou deux fois par un radical méthyle,
R⁶ peut représenter H, un groupe alkyle en C₁ à C₄ éventuellement substitué par un radical hydroxy, ainsi qu'un groupe aryle, hétéroaryle, benzyle, (alkoxy en C₁ à C₄)carbonyle, acyle en C₁ à C₄, (5-méthyl-2-oxo-1,3-dioxol-4-yl)méthyle ou cycloalkyle en C₃ à C₆,
R⁷ peut représenter H ou un groupe alkyle en C₁ à C₄,
R' peut représenter H, un groupe CH₃ ou phényle,
R" peut représenter H, CH₃ ou un groupe phényle,
R"' peut représenter H ou CH₃,
Y peut représenter O, un groupe CH₂, CH₂CH₂ ou CH₂-0, la liaison du groupe CH₂-0 à l'azote pouvant s'effectuer tant par de l'oxygène que par un groupe CH₂, et
Z peut représenter O ou S,
A représente N ou un groupe C-R⁸, dans lequel
R⁸ est de l'hydrogène, un halogène, un groupe méthyle, cyano, nitro, hydroxy ou méthoxy ou peut aussi former conjointement avec R¹ un pont de structure 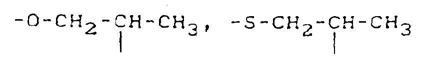 ou 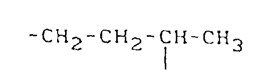 et de leurs hydrates et leurs sels d'addition d'acides pharmaceutiquement utilisables ainsi que des sels de métaux alcalins, de métaux alcalino-terreux, d'argent et de guanidinium des acides carboxyliques correspondants, comprenant la réaction d'un composé de formule (II) 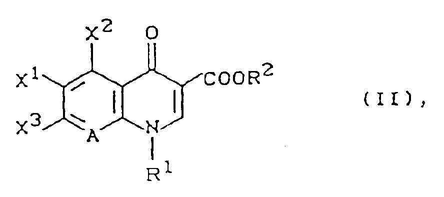 dans laquelle
A R¹, R², X¹ et X² ont la définition indiquée ci-dessus et
X³ est un halogène, notamment le fluor ou le chlore, avec des composés de formule (III) 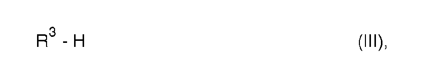 dans laquelle
R³ a la définition indiquée ci-dessus,
le cas échéant en présence d'accepteurs d'acides et le cas échéant avec élimination des groupes protecteurs contenus dans R³.

2. Procédé de production de composés de formule (I) suivant la revendication 1 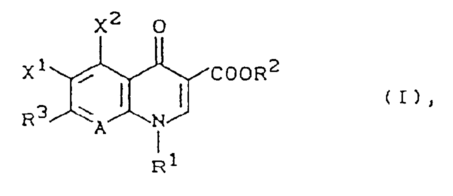 dans laquelle
X¹, R¹, R², R³ et A ont la définition indiquée dans la revendication 1 et
X² est un groupe amino, alkylamino ayant 1 à 4 atomes de carbone, dialkylamino ayant 1 à 3 atomes de carbone par groupe alkyle, hydroxy, alkoxy ayant 1 à 4 atomes de carbone, mercapto, alkylthio ayant 1 à 4 atomes de carbone, ou arylthio,
caractérisé en ce qu'on fait réagir un composé de formule (IV) 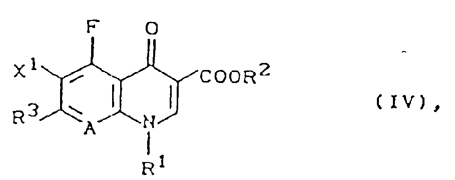 dans laquelle
X¹, R¹, R², R³ et A ont la définition indiquée ci-dessus, avec des composés de formule (V) 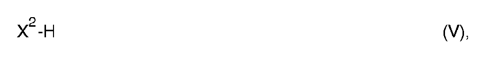 dans laquelle
X² a la définition indiquée ci-dessus, le cas échéant en présence d'accepteurs d'acides.

3. Procédé de production de composés de formule (la) 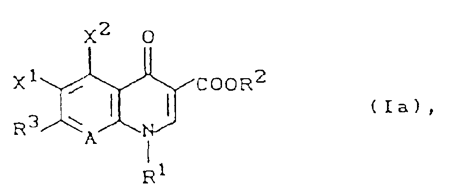 dans laquelle
X¹, X², R¹, R² et A ont la définition indiquée dans la revendication 1, et R³ est un reste de structure 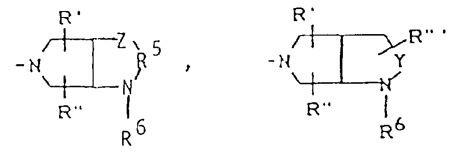 où
R⁵, R⁶, R', R" R"', Y et Z ont la définition indiquée dans la revendication 1,
caractérisé en ce qu'on fait réagir un composé de formule (VI) 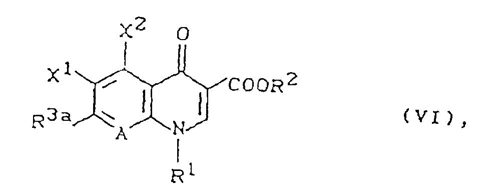 dans laquelle
X¹, X², R¹, R² et A ont la définition indiquée ci-dessus et
_{R}3a représente un reste de structure 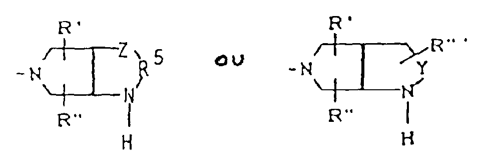 où R⁵, R', R", R"', Y et Z ont la définition indiquée ci-dessus,
avec des composés de formule (VII) 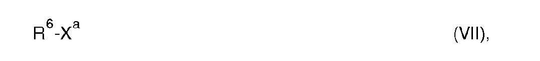 dans laquelle
R⁶ a la définition indiquée ci-dessus et
X^{a} représente du chlore, du brome, de l'iode, un groupe hydroxy ou acyloxy,
le cas échéant en présence d'accepteurs d'acides.

4. Procédé suivant l'une quelconque des revendications 1 à 3, dans lequel, dans la formule (I) ou (la)
X¹ représente du fluor ou du chlore,
X² est de l'hydrogène, un groupe amino, alkylamino ayant 1 ou 2 atomes de carbone, diméthylamino, hydroxy méthoxy, mercapto, méthylthio, phénylthio, du fluor, du chlore,
R¹ est un groupe alkyle ayant 1 à 3 atomes de carbone, un groupe alcényle ayant 2 ou 3 atomes de carbone, un groupe cycloalkyle ayant 3 à 5 atomes de carbone, un groupe 2-hydroxyéthyle, 2-fluoréthyle, méthoxy, amino, méthylamino, éthylamino, diméthylamino, phényle éventuellement substitué par un ou deux atomes de fluor,
R² est de l'hydrogène, un groupe alkyle ayant 1 à 3 atomes de carbone ou (5-méthyl-2-oxo-1,3-dioxol-4-yl)méthyle et
R³ est un reste de structure 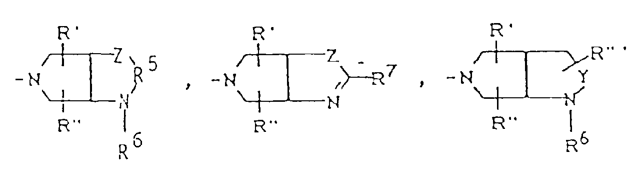 où
R⁵ est un pont alkylène en C₁ ou C₂ éventuellement substitué une ou deux fois par un radical méthyle,
R⁶ peut représenter H, un groupe alkyle en C₁ à C₃ éventuellement substitué par un radical hydroxy, ainsi qu'un groupe phényle, benzyle, (alkoxy en C₁ à C₄)carbonyle, acyle en C₁ ou C₂, (5-méthyl-2-oxo-1,3-dioxol-4-yl) méthyle ou alkyle en C₃ à C₅,
R⁷ peut représenter H ou un groupe alkyle en C₁ ou C₂,
R' peut représenter H ou un groupe CH₃,
R" peut représenter H ou un groupe CH₃,
R"' peut représenter H ou un groupe CH₃,
Y peut représenter O, un groupe CH₂, CH₂CH₂ ou CH₂-0, la liaison du groupe CH₂-0 à l'azote pouvant s'effectuer tant par de l'oxygène que par un groupe CH₂, et
Z peut représenter O ou S,
A représente N ou un groupe C-R⁸, dans lequel
R⁸ est de l'hydrogène, du fluor, du chlore, du brome, un groupe hydroxy ou méthoxy, ou peut aussi former conjointement avec R¹ un pont de structure

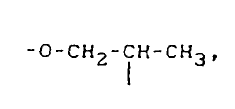

5. Procédé suivant l'une quelconque des revendications 1 à 3, dans lequel, dans la formule (I) ou (la)
X¹ est du fluor,
X² est de l'hydrogène, un groupe amino, méthylamino, du fluor,
R¹ est un groupe alkyle ayant 1 ou 2 atomes de carbone, vinyle, cyclopropyle, 2-hydroxyéthyle, 2-fluoréthyle, méthoxy, méthylamino, 4-fluorophényle, 2,4-difluorophényle,
R² est de l'hydrogène, un groupe alkyle ayant 1 ou 2 atomes de carbone,
R³ est un reste de structure 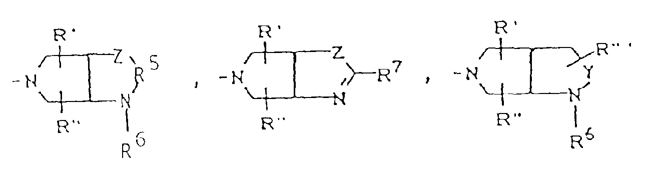 où
R⁵ représente un pont alkylène en C₁ ou C₂ substitué par un radical méthyle,
R⁶ peut être de l'hydrogène, un groupe CH₃, C₂H₅, HOCH₂CH₂, benzyle, (alkoxy en C₁ à C₄)carbonyle, acyle en C₁ ou C_{2'}
R⁷ peut représenter H ou CH₃,
R' peut représenter H ou CH₃,
R" peut représenter H ou CH₃,
R"' peut représenter H ou CH₃,
Y peut représenter O, un groupe CH₂, CH₂CH₂ ou CH₂-O, la liaison du groupe CH₂-O à l'azote pouvant s'effectuer tant par l'intermédiaire d'oxygène qu'au moyen d'un groupe CH₂, et
Z peut représenter O ou S,
A représente de l'azote ou un groupe C-R⁸ dans lequel
R⁸ est de l'hydrogène, du fluor, du chlore ou un groupe méthoxy, ou peut aussi former conjointement avec R¹ un pont de structure

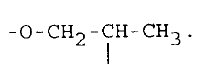

6. Procédé suivant la revendication 1, dans lequel l'un des composés suivants de formule (III) est amené à réagir :
le dichlorhydrate de 2-oxa-5,8-diazabicyclo-[4.3.0]nonane,
le trans-2-oxa-5,8-diazabicyclo[4.3.0]nonane,
le dichlorhydrate de 5-méthyl-2-oxa-5,8-diazabicyclo[4.3.0] nonane,
le 2,8-diazabicyclo[4.3.0]nonane,
le 4-méthyl-2,8-diazabicyclo[4.3.0]nonane,
le 2-méthyl-2,8-diazabicyclo[4.3.0]nonane,
le 2,7-diazabicyclo[3.3.0]octane,
le 2-méthyl-2,7-diazabicyclo[3.3.0]octane,
le 3-oxa-2,7-diazabicyclo[3.3.0]octane,
le 2-méthyl-3-oxa-2,7-diazabicyclo[3.3.0]octane,
le 2,5-diméthyl-3-oxa-2,7-diazabicyclo[3.3.0]-octane,
le 2,8-diméthyl-3-oxa-2,7-diazabicyclo[3.3.0]-octane,
le 2-méthyl-4-oxa-2,8-diazabicyclo[4.3.0]nonane,
le 3-méthyl-2,7-diazabicyclo[3.3.0]octane,
le 2,3-diméthyl-2,7-diazabicyclo[3.3.0]octane,
l'ester éthylique d'acide 2,7-diazabicyclo-[3.3.0]octane-2-carboxylique,
le 2-phényl-2,7-diazabicyclo[3.3.0]octane,
le 4-oxa-2,8-diazabicyclo[4.3.0]nonane.

7. Procédé suivant la revendication 1, dans lequel l'un des composés suivants de formule (II) est amené à réagir :
l'acide 5,6,7,8-tétrafluoro-1-(2,4-difluorophényl)-1,4-dihydro-4-oxo-3-quinoléine-carboxylique,
l'acide 5,7-dichloro-1 -cyclopropyl-6-fluoro-l ,4-dihydro-4-oxo-3-quinoléine-carboxylique et
l'acide 5,7-dichloro-6-fluoro-1-(2,4-difluorophényl)-1,4-dihydro-4-oxo-3-quinoléine-carboxylique.

8. Procédé suivant la revendication 1, pour la préparation de dérivés d'acides carboxyliques de formule (I), dans laquelle :
X¹ est le fluor,
X² est l'hydrogène,
R¹ est le groupe cyclopropyle,
R² est l'hydrogène,
R³ est un reste de formule 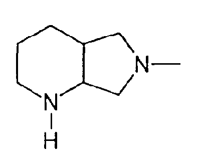 et
A est un groupe C-CI, C-F Ou -C-OCH₃.

9. Dérivés d'acides 7-(1-pyrrolidinyl)-3-quinolone- et -naphtyridone-carboxyliques de formule (I) 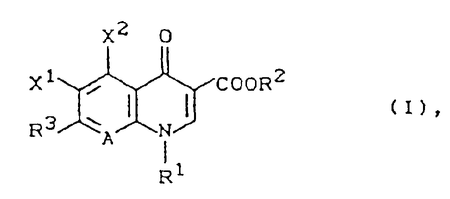 dans laquelle
X¹ représente un halogène,
X² est de l'hydrogène, un groupe amino, alkylamino ayant 1 à 4 atomes de carbone, dialkylamino ayant 1 à 3 atomes de carbone par groupe alkyle, hydroxy, alkoxy ayant 1 à 4 atomes de carbone, mercapto, alkylthio ayant 1 à 4 atomes de carbone, arylthio, un halogène,
R¹ est un groupe alkyle ayant 1 à 4 atomes de carbone, un groupe alcényle ayant 2 à 4 atomes de carbone, un groupe cycloalkyle ayant 3 à 6 atomes de carbone, un groupe 2-hydroxyéthyle, 2-fluoréthyle, méthoxy amino, méthylamino, éthylamino, diméthylamino, phényle éventuellement substitué par un ou deux atomes de fluor,
R² est de l'hydrogène, un groupe alkyle ayant 1 à 4 atomes de carbone ou un groupe (5-méthyl-2-oxo-1,3-dioxol-4-yl)méthyle et
R³ est un reste de structure 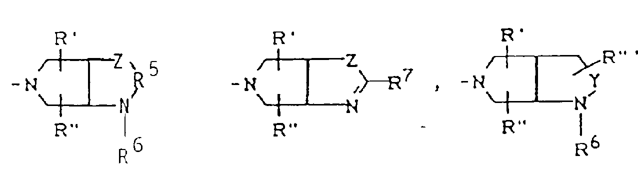 où
R⁵ est un pont alkylène en C₁ à C₃ éventuellement substitué une ou deux fois par un radical méthyle,
R⁶ peut représenter H, un groupe alkyle en C₁ à C₄ éventuellement substitué par un radical hydroxy, ainsi qu'un groupe aryle, hétéroaryle, benzyle, (alkoxy en C₁ à C₄)carbonyle, acyle en C₁ à C₄, (5-méthyl-2-oxo-1,3-dioxol-4-yl)méthyle ou cycloalkyle en C₃ à C₆,
R⁷ peut représenter H ou un groupe alkyle en C₁ à C₄,
R' peut représenter H, CH₃ ou un groupe phényle,
R" peut représenter H, un groupe CH₃ ou phényle,
R"' peut représenter H ou CH₃,
Y peut représenter O, un groupe CH₂, CH₂CH₂ ou CH₂-O, la liaison du groupe CH₂-0 à l'azote pouvant s'effectuer tant par de l'oxygène que par un groupe CH₂, et
Z peut représenter O ou S,
A représente N ou un groupe C-R⁸, dans lequel
R⁸ est de l'hydrogène, un halogène, un groupe méthyle, cyano, nitro, hydroxy ou méthoxy ou peut aussi former conjointement avec R¹ un pont de structure 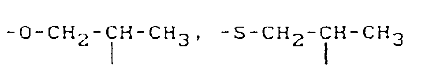 ou 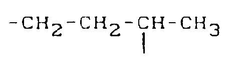 et leurs hydrates et leurs sels d'addition d'acides pharmaceutiquement utilisables ainsi que les sels de métaux alcalins, de métaux alcalino-terreux, d'argent et de guanidinium, des acides carboxyliques correspondants.

10. Composés de formule (I) suivant la revendication 9, dans lesquels
X¹ représente du fluor ou du chlore,
X² est de l'hydrogène, un groupe amino, alkylamino ayant 1 ou 2 atomes de carbone, diméthylamino, hydroxy méthoxy, mercapto, méthylthio, phénylthio, du fluor, du chlore,
R¹ est un groupe alkyle ayant 1 à 3 atomes de carbone, un groupe alcényle ayant 2 ou 3 atomes de carbone, un groupe cycloalkyle ayant 3 à 5 atomes de carbone, un groupe 2-hydroxyéthyle, 2-fluoréthyle, méthoxy, amino, méthylamino, éthylamino, diméthylamino, phényle éventuellement substitué par un ou deux atomes de fluor,
R² est de l'hydrogène, un groupe alkyle ayant 1 à 3 atomes de carbone ou (5-méthyl-2-oxo-1,3-dioxol-4-yl)méthyle,
R³ représente un reste de structure 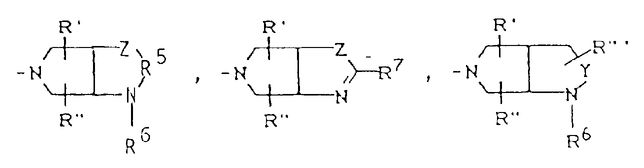 où
R⁵ représente un pont alkylène en C₁ ou C₂ éventuellement substitué une ou deux fois par un radical méthyle,
R⁶ peut représenter H, un groupe alkyle en C₁ à C₃ éventuellement substitué par un radical hydroxy, ainsi qu'un groupe phényle, benzyle, (alkoxy en C₁ à c₄)carbonyle, acyle en C₁ ou C₂, (5-méthyl-2-oxo-1,3-dioxol-4-yl) méthyle ou alkyle en C₃ à C₅,
R⁷ peut représenter H ou un groupe alkyle en C₁ ou C₂,
R' peut représenter H ou un groupe CH₃,
R" peut représenter H ou un groupe CH₃,
R"' peut représenter H ou un groupe CH₃,
Y peut représenter O, un groupe CH_{2;} CH₂CH₂ ou CH₂-O, la liaison du groupe CH₂-O avec l'azote pouvant s'effectuer tant par l'intermédiaire d'oxygène que par l'intermédiaire d'un groupe CH₂, et
Z peut représenter O ou S,
A représente N ou un groupe C-R⁸, dans lequel
R⁸ représente de l'hydrogène, du fluor, du chlore, du brome, un groupe hydroxy ou méthoxy ou peut aussi former conjointement avec R¹ un pont de structure

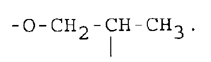

11. Composés de formule (I) suivant la revendication 9, dans lesquels
X¹ est du fluor,
X² est de l'hydrogène, un groupe amino, méthylamino, du fluor,
R¹ est un groupe alkyle ayant 1 ou 2 atomes de carbone, vinyle, cyclopropyle, 2-hydroxyéthyle, 2-fluoréthyle, méthoxy, méthylamino, 4-fluorophényle, 2,4-difluorophényle,
R² est de l'hydrogène, un groupe alkyle ayant 1 ou 2 atomes de carbone,
R³ est un reste de structure 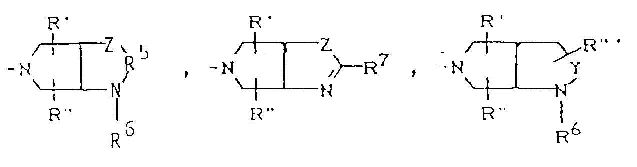 où
R⁵ représente un pont alkylène en C₁ ou C₂ substitué par un radical méthyle,
R⁶ peut être de l'hydrogène, un groupe CH₃, C₂H₅, HOCH₂CH₂, benzyle, (alkoxy en C₁ à C₄)carbonyle, acyle en C₁ ou C₂,
R7 peut représenter H ou CH₃,
R' peut représenter H ou CH₃,
R" peut représenter H ou CH₃,
R"' peut représenter H ou CH₃,
Y peut représenter O, un groupe CH₂, CH₂CH₂ ou CH₂-O, la liaison du groupe CH₂-0 à l'azote pouvant s'effectuer tant par l'intermédiaire d'oxygène qu'au moyen d'un groupe CH₂, et
Z peut représenter O ou S,
A représente de l'azote ou un groupe C-R⁸ dans lequel
R⁸ est de l'hydrogène, du fluor, du chlore ou un groupe méthoxy, ou peut aussi former conjointement avec R¹ un pont de structure

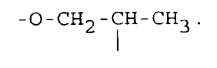

12. Utilisation de composés de formule (I) suivant la revendication 9 pour la préparation de médicaments.

13. Utilisation de composés de formule (I) suivant la revendication 9 comme additifs dans des aliments pour animaux.

14. Aliments pour animaux ou additifs d'aliments pour animaux et mélanges préalables contenant des composés de formule (I) suivant la revendication 9.

15. Composés du groupe comprenant :
le dichlorhydrate de 2-oxa-5,8-diazabicyclo-[4.3.0]nonane,
le trans-2-oxa-5,8-diazabicyclo[4.3.0]nonane,
le dichlorhydrate de 5-méthyl-2-oxa-5,8-diazabicyclo[4.3.0]nonane,
le 2,8-diazabicyclo[4.3.0]nonane,
le 4-méthyl-2,8-diazabicyclo[4.3.0]nonane,
le 2-méthyl-2,8-diazabicyclo[4.3.0]nonane,
le 2,7-diazabicyclo[3.3.0]octane,
le 2-méthyl-2,7-diazabicyclo[3.3.0]octane,
le 3-oxa-2,7-diazabicyclo[3.3.0]octane,
le 2-méthyl-3-oxa-2,7-diazabicyclo[3.3.0]octane,
le 2,5-diméthyl-3-oxa-2,7-diazabicyclo[3.3.0]-octane,
le 2,8-diméthyl-3-oxa-2,7-diazabicyclo[3.3.0]-octane,
le 2-méthyl-4-oxa-2,8-diazabicyclo[4.3.0]nonane,
le 3-méthyl-2,7-diazabicyclo[3.3.0]octane,
le 2,3-diméthyl-2,7-diazabicyclo[3.3.0]octane,
l'ester éthylique d'acide 2,7-diazabicyclo-[3.3.0]octane-2-carboxylique,
le 2-phényl-2,7-diazabicyclo[3.3.0]octane,
le 4-oxa-2,8-diazabicyclo[4.3.0]nonane.

16. Composés du groupe comprenant
l'acide 5,6,7,8-tétrafluoro-1-(2,4-difluorophényl)-1,4-dihydro-4-oxo-3-quinoléine-carboxylique,
l'acide 5,7-dichloro-1-cyclopropyl-6-fluoro-1,4-dihydro-4-oxo-3-quinoléine-carboxylique et
l'acide 5,7-dichloro-6-fluoro-1-(2,4-difluorophényl)-1,4-dihydro-4-oxo-3-quinoléine-carboxylique.

17. Dérivés d'acides carboxyliques de formule (I) suivant la revendication 1, dans lesquels
X¹ représente le fluor,
X² représente l'hydrogène,
R¹ représente le groupe cyclopropyle,
R² représente l'hydrogène,
R³ est un reste de formule 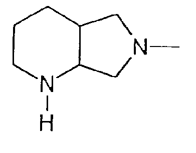 et
A représente C-CI, C-F ou -C-OCH₃,
et leurs sels acceptables du point de vue pharmaceutique.

18. Dérivés d'acides carboxyliques suivant la revendication 17, destinés à être utilisés comme agents antibactériens.

19. Utilisation de composés suivant la revendication 17 pour la préparation de médicaments contre des maladies bactériennes.

20. Utilisation de composés suivant la revendication 17 dans l'alimentation du bétail.
